(19) Europäisches Patentamt
European Patent Office
Office européen des brevets

(11) EP 0 946 503 B1

(12) FASCICULE DE BREVET EUROPEEN

(45) Date de publication et mention de la délivrance du brevet:
**27.03.2002 Bulletin 2002/13**

(21) Numéro de dépôt: **97953797.4**

(22) Date de dépôt: **12.12.1997**

(51) Int Cl.7: **C07C 317/34**, C07C 323/49, C07C 317/42, C07C 317/40, C07D 333/34, C07D 307/91, C07D 213/71, A61K 31/18

(86) Numéro de dépôt international:
**PCT/EP97/06981**

(87) Numéro de publication internationale:
**WO 98/28265 (02.07.1998 Gazette 1998/26)**

# (54) DERIVES DU NITROMETHYLTHIOBENZENE COMME INHIBITEURS DE L'ALDOSE REDUCTASE

NITROMETHYLTHIOBENZOLDERIVATE ALS ALDOSE-REDUCTASE-INHIBITOREN

NITROMETHYLTHIOBENZENE DERIVATIVES AS INHIBITORS OF ALDOSE REDUCTASE

(84) Etats contractants désignés:
**AT BE CH DE DK ES FI FR GB GR IE IT LI LU NL PT SE**
Etats d'extension désignés:
**LT LV SI**

(30) Priorité: **23.12.1996 FR 9615887**

(43) Date de publication de la demande:
**06.10.1999 Bulletin 1999/40**

(73) Titulaire: **MERCK PATENT GmbH**
**64293 Darmstadt (DE)**

(72) Inventeurs:
- **COLLONGES, François**
  **F-01700 Beynost (FR)**
- **DUMAS, Hervé**
  **F-38090 Vaulx-Milieu (FR)**
- **LARDY, Claude**
  **F-69008 Lyon (FR)**
- **DURBIN, Philippe**
  **F-69100 Villeurbanne (FR)**

(56) Documents cités:
**EP-A- 0 304 190**
**EP-A- 0 469 887**
**EP-A- 0 469 888**
**EP-A- 0 469 889**
**WO-A-90/08761**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux dérivés de nitrométhylthiobenzène, les procédés permettant de les préparer et leur application dans le domaine thérapeutique, et plus particulièrement dans le traitement ou la prévention des complications diabétiques.

Le diabète est caractérisé par une concentration élevée de glucose dans le sang. Ce glucose est métabolisé normalement par l'enzyme hexokinase lors de la première étape de la glycolyse, aboutissant à la dégradation en pyruvate. Lorsque la concentration en glucose est trop élevée, l'hexokinase se trouve saturée, et une deuxième voie de métabolisation du glucose entre en jeu ; il s'agit de la voie des polyols qui fait intervenir successivement deux enzymes : l'aldose réductase qui transforme le glucose en sorbitol et la sorbitol déshydrogénase qui transforme le sorbitol en fructose. En cas de diabète, l'excès de glucose accélère la formation de sorbitol qui tend à s'accumuler. Ceci entraîne de graves perturbations métaboliques, comme par exemple une augmentation de pression osmotique, susceptible d'entraîner une dégénérescence tissulaire. Les inhibiteurs d'aldose réductase sont donc utiles pour traiter ou prévenir certaines des complications induites par le diabète.

De nombreux produits sont décrits dans la littérature comme inhibiteurs d'aldose réductase actifs in vitro et in vivo. Ce sont principalement des dérivés d'hydantoïnes, de succinimides et d'acides acétiques. Plus récemment sont apparus dans le brevet européen 304.190 des dérivés de (phénylsulfonyl) nitrométhanes et en particulier dans le brevet WO 90/08761, le composé 3,5-diméthyl-4-[(nitrométhyl)sulfonyl] aniline. Ce demier composé a généré plusieurs séries dérivées comme les produits de N-acylation décrits dans le brevet européen 469.887 et les (oxamido-et uréido-phénylsulfonyl) nitrométhanes décrits dans le brevet européen 469.889.

La présente invention concerne des dérivés du nitrométhylthiobenzène répondant à la formule générale 1,

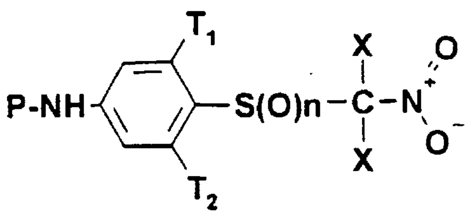

**(1)**

dans laquelle :

P représente

le radical (i) : $-(CO-NH)_m-SO_2-R$;
le radical (ii) :

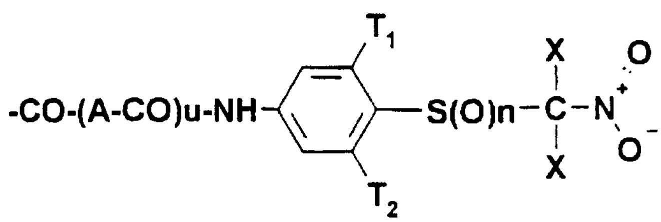

ou bien le radical (iii) :

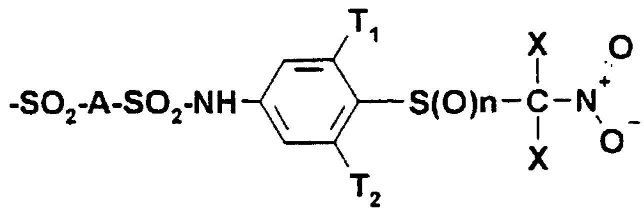

R représente un radical choisi parmi phényle, benzyle, diphénylméthyle, naphtyle, cycloalkylakyle dans lequel la partie alkyle est en $C_1$-$C_4$ et la partie cycloalkyle est en $C_3$-$C_7$, et styryle , ledit radical étant éventuellement substitué par un ou plusieurs groupes Z identiques ou différents, ou bien
R représente un radical hétérocyclique aromatique en $C_3$-$C_5$ comprenant 1 ou 2 hétéroatomes choisis parmi O, S et N, ledit radical étant éventuellement substitué par un ou plusieurs groupes Z identiques ou différents et étant éventuellement condensé à 1 ou 2 noyaux phényle éventuellement substitués par un ou plusieurs groupes Z identiques ou différents; ou bien
R représente alkyle en $C_1$-$C_4$ éventuellement substitué par un ou plusieurs atomes d'halogène identiques ou différents, cycloalkyle en $C_3$-$C_7$ ou ($C_3$-$C_7$)-cycloalkyle-($C_1$-$C_4$)alkyle;
Z est choisi parmi un atome d'halogène, un groupe alkyle en $C_1$-$C_4$, alcoxy en $C_1$-$C_4$, nitro, cyano, trifluorométhyle, trifluorométhoxy, ($C_2$-$C_5$)alcanoylamino, ($C_1$-$C_4$)alkylsulfonyle, ($C_1$-$C_4$)alkylthio et phényle;
X représente un atome d'hydrogène ou d'halogène;
m est 0 ou 1 ;
n est 0, 1 ou 2;
$T_1$ et $T_2$ représentent indépendamment l'un de l'autre un atome d'hydrogène ou un groupe alkyle en $C_1$-$C_4$,
u est 0 ou 1;
A représente alkylène en $C_1$-$C_8$ ou le groupe

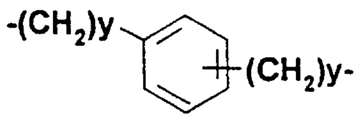

y étant un entier choisi parmi 0, 1, 2, 3 et 4; étant entendu que lorsque P représente le radical (ii), A peut également représenter une liaison;

leurs formes tautomères, et leurs sels d'addition avec des bases pharmaceutiquement acceptables.

On désigne par alkyle en $C_1$-$C_4$ un radical hydrocarboné saturé linéaire ou ramifié comprenant de 1 à 4 atomes de carbone. Le groupe alcoxy désigne en conséquence le groupe alkyle-O- dans lequel alkyle a la signification indiquée ci-dessus.

En tant qu'hétérocycle aromatique en $C_3$-$C_5$ comprenant 1 ou 2 hétéroatomes choisis parmi O, S et N, on peut citer les furane, thiophène, pyrrole, oxazole, thiazole, imidazole, pyrazole, isoxazole, isothiazole, pyridine, pyridazine, pyrimidine et pyrazine, la pyridine et le thiophène étant préférés.

Tel qu'on l'emploie ici, le terme halogène désigne un atome de fluor, de brome, de chlore ou d'iode.

Des exemples de groupe cycloalkyle sont les groupes cyclopropyle, cyclobutyle, cyclopentyle ou cyclohexyle. Selon l'invention le groupe cycloalkyl-alkyle désigne un groupe alkyle substitué par un groupe cycloalkyle.

Par radical alkylène, on entend une chaîne saturée hydrocabonée linéaire ou ramifiée divalente telle que $-CH_2-$ ; $-CH_2-CH_2-CH_2-$; ou $-CH_2-CH(CH_3)-CH_2-$.

Le groupe de formule :

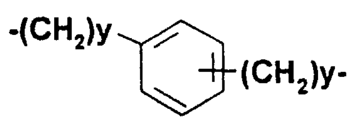

correspond à l'une des formules suivantes :

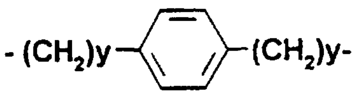
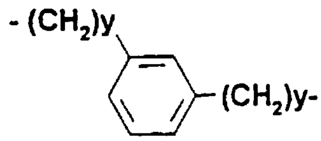
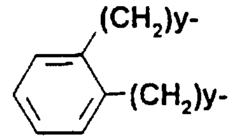

dans laquelle y représente 0, 1, 2, 3 ou 4.

Selon l'invention, lorsque P représente le radical (ii) de formule :

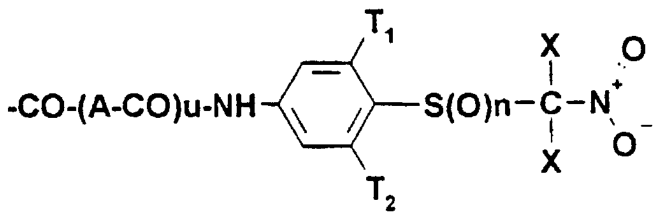

A est choisi parmi :

- une liaison, un radical alkylène en $C_1$-$C_8$, et
- le groupe de formule

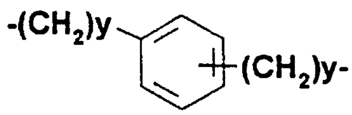

dans lequel y est un entier choisi parmi 0, 1, 2, 3 et 4.

Inversement lorsque P est $-(CO\text{-}NH)_m\text{-}SO_2\text{-}R$ ou le radical (iii) de formule :

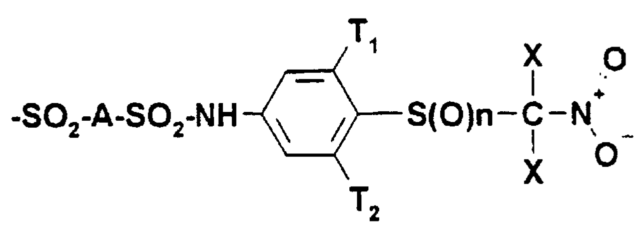

alors A est choisi parmi :

- un radical alkylène en $C_1$-$C_8$, et
- le groupe de formule :

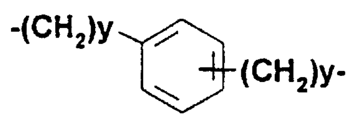

dans lequel y est un entier choisi parmi 0, 1, 2, 3 et 4.

Les formes tautomères éventuelles des composés de formule 1 font partie intégrante de l'invention.

Les sels d'addition avec des bases pharmaceutiquement acceptables de composés de formule 1 dans lesquels X est un atome d'hydrogène et n est égal à 1 ou 2 font également partie intégrante de l'invention, par exemple un sel de métal alcalin ou alcalino-terreux, comme un sel de sodium, de potassium, de calcium, de magnésium, un

sel d'aluminium, un sel d'ammonium ou un sel d'une base organique apportant un cation pharmaceutiquement acceptable.

Un premier groupe de composés préférés est constitué des composés de formule 1 dans lesquels :

P représente $-(CO-NH)_m-SO_2-R$
R représente un radical choisi parmi phényle, diphénylméthyle, naphtyle et styryle, ledit radical étant éventuellement substitué par un ou plusieurs groupes Z identiques ou différents, ou bien
R représente un radical hétérocyclique aromatique en $C_3$-$C_5$ comprenant 1 ou 2 hétéroatomes choisis parmi O, S et N, ledit radical étant éventuellement substitué par un ou plusieurs groupes Z identiques ou différents et étant éventuellement condensé à 1 ou 2 noyaux phényle éventuellement substitués par un ou plusieurs groupes Z identiques ou différents; ou bien
R représente alkyle en $C_1$-$C_4$ éventuellement substitué par un ou plusieurs atomes d'halogène identiques ou différents, cycloalkyle en $C_3$-$C_7$ ou ($C_3$-$C_7$)-cycloalkyle-($C_1$-$C_4$)alkyle;
Z, X, m et n étant tels que définis ci-dessus pour ta formule (1).

Un second groupe de composés préférés englobe les composés de formule 1 dans lesquels :

P représente $-(CO-NH)_m-SO_2-R$,
R représente phényle; phényle substitué par un ou plusieurs groupes Z identiques ou différents; benzyle; benzyle substitué par un ou plusieurs groupes Z identiques ou différents; alkyle en $C_1$-$C_4$ éventuellement substitué par un ou plusieurs atomes d'halogène identiques ou différents; cycloalkyle en $C_3$-$C_7$; ($C_3$-$C_7$)cycloalkyle-($C_1$-$C_4$)alkyle; styryle; thiényle; pyridyle; naphtyle; dibenzofuranyle; ou diphénylméthyle;
Z est choisi parmi un atome d'halogène, un groupe alkyle en $C_1$-$C_4$, alkoxy en $C_1$-$C_4$, nitro, trifluorométhyle, trifluorométhoxy, ($C_2$-$C_5$)alcanoylamino, ($C_1$-$C_4$)alkylsulfonyle et phényle;
X, m et n étant tels que définis ci-dessus par la formule (1).

Parmi ce deuxième groupe de composés préférés, les composés pour lesquels :

P représente $-(CO-NH)_m-SO_2-R$,
R représente phényle; phényle substitué par un ou plusieurs groupes Z identiques ou différents; benzyle; benzyle substitué par un ou plusieurs groupes Z identiques ou différents; méthyle; cycloalkyle en $C_3$-$C_7$; ($C_3$-$C_7$)cycloalkyle-($C_1$-$C_4$)alkyle; styryle; thiényle; pyridyle, naphtyle; dibenzofuranyle; diphénylméthyle ou 2,2,2-trifluoroéthyle;
Z est choisi parmi fluoro, chloro, bromo, méthyle, méthoxy, nitro, trifluorométhyle, trifluorométhoxy, acétamido, méthylsulfonyle et phényle;
X représente hydrogène ou chlore;
m et n étant tels que définis ci-dessus pour la formule (1), sont particulièrement avantageux.

Un troisième groupe de composés préférés est constitué des composés de formule 1 dans lesquels :

P représente $-(CO-NH)_m-SO_2-R$,
R représente phényle; phényle substitué par un ou plusieurs groupes Z identiques ou différents;
méthyle; cycloalkyle en $C_3$-$C_7$; ($C_3$-$C_7$)cycloalkyle-($C_1$-$C_4$)alkyle; styryle; thiényle; pyridyle, naphtyle; dibenzofuranyle; diphénylméthyle ou 2,2,2-trifluoroéthyle;
Z est choisi parmi fluoro, chloro, bromo, méthyle, méthoxy, nitro, trifluorométhyle, trifluorométhoxy, acétamido, méthylsulfonyle et phényle;
X représente hydrogène ou chlore;
m et n étant tels que définis ci-dessus pour la formule (1).

Un quatrième groupe de composés préférés est constitué des composés de formule (1) dans lesquels :

P représente :

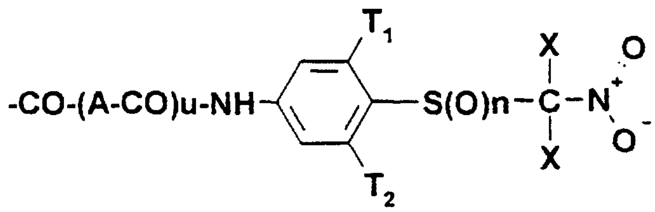

A représente une liaison ou alkylène en $C_1$-$C_8$,
u, n, X, $T_1$ et $T_2$ étant tels que définis ci-dessus pour la formule (1).

Un cinquième groupe de composés préférés est constitué des composés de formule (1) dans lesquels :

P représente :

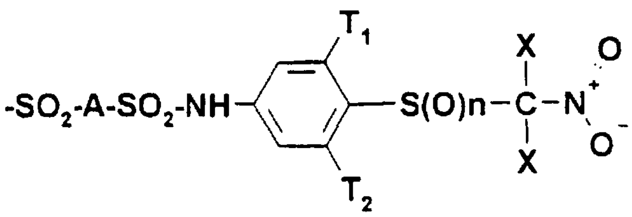

A représente le groupe

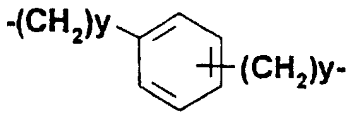

n, X, y, $T_1$ et $T_2$ étant tels que définis ci-dessus pour la formule (1).

Des composés particulièrement préférés sont les suivants :

N-[3,5-Diméthyl-4-[(nitrométhyl)sulfonyl]phényl]benzènesulfonamide;
3,4-Difluoro-N-[3,5-diméthyl-4[(nitrométhyl)sulfonyl]phényl]benzènesulfonamide;
3-Bromo-N-[3,5-diméthyl-4-[(nitrométhyl)sulfonyl]phényl]benzènesulfonamide;
N-[3,5-Diméthyl-4-[(nitrométhyl)sulfonyl]phényl]-2-(trifluorométhyl)benzènesulfonamide;
N-[3,5-Diméthyl-4-[(nitrométhyl)sulfonyl]phényl]-4-fluorobenzènesulfonamide;
N-[3,5-Diméthyl-4-[(nitrométhyl)sulfonyl]phényl]-3-fluorobenzènesulfonamide;
N-[3,5-Diméthyl-4-[(nitrométhyl)sulfonyl]phényl]phénylméthanesulfonamide;
2,3-Difluoro-N-[3,5-diméthyl-4[(nitrométhyl)sulfonyl]phényl]benzènesulfonamide;
3,5-Difluoro-N-[3,5-diméthyl-4[(nitrométhyl)sulfonyl]phényl]benzènesulfonamide;
N-[3,5-Diméthyl-4-[(nitrométhyl)sulfonyl]phényl]-2-fluorobenzènesulfonamide.

et les composés suivants :

N, N'-bis[3,5-diméthyl-4-[(nitrométhyl)sulfonyl]phényl]-1,5-pentanediamide ;
N,N'-bis[3,5-diméthyl-4-[(nitrométhyl)sulfonyl]phényl]-1,8-octanediamide ;
N,N'-bis[4-[(nitrométhyl)sulfonyl]phényl]-1,5-pentanediamide ;
N,N'-bis[3,5-diméthyl-4-[(nitrométhyl)sulfonyl]phényl]-éthanediamide ;
N,N'-bis[3,5-diméthyl-4-[(nitrométhyl)sulfonyl]phényl]-urée ;
N,N'-bis[3,5-diméthyl-4-[(nitrométhyl)sulfonyl]phényl]-1,4-butanediamide ;
N,N'-bis[3,5-diméthyl-4-[(nitrométhyl)sulfonyl]phényl]-1,3-propanediamide ;
N,N'-bis[3,5-diméthyl-4-[(nitrométhyl)sulfonyl]phényl]-1,3-benzènedisulfonamide;

N,N'-bis[3,5-diméthyl-4-[(nitrométhyl)sulfonyl]phényl]-1,3-benzènediméthane sulfonamide.

Les composés de l'invention sont préparés selon les méthodes suivantes

(A) Lorsque P représente $-(CO-NH)_m-SO_2-R$, X est un atome d'hydrogène, m est égal à 0 et n est égal à 2, on fait réagir un chlorure de sulfonyle de formule $RSO_2Cl$, R ayant les significations définies ci-dessus, sur le composé de formule 2,

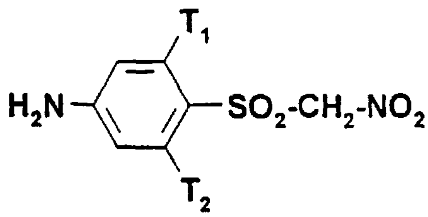

**2**

dans laquelle $T_1$ et $T_2$ sont tels que définis ci-dessus pour (1),
en présence d'une base appropriée.

La réaction est réalisée de préférence dans un solvant, par exemple un solvant aprotique polaire tel que le tétrahydrofurane à une température comprise entre 10°C et la température d'ébullition du solvant.

Comme base particulièrement appropriée, on peut citer le carbonate de calcium (méthode A1) ou la pyridine (méthode A2).

(B) Lorsque P représente $-(CO-NH)_m-SO_2-R$, X est un atome d'halogène de préférence le chlore, m est égal à 0 et n est égal à 2, on fait réagir le N-halogénosuccinimide approprié sur un composé de formule 3

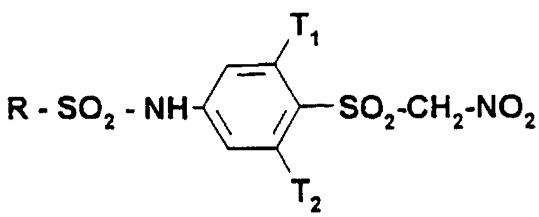

**3**

dans laquelle R, $T_1$ et $T_2$ ont les significations définies ci-dessus pour (1), en présence d'un générateur de radicaux libres comme le 2,2'-azobisisobutyronitrile.

La réaction est réalisée de préférence dans un solvant, par exemple un hydrocarbure halogéné tel que le tétrachlorure de carbone porté à reflux.

(C) Lorsque P représente $-(CO-NH)_m-SO_2-R$, X est un atome d'hydrogène, m est égal à 1 et n est égal à 2, on fait réagir un sulfonylisocyanate de formule $R-SO_2-N=C=0$, R ayant les significations définies ci-dessus, sur le composé de formule 2 défini ci-dessus.

La réaction est réalisée de préférence dans un solvant, par exemple un hydrocarbure halogéné tel que le chlorure de méthylène à une température voisine de la température ambiante.

(D) Lorsque P représente $-(CO-NH)_m-SO_2-R$, X est un atome d'hydrogène, m et n sont égaux à 0, on fait réagir un chlorure de sulfonyle de formule $RSO_2Cl$, R ayant les significations définies ci-dessus, sur le composé de formule 10,

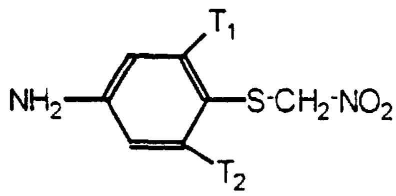

10

dans laquelle $T_1$ et $T_2$ sont tels que définis ci-dessus pour (1),
en présence d'une base appropriée.

La réaction est réalisée de préférence dans un solvant, par exemple un solvant aprotique polaire tel que le tétrahydrofurane à une température voisine de la température ambiante. Une base particulièrement appropriée est, par exemple, le carbonate de calcium.

(E) Lorsque P représente $-(CO-NH)_m-SO_2-R$, X est un atome d'hydrogène, m est égal à 0 et n est égal à 1, on fait réagir un agent oxydant tel que l'acide m-chloroperbenzoïque sur un composé de formule 4.

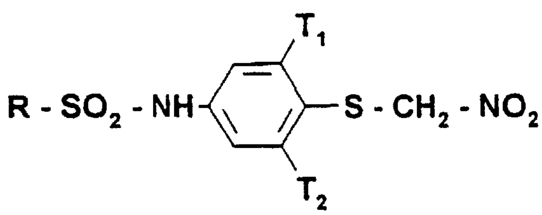

4

dans lequel R, $T_1$ et $T_2$ ont les significations définies ci-dessus.

Le procédé est exécuté de préférence dans un solvant, par exemple le chloroforme, à une température voisine de la température ambiante.

(F) Lorsque P représente le radical (ii) de formule :

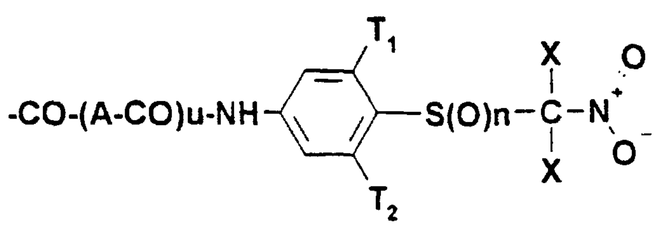

u est 1, X représente un atome d'hydrogène et n est égal à 2, on fait réagir le dichlorure de formule 5 suivant :

$$Cl-(CO-A)_u-COCl \qquad \underline{5}$$

avec au moins deux équivalents molaires de composé de formule 2 défini ci-dessus, en présence d'une base.

La réaction est de préférence réalisée dans un solvant, par exemple un solvant aprotique polaire tel que le tétrahydrofurane à une température comprise entre 10° C et la température d'ébullition du solvant.

Comme base particulièrement appropriée, on peut citer le carbonate de calcium (méthode F1) ou la pyridine (méthode F2).

Le rapport molaire du composé de formule 2 au composé de formule 5 est de préférence compris entre 2 et 4. Il doit être entendu cependant qu'une quantité supérieure de dérivé de l'aniline peut être utilisé sans dommage.

(G) Lorsque P représente le radical (iii) de formule :

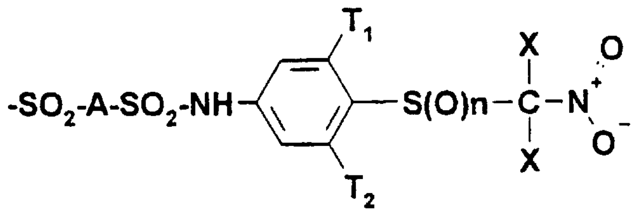

X représente l'atome d'hydrogène et n est égal à 2, on fait réagir le dichlorure de sulfonyle de formule 6 suivant

$$Cl\text{-}SO_2\text{-}A\text{-}SO_2\text{-}Cl \qquad \underline{6}$$

avec au moins deux équivalents molaires de composé de formule 2 défini ci-dessus, en présence d'une base.

Là encore, le carbonate de calcium (méthode G1) et la pyridine (méthode G2) constituent des exemples préférés de base appropriée.

Avantageusement la réaction est effectuée dans un solvant aprotique polaire entre 10° C et la température d'ébullition du solvant.

Il suffit généralement de faire réagir 2 à 4 équivalents molaires de composé de formule 2 sur le dichlorure de formule 6, toutefois une quantité supérieure ne nuit pas à la réaction.

(H) Lorsque P représente le radical (ii) de formule :

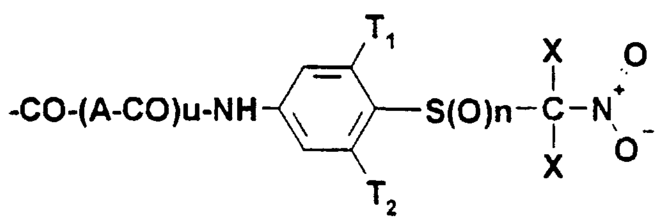

dans lequel u est zéro, X représente un atome d'hydrogène, n est égal à 2, on fait réagir le chloroformiate de trichlorométhyle avec au moins deux équivalents molaires de composé de formule 2 défini ci-dessus en présence de base.

La réaction est de préférence réalisée dans un solvant aprotique polaire tel que le tétrahydrofurane, à une température comprise entre 10° C et la température d'ébullition du solvant.

Comme base particulièrement appropriée, on peut citer le carbonate de calcium. Le rapport molaire du composé de formule 2 au chloroformiate de trichlorométhyle est de préférence compris entre 2 et 4.

Le composé intermédiaire de formule 2 défini ci-dessus est obtenu comme décrit dans le brevet WO 90/08761 par hydrolyse basique du composé de formule 9. Ce demier composé a été préparé selon la méthode suivante, mettant en oeuvre en particulier la réaction originale du nitrométhane sodé sur le thiocvanate d'arvie 7 donnant le composé 8 :

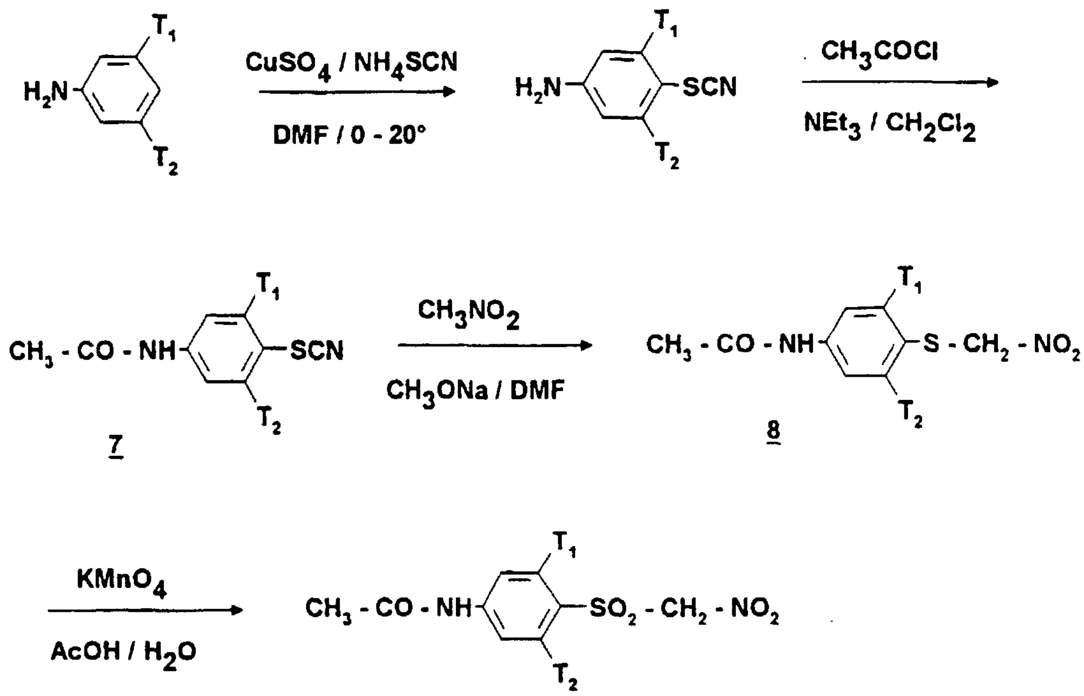


7

8

9

Le composé intermédiaire de formule 10 est obtenu par hydrolyse basique du composé de formule 8.

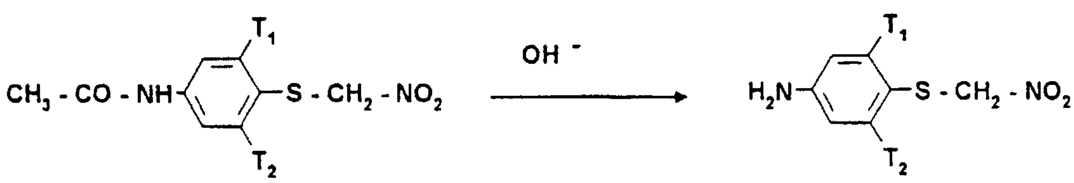


8

10

Un cas particulier de mise en oeuvre de ce procédé consiste à préparer la 3,5-diméthyl-4-[(nitrométhyl)sulfonyl]aniline, laquelle correspond au composé de formule 2 dans laquelle $T_1$ et $T_2$ représentent $-CH_3$.

L'inhibition de l'enzyme aldose réductase et la réduction de l'accumulation de sorbitol peuvent être mises en évidence au cours d'épreuves de laboratoire normalisées ci-après :

1) Etude in vitro : inhibition de l'aldose réductase

L'aldose réductase utilisée est obtenue à partir de cristallins de rats Wistar mâles selon une modification de la méthode de S. HAYMAN et coll. (Journal of Biological Chemistry 240, p. 877, 1965). L'extrait enzymatique est dilué dans un tampon phosphate en présence de NADPH et de différentes concentrations des produits à tester. La réaction est déclenchée par le L-glycéraldéhyde et la vitesse de réaction est mesurée en suivant la disparition du NADPH par spectrophotométrie à 340 nm. La vitesse de réaction est calculée pour chaque concentration de produits puis la concentration nécessaire pour une réduction de 50 % de la vitesse de réaction ($CI_{50}$) est évaluée par interpolation linéaire.

2) Etude in vivo : réduction de l'accumulation de sorbitol

Des rats Wistar mâles de 200 à 250 g sont rendus diabétiques par injection intraveineuse de streptozotocine (60 mg/kg). Ils reçoivent ensuite un traitement oral des produits à tester, sous la forme d'une suspension, 4 heures, 30 heures et 52 heures après l'injection de streptozotocine. Dix huit heures après le dernier traitement oral, les rats sont assommés et décapités puis leurs nerfs sciatiques sont prélevés. Après extraction, le taux de sorbitol des nerfs est mesuré selon la méthode enzymatique décrite par H.U. BERGMEYER (Methods of enzymatic analysis. H.U. BERGMEYER ed., Academic Press New York 3, p 1323, 1974).

Le pourcentage de protection est calculé pour chaque produit par rapport au lot d'animaux diabétiques en tenant compte du taux de sorbitol dans les nerfs sciatiques de rat non-diabétique.

A titre d'exemple, les résultats obtenus pour certains des produits testés sont donnés dans le tableau suivant :

| Produits Exemple N° | Inhibition de l'aldose réductase in vitro $CI_{50}$ (nM) | Protection contre l'augmentation de sorbitol (%) (5 mg/kg/j p.o.) |
|---|---|---|
| 1 | 8 | 65 |
| 10 | 7 | 66 |
| 13 | 8 | 70 |
| 14 | 7 | 70 |
| 24 | 8 | 80 |
| 25 | 7 | 85 |
| 26 | 6 | 104 |
| 29 | 7 | 71 |
| 30 | 8 | 87 |
| 34 | 9 | 69 |

Les composés de l'invention peuvent être utilisés à titre de médicaments en tant qu'inhibiteurs de l'aldose réductase, et notamment dans le traitement des complications du diabète telles que la cataracte, les rétinopathies, les neuropathies, les néphropathies et certaines maladies vasculaires.

Ces médicaments peuvent être administrés par voie orale sous forme de comprimés, de gélules ou de granules à libération immédiate ou à libération contrôlée, par voie intraveineuse sous forme de solution injectable, par voie transdermique sous forme de dispositif transdermique adhésif, par voie topique sous forme de collyre, solution, crème ou gel.

Le principe actif est associé à divers excipients pharmaceutiques. Les posologies journalières peuvent varier de 5 mg à 200 mg de principe actif.

On donne ci-dessous à titre d'exemples non limitatifs quelques formulations pharmaceutiques :

- Composition d'un comprimé à libération immédiate
  Principe actif 100 mg
  Excipients : lactose, amidon de blé, polyvidone, talc, stéarate de magnésium.
- Composition d'un comprimé à libération contrôlée
  Principe actif 100 mg
  Excipients : lactose, polyvidone, talc, stéarate de magnésium, polymère (dérivé cellulosique ou acrylique et méthacrylique ou vinylique ou glycéridique).
- Composition d'une gélule
  Principe actif 100 mg
  Excipients : lactose, amidon de blé, talc, stéarate de magnésium.
- Composition d'une ampoule de solution injectable
  Principe actif 200 mg
  Excipients : mannitol, eau pour préparation injectable
- Composition d'une crème (composition pour 100 g de crème)
  Principe actif 2 g
  Excipients : alcool cétylstéarylique auto émulsionnable, céthylaryloctanoate, nipasol, acide sorbique, propylèneglycol, carbopol.
- Composition d'un collyre
  Principe actif 15 mg
  Excipients : chlorure de sodium, chlorure de benzalkonium, eau pour préparation injectable.

Les exemples suivants illustrent l'invention.

Dans les données de résonnance magnétique nucléaire (RMN), les abréviations suivantes ont été utilisées : s pour singulet, d pour doublet, t pour triplet, q pour quadruplet et m pour massif complexe ; les déplacements chimiques $\delta$ sont exprimés en ppm.

**Exemple 1 (méthode A1)**

**N-[3,5-Diméthyl-4-[(nitrométhyl)sulfonyl]phényl] benzènesulfonamide**

A un mélange maintenu sous atmosphère d'azote de 9,5 g (38,9 mmoles) de 3,5-diméthyl-4-[(nitrométhyl) sulfonyl] aniline dans 180 ml de tétrahydrofurane anhydre, sont ajoutés successivement 7,85 g (78,4 mmoles) de carbonate de calcium et 7,45 ml (58,4 mmoles) de chlorure de benzènesulfonyle. Le milieu réactionnel est porté à reflux pendant 8 heures. Après addition de 2,5 ml (19,6 mmoles) de chlorure de benzènesulfonyle, le reflux est poursuivi pendant 6 h. On ajoute encore 2,5 ml (19,6 mmoles) de chlorure de benzènesulfonyle et le reflux est continué pendant 22 h. Après refroidissement, le milieu réactionnel est jeté dans 600 ml d'eau et extrait au chlorure de méthylène. Ces extraits organiques rassemblés sont séchés sur sulfate de sodium et concentrés à sec sous pression réduite. Le résidu pâteux obtenu est purifié par chromatographie sur colonne de silice (éluant : chlorure de méthylène) et cristallisation dans un mélange d'hexane et de chlorure de méthylène pour donner 6,2 g (41 %) de N-[3,5-diméthyl-4-[(nitrométhyl) sulfonyl]phényl] benzènesulfonamide.

F = 129 - 131°C (hexane-chlorure de méthylène)

| Analyse centésimale : $C_{15}H_{16}N_2O_6S_2$ (M = 384,41) | | | | |
|---|---|---|---|---|
| | C % | H % | N % | S % |
| calculé | 46,86 | 4,20 | 7,29 | 16,68 |
| trouvé | 47,16 | 4,14 | 7,38 | 16,87 |

IR : $\bar{\nu}$ = 3241, 1594, 1558, 1474, 1349, 1322, 1160, 1140 1090, 601 $cm^{-1}$

RMN $^1H$ (DMSO $d_6$) : $\delta$ = 2,52 (6H,s) ; 6,50 (2H, s échangeable par $CF_3COOD$) ; 7,04 (2H,s) ; 7,63-7,72 (3H, m) ; 7,94 - 7,97 (2H,m) ; 11,16 (1H, s, échangeable par $CF_3COOD$).

Le produit de départ 3,5-diméthyl-4-[(nitrométhyl)sulfonyl] aniline est obtenu par mise en oeuvre, dans l'ordre, des étapes 1 à 5 suivantes :

Etape 1 :

A une solution maintenue sous atmosphère d'azote de 121,2 g (1 mole) de 3,5-diméthylaniline dans 1,75 l de N,N-diméthylformamide anhydre, sont ajoutés rapidement 251,2 g (3,30 moles) de thiocyanate d'ammonium. La température s'élève de 18 à 35°C. Une heure et demie plus tard, la température de la solution étant revenue à la température ambiante, on ajoute rapidement 263,3 g (1,65 mole) de sulfate cuivrique anhydre. Le milieu réactionnel est laissé sous agitation à température ambiante pendant 3 jours puis est versé dans 8 l d'eau. Le pH est amené à 7,5 par addition de 550 g (6,55 moles) de bicarbonate de soude. Le milieu est alors filtré, la partie solide étant lavée à l'acétate d'éthyle et la partie liquide étant extraite à l'acétate d'éthyle. Les phases liquides organiques rassemblées sont lavées par 500 ml d'eau, séchées sur sulfate de sodium et concentrées à sec sous pression réduite. Le résidu pâteux obtenu est cristallisé dans 500 ml d'hexane pour donner 128,8 g (72 %) de thiocyanate de 4-amino-2,6-diméthylphényle que l'on utilise dans l'étape suivante sans autre purification.

IR : $\bar{\nu}$ = 3483, 3382, 2142, 1627, 1591, 1470, 1435, 1342, 1191, 856, 850 $cm^{-1}$

RMN $^1H$ (DMSO $d_6$) : $\delta$ = 2,58 (6H,s) ; 5,86 (2H,s) ; 6,65 (2H, s, échangeables par $CF_3COOD$).

Etape 2 :

A un mélange maintenu sous atmosphère d'azote de 75,2 g (422 mmoles) de thiocyanate de 4-amino-2,6-diméthylphényle dans 500 ml de chlorure de méthylène anhydre, sont ajoutés rapidement 46,9 g (463 mmoles) de triéthylamine, puis, goutte à goutte, 32,8 g (418 mmoles) de chlorure d'acétyle. La température s'élève jusqu'à 41°C et il se forme un précipité. Le lendemain, le milieu réactionnel est versé sur 400 ml d'eau. La partie insoluble est isolée par filtration, lavée par 100 ml de chlorure de méthylène et séchée. On obtient 61,8 g (67 %) d'un solide beige irritant et lacrymogène fondant à 204-206°C, constitué essentiellement de thiocyanate de 4-acétamido-2,6-diméthylphényle que l'on utilise dans l'étape suivante sans autre purification.

IR : $\bar{\nu}$ = 1670, 1595, 1541, 1401, 1369, 1325, 1261 $cm^{-1}$

RMN $^1H$ (DMSO $d_6$) : $\delta$ = 2,08 (3H,s) ; 2,52 (6H,s) ; 7,54 (2H,s) ; 10,13 (1H, s, échangeable par $CF_3COOD$).

Etape 3 :

A une solution maintenue sous atmosphère d'azote de 6,95 ml (128 mmoles) de nitrométhane dans 140 ml de N,N-diméthylformamide anhydre, sont ajoutés rapidement 9,4 g (174 mmoles) de méthylate de sodium. La température s'élève spontanément jusqu'à 35°C puis le milieu réactionnel est porté à 40°C et maintenu ainsi pendant 1 h. Après retour à température ambiante, on ajoute goutte à goutte en 1 heure 20, une solution de 12,9 g (58,6 mmoles) de thiocyanate de 4-acétamido-2,6-diméthylphényle dans 140 ml de N,N-diméthylformamide anhydre. Le mélange est agité pendant 20 heures à température ambiante avant d'être versé dans 1 l d'eau. La solution obtenue, de pH 10 environ, est lavée par 2 fois 500 ml d'acétate d'éthyle puis neutralisée jusqu'à pH 7 avec de l'acide chlorhydrique dilué. Cette solution aqueuse neutre est extraite par 4 fois 500 ml d'acétate d'éthyle. Les extraits organiques sont séchés sur sulfate de sodium et concentrés à sec sous pression réduite. Le résidu solide obtenu est purifié par chromatographie sur colonne de silice (éluant : chlorure de méthylène / acétate d'éthyle : 2/1) pour donner 8,3 g (56 %) d'un solide fondant à 160 - 163°C, constitué essentiellement de N-[3,5-diméthyl-4-[(nitrométhyl)thio]phényl] acétamide.
IR : $\bar{\nu}$ = 1666, 1599, 1554, 1544 $cm^{-1}$
RMN $^1H$ (DMSO $d_6$) : δ = 2,10 (3H,s) ; 2,46 (6H,s) ; 5,69 (2H,s) ; 7,47 (2H,s) ; 10,03 (1H, s, échangeable par $CF_3COOD$).

Etape 4 :

Une solution chauffée à 50°C de 18,6 g (118 mmoles) de permanganate de potassium dans 500 ml d'eau est additionnée rapidement à une solution de 10,0 g (39,3 mmoles) de N-[3,5-diméthyl-4-[(nitrométhyl)thio]phényl] acétamide dans 1 l d'acide acétique. Le milieu réactionnel est porté à 65°C pendant 30 min puis est ramené à température ambiante par un bain de glace. On ajoute alors une solution aqueuse saturée de métabisulfite de sodium jusqu'à décoloration (environ 25 ml). Le mélange est jeté dans 2,5 l d'eau et agité pendant 1 h. Le solide formé est séparé par filtration, rincé à l'eau et séché pour donner 6,1 g (54 %) d'un solide blanc fondant à 180-182°C, constitué essentiellement de N-[3,5-diméthyl-4-[(nitrométhyl)sulfonyl]phényl] acétamide que l'on utilise dans l'étape suivante sans autre purification.
IR : $\bar{\nu}$ = 1677, 1595, 1559, 1534, 1382, 1367, 1330, 1315, 1260, 1152, 871, 746, 639, 615 $cm^{-1}$
RMN $^1H$ (DMSO $d_6$) : δ = 2,25 (3H,s) ; 2,71 (6H,s) ; 6,64 (2H, s, échangeables par $CF_3COOD$) ; 7,68 (2H,s) ; 10,48 (1H, s, échangeable par $CF_3COOD$).

Etape 5 :

Un mélange de 13,1 g (45,8 mmoles) de N-[3,5-diméthyl-4-[(nitrométhyl)sulfonyl]phényl] acétamide et de 10,6 g (265 mmoles) de soude en pastilles dans 150 ml d'eau est porté à 80°C pendant 1 h. Après refroidissement, le milieu réactionnel est jeté dans 850 ml d'eau. L'acidification jusqu'à pH 5 par de l'acide acétique (environ 20 ml) provoque la précipitation d'un solide qui est isolé par filtration. Ce solide est lavé par 2 fois 50 ml d'eau et séché pour donner 10,6 g (95 %) d'un solide de couleur beige, fondant à 128-130°C, constitué essentiellement de 3,5-diméthyl-4-[(nitrométhyl)sulfonyl] aniline que l'on utilise dans l'étape suivante sans autre purification.
Un échantillon analytique est obtenu par recristallisation dans un mélange d'hexane et d'acétate d'éthyle.
F = 130 - 132°C

| Analyse centésimale : $C_9H_{12}N_2O_4S$ (M = 244,26) | | | | |
|---|---|---|---|---|
| | C% | H% | N% | S% |
| calculé | 44,25 | 4,95 | 11,47 | 13,13 |
| trouvé | 44,24 | 4,95 | 11,55 | 13,15 |

IR : $\bar{\nu}$ = 3472, 3374, 1630, 1600, 1550, 1341, 1317, 1154, 726 $cm^{-1}$
RMN $^1H$ (DMSO $d_6$) : δ = 2,40 (6H,s) ; 6,23 (4H, s, échangeables par $CF_3COOD$) ; 6,35 (2H,s).

**Exemple 2 (méthode A2)**

**N-[3,5-Diméthyl-4-[(nitrométhyl)sulfonyl]phényl] benzènesulfonamide**

A un mélange maintenu sous atmosphère d'azote de 1,0 g (4,09 mmoles) de 3,5-diméthyl-4-[(nitrométhyl) sulfonyl] aniline et de 0,65 g (8,22 mmoles) de pyridine séchée sur potasse, dans 19 ml de tétrahydrofurane anhydre,

on ajoute rapidement 1,08 g (6,11 mmoles) de chlorure de benzènesulfonyle. Le milieu réactionnel est agité pendant 2 h à température ambiante. Le lendemain, il est jeté dans un mélange d'eau et de glace et acidifié par de l'acide chlorhydrique N. Le mélange est alors extrait au chlorure de méthylène. Les extraits organiques rassemblés sont lavés à l'eau, séchés sur sulfate de sodium et concentrés à sec sous pression réduite.

Le résidu huileux obtenu est purifié par chromatographie sur colonne de silice (éluant : chlorure de méthylène) et cristallisation dans un mélange d'hexane et de chlorure de méthylène pour donner 1,1 g (70 %) de N-[3,5-diméthyl-4-[(nitrométhyl)sulfonyl]phényl] benzènesulfonamide en tout point identique au composé obtenu dans l'exemple 1.

**Exemple 3 (méthode D)**

**N-[3,5-Diméthyl-4-[(nitrométhyl)thio]phényl] benzènesulfonamide**

A une solution maintenue sous atmosphère d'azote de 0,80 g (3,77 mmoles) de 3,5-diméthyl-4-[(nitrométhyl) thio] aniline dans 30 ml de tétrahydrofurane anhydre, sont ajoutés 0,75 g (7,49 mmoles) de carbonate de calcium puis 1,0 g (5,66 mmoles) de chlorure de benzènesulfonyle. Après 3 jours d'agitation à température ambiante, le milieu réactionnel est jeté dans l'eau et extrait au chlorure de méthylène. Les extraits organiques rassemblés sont lavés à l'eau, séchés sur sulfate de sodium et concentrés à sec sous pression réduite. Le résidu obtenu est puritié par chromatographie sur colonne de silice (éluant : chlorure de méthylène) pour donner 0,9 g (68 %) de N-[3,5-diméthyl-4-[(nitrométhyl)thio]phényl] benzènesulfonamide.
F = 146 - 148°C (hexane-acétate d'éthyle)

| Analyse centésimale : $C_{15}H_{16}N_2O_4S_2$ (M = 352,41) | | | | |
|---|---|---|---|---|
| | C% | H% | N% | S% |
| calculé | 51,12 | 4,58 | 7,95 | 18,19 |
| trouvé | 50,95 | 4,54 | 8,03 | 18,05 |

IR : $\bar{\nu}$ = 3275, 1596, 1554, 1474, 1467, 1379, 1323, 1163, 1092, 872, 688 $cm^{-1}$
RMN $^1H$ (DMSO $d_6$) : δ = 2,26 (6H,s) ; 5,53 (2H, s) ; 6,85 (2H,s) ; 7,48-7,60 (3H,m) ; 7,74-7,77 (2H,m) ; 10,46 (1H, s, échangeable par $CF_3COOD$).

Le produit de départ, 3,5-diméthyl-4-[(nitrométhyl)thio] aniline, est obtenu selon le mode opératoire suivant :

Un mélange de 1,6 g (6,29 mmoles) de N-[3,5-diméthyl-4-[(nitrométhyl)thio]phényl] acétamide et de 1,44 g (36,0 mmoles) de soude en pastilles dans 20 ml d'eau est porté à 80°C pendant 1 h. Après refroidissement, le milieu réactionnel est jeté dans l'eau et extrait 2 fois à l'acétate d'éthyle. Les extraits organiques rassemblés sont séchés sur sulfate de sodium et concentrés à sec sous pression réduite. Le résidu obtenu est purifié par chromatographie sur colonne de silice (éluant : hexane / acétate d'éthyle : 2/1) pour donner 0,8 g (60 %) d'une huile constituée de 3,5-diméthyl-4-[(nitrométhyl)thio] aniline.
RMN $^1H$ (DMSO $d_6$) : δ = 2,31 (6H,s) ; 5,43 (2H, s, échangeables par $CF_3COOD$) ; 5,52 (2H,s) ; 6,39 (2H,s).

**Exemple 4 (méthode E)**

**N-[3,5-Diméthyl-4-[(nitrométhyl)sulfinyl]phényl] benzènesulfonamide**

A une solution de 2,2 g (6,24 mmoles) de N-[3,5-diméthyl-4-[(nitrométhyl)thio]phényl] benzènesulfonamide dans 79 ml de chloroforme anhydre, est ajouté rapidement 1,3 g (5,46 mmoles) d'acide m-chloroperbenzoïque 70-75 %. Le milieu réactionnel est agité pendant 24 h à température ambiante avant d'être versé dans une solution de 0,33 g (3,93 mmoles) de bicarbonate de soude. Le mélange est extrait au chlorure de méthylène. Les extraits organiques rassemblés sont séchés sur sulfate de sodium et concentrés à sec sous pression réduite. Le résidu obtenu est purifié par chromatographies successives sur colonne de silice (éluant de la première chromatographie : chlorure de méthylène ; éluant de la deuxième chromatographie : hexane / acétate d'éthyle : 2/1) et cristallisation dans l'hexane pour donner 0,98 g (42 %) de N-[3,5-diméthyl-4-[(nitrométhyl)sulfinyl]phényl] benzènesulfonamide.
F > 50°C (décomposition à 130°C)

| Analyse centésimale : $C_{15}H_{16}N_2O_5S_2$ (M = 368,41) | | | | |
|---|---|---|---|---|
| | C % | H % | N % | S % |
| calculé | 48,90 | 4,38 | 7,60 | 17,40 |

(suite)

| Analyse centésimale : $C_{15}H_{16}N_2O_5S_2$ (M = 368,41) | | | | |
|---|---|---|---|---|
| | C % | H % | N % | S % |
| trouvé | 48,96 | 4,66 | 7,47 | 17,58 |

IR: $\bar{\nu}$ = 1595, 1557, 1160, 1091, 1074, 602 $cm^{-1}$
RMN $^1H$ (DMSO $d_6$) : δ = 2,43 (6H,s) ; 6,14 (2H, s, échangeables par $CF_3COOD$) ; 6,88 (2H,s) ; 7,57-7,66 (3H,m) ; 7,85 - 7,88 (2H,m) ; 10,79 (1H, s, échangeable par $CF_3COOD$).

**Exemple 5 (méthode A1)**

**4-Chloro-N-[3,5-diméthyl-4-[(nitrométhyl)sulfonyl]phényl] benzènesulfonamide**

A un mélange de 1,8 g (7,37 mmoles) de 3,5-diméthyl-4-[(nitrométhyl)sulfonyl] aniline dans 45 ml de tétrahydrofurane anhydre, sont ajoutés successivement 1,45 g(14,5 mmoles) de carbonate de calcium, puis 2,3 g (10,9 mmoles) de chlorure de 4-chlorobenzènesulfonyle. Le milieu réactionnel est agité pendant 27 h à température ambiante avant d'être jeté dans 200 ml d'eau. On extrait 3 fois au chlorure de méthylène. Les extraits organiques rassemblés sont lavés à l'eau, séchés sur sulfate de sodium et concentrés à sec sous pression réduite. Le résidu obtenu est purifié par chromatographie sur colonne de silice (éluant : chlorure de méthylène) puis par recristallisation dans le chloroforme pour donner 0,44 g (14 %) de 4-chloro-N-[3,5-diméthyl-4-[(nitrométhyl)sulfonyl]phényl] benzènesulfonamide.
F = 160 - 162°C (chloroforme)

| Analyse centésimale : $C_{15}H_{15}ClN_2O_6S_2$ (M = 418,86) | | | |
|---|---|---|---|
| | C % | H % | N % |
| calculé | 43,01 | 3,61 | 6,69 |
| trouvé | 42,99 | 3,59 | 6,72 |

IR : $\bar{\nu}$ = 1594, 1562, 1346, 1167, 1147, 621 $cm^{-1}$
RMN $^1H$ (DMSO $d_6$) : δ = 2,48 (6H,s) ; 6,46 (2H, s, échangeables par $CF_3COOD$) ; 6,99 (2H,s) ; 7,67-7,72 (2H,m) ; 7,89 - 7,93 (2H,m) ; 11,18 (1H, s, échangeable par $CF_3COOD$).

**Exemple 6 (méthode A2)**

**N-[3,5-Diméthyl-4-[(nitrométhyl)sulfonyl]phényl]-3-méthyl-benzènesulfonamide**

A une solution maintenue sous atmosphère d'azote de 2,0 g (8,19 mmoles) de 3,5-diméthyl-4-[(nitrométhyl) sulfonyl] aniline dans 50 ml de tétrahydrofurane anhydre sont ajoutés successivement 1,3 ml (16,1 mmoles) de pyridine séchée sur potasse de 2,3 g (12,1 mmoles) de chlorure de 3-méthylbenzènesulfonyle. Le milieu réactionnel est agité pendant 24 h à température ambiante, puis est porté à 40°C pendant 1 h avant d'être jeté dans un mélange de 100 ml d'eau et de 100 g de glace. On acidifie jusqu'à pH 3 par addition d'acide chlorhydrique N et on extrait par 3 fois 100 ml de chlorure de méthylène. Les extraits organiques rassemblés sont lavés par 100 ml d'eau, séchés sur sulfate de sodium et concentrés à sec sous pression réduite. Le résidu obtenu est purifié par chromatographie sur colonne de silice (éluant : chlorure de méthylène) pour donner 2,3 g (71 %) de N-[3,5-diméthyl-4-[(nitrométhyl)sulfonyl]phényl]-3-méthyl-benzènesulfonamide.
F = 179 - 181° C (hexane-acétate d'éthyle)

| Analyse centésimale : $C_{16}H_{18}N_2O_6S_2$ (M = 398,44) | | | | |
|---|---|---|---|---|
| | C% | H % | N% | S% |
| calculé | 48,23 | 4,55 | 7,03 | 16,09 |
| trouvé | 48,21 | 4,61 | 7,06 | 15,99 |

IR : $\bar{\nu}$ = 3244, 1594, 1565, 1339, 1308, 1182, 1159, 1148, 607 $cm^{-1}$
RMN $^1H$ (DMSO $d_6$) : δ = 2,38 (3H,s) ; 2,48 (6H,s) ; 6,45 (2H, s, échangeables par $CF_3COOD$) ; 6,99 (2H,s) ; 7,48-7,53

(2H,m) ; 7,69 - 7,72 (2H, m) ; 11,05 (1H, s, échangeable par $CF_3COOD$).

**Exemple 7 (méthode A2)**

**N-[3,5-Diméthyl-4-[(nitrométhyl)sulfonyl]phényl]-4-méthylbenzènesulfonamide**

Le composé du titre est obtenu en opérant comme dans l'exemple 6 en utilisant 2,3 g (12,1 mmoles) de chlorure de 4-méthylbenzènesulfonyle.

Durée du chauffage à 40°C : 3 h
Rendement : 0,31 g (10 %)
F = 163 - 165°C (hexane-acétate d'éthyle)

| Analyse centésimale : $C_{16}H_{18}N_2O_6S_2$ (M = 398,44) | | | | |
|---|---|---|---|---|
| | C % | H % | N % | S % |
| calculé | 48,23 | 4,55 | 7,03 | 16,09 |
| trouvé | 48,27 | 4,66 | 6,97 | 15,83 |

IR : $\bar{\nu}$ = 3254, 1596, 1562, 1341, 1309, 1178, 1161, 1147, 1090, 1071, 664, 635, 599 $cm^{-1}$
RMN $^1H$ (DMSO $d_6$) : $\delta$ = 2,36 (3H,s) ; 2,47 (6H,s) ; 6,45 (2H, s, échangeables par $CF_3COOD$) : 6,98 (2H,s) ; 7,41 (2H, d, J = 8 Hz) ; 7,79 (2H, d, J = 8 Hz) ; 11,03 (1H, s, échangeable par $CF_3COOD$).

**Exemple 8 (méthode A2)**

**2,4-Difluoro-N-[3,5-diméthyl-4-[(nitrométhyl)sulfonyl]phényl] benzènesulfonamide**

A une solution refroidie vers 10°C de 1,0 g (4,09 mmoles) de 3,5-diméthyl-4-[(nitrométhyl)sulfonyl] aniline dans 20 ml de tétrahydrofurane anhydre, sont ajoutés successivement 0,65 g (8,22 mmoles) de pyridine séchée sur potasse et 1,3 g (6,11 mmoles) de chlorure de 2,4-difluorobenzènesulfonyle. Le milieu réactionnel est agité pendant 2 h à 10°C puis pendant 20 h à température ambiante avant d'être jeté dans 100 ml d'eau. Le mélange est amené à pH 3 par addition d'acide chlorhydrique concentré, avant d'être extrait au chlorure de méthylène. Les extraits organiques rassemblés sont lavés à l'eau, séchés sur sulfate de sodium et concentrés à sec sous pression réduite. Le résidu huileux obtenu est purifié par chromatographie sur colonne de silice (éluant : chlorure de méthylène) et par recristallisation dans le toluène pour donner 0,38 g (22 %) de 2,4-difluoro-N-[3,5-diméthyl-4-[(nitrométhyl)sulfonyl]phényl] benzènesulfonamide.
F = 154 - 156°C (toluène)

| Analyse centésimale : $C_{15}H_{14}F_2N_2O_6S_2$ (M = 420,40) | | | | | |
|---|---|---|---|---|---|
| | C% | H% | F% | N% | S% |
| calculé | 42,85 | 3,36 | 9,04 | 6,66 | 15,25 |
| trouvé | 42,81 | 3,41 | 8,80 | 6,69 | 15,26 |

IR : $\bar{\nu}$ = 3261, 1599, 1559, 1478, 1339, 1165, 1149, 1124, 1075, 866, 672 $cm^{-1}$
RMN $^1H$ (DMSO $d_6$) : $\delta$ = 2,48 (6H,s) ; 6,46 (2H, s, échangeables par $CF_3COOD$) ; 6,98 (2H,s) ; 7,31 - 7,37 (1H,m) ; 7,54 - 7,62 (1H,m) ; 8,09 - 8,17 (1H,m) ; 11,49 (1H, s, échangeable par $CF_3COOD$).

**Exemple 9 (méthode A2)**

**N-[3,5-Diméthyl-4-[(nitrométhyl)sulfonyl]phényl]-3-(trifluorométhyl)benzènesulfonamide**

Le composé du titre est obtenu en opérant comme dans l'exemple 6 en utilisant 3,0 g (12,3 mmoles) de chlorure de 3-(trifluorométhyl) benzènesulfonyle
Durée du chauffage à 40°C : 4 h
Rendement : 1,7 g (46 %)
F = 146 - 148°C (hexane-acétate d'éthyle)

| Analyse centésimale : $C_{16}H_{15}F_3N_2O_6S_2$ (M = 452,42) | | | | | |
|---|---|---|---|---|---|
| | C% | H% | F% | N% | S% |
| calculé | 42,47 | 3,34 | 12,60 | 6,19 | 14,17 |
| trouvé | 42,80 | 3,41 | 12,23 | 6,26 | 14,50 |

IR : $\bar{\nu}$ = 3244, 1593, 1569, 1359, 1344, 1326, 1182, 1167, 1148, 1139, 1104, 1071, 641 $cm^{-1}$
RMN $^1H$ (DMSO $d_6$) : $\delta$ = 2,48 (6H,s) ; 6,67 (2H, s, échangeables par $CF_3COOD$) ; 7,22 (2H,s) ; 8,09 (1H,t,J=7,8 Hz) ; 8,30 (1H,d, J = 8 Hz) ; 8,38 - 8,43 (1H,m) ; 10,44 (1H, s, échangeable par $CF_3COOD$).

**Exemple 10 (méthode A2)**

**3,4-Difluoro-N-[3,5-diméthyl-4-[(nitrométhyl)sulfonyl]phényl] benzènesulfonamide**

Le composé du titre est obtenu en opérant comme dans l'exemple 8 à partir d'une solution de 2,0 g (8,19 mmoles) de 3,5-diméthyl-4-[(nitrométhyl)sulfonyl] aniline dans 40 ml de tétrahydrofurane, de 1,3 g (16,4 mmoles) de pyridine et de 2,6 g (12,2 mmoles) de chlorure de 3,4-difluorobenzènesulfonyle. Le milieu réactionnel est agité successivement pendant 1 h à 10°C, 19 h à température ambiante, 3 h à 50°C et 16 h à température ambiante.

Rendement : 1,2 g (35 %)
F = 158 - 160°C (toluène)

| Analyse centésimale : $C_{15}H_{14}F_2N_2O_6S_2$ (M = 420,40) | | | | | |
|---|---|---|---|---|---|
| | C% | H% | F% | N% | S% |
| calculé | 42,85 | 3,36 | 9,04 | 6,66 | 15,25 |
| trouvé | 42,72 | 3,33 | 8,83 | 6,73 | 15,37 |

IR : $\bar{\nu}$ = 3273, 1600, 1556, 1511, 1362, 1345, 1329, 1277, 1148, 1120, 1069, 637 $cm^{-1}$
RMN $^1H$ (DMSO $d_6$) : $\delta$ = 2,37 (6H,s) ; 6,34 (2H, s, échangeables par $CF_3COOD$) 6,89 (2H,s) ; 7,56- 7,67 (2H,m) ; 7,86 - 7,92 (1H,m) ; 11,07 (1H, s, échangeable par $CF_3COOD$).

**Exemple 11 (méthode A2)**

**N-[3,5-Diméthyl-4-[(nitrométhyl)sulfonyl]phényl]-4-méthoxybenzènesulfonamide**

A une solution maintenue sous atmosphère d'azote de 2,0 g (8,19 mmoles) de 3,5-diméthyl-4-[(nitrométhyl) sulfonyl] aniline dans 40 ml de tétrahydrofurane anhydre sont ajoutés successivement 1,33 ml (16,4 mmoles) de pyridine séchée sur potasse et 2,54 g (12,3 mmoles) de chlorure de 4-méthoxybenzènesulfonyle. Le milieu réactionnel est agité pendant 24 h à température ambiante, puis est porté à 40°C pendant 1 h avant d'être jeté sur un mélange d'eau et de glace. La solution aqueuse obtenue est amenée à pH 1 par addition d'acide chlorhydrique 1N et est extraite au chlorure de méthylène.

Les extraits organiques rassemblés sont lavés à l'eau, séchés sur sulfate de sodium et concentrés à sec sous pression réduite. Le résidu huileux obtenu est purifié par chromatographie sur colonne de silice (éluant : chlorure de méthylène) et recristallisation dans un mélange d'hexane et d'acétate d'éthyle pour donner 1,1 g (33 %) de N-[3,5-diméthyl-4-[(nitrométhyl)sulfonyl]phényl]- 4-méthoxy-benzènesulfonamide.
F = 141 - 142°C (hexane - acétate d'éthyle)

| Analyse centésimale : $C_{16}H_{18}N_2O_7S_2$ (M = 414,44) | | | | |
|---|---|---|---|---|
| | C % | H% | N % | S% |
| calculé | 46,37 | 4,38 | 6,76 | 15,47 |
| trouvé | 46,40 | 4,38 | 6,89 | 15,22 |

IR : $\bar{\nu}$ = 3251, 1595, 1557, 1486, 1398, 1346, 1308, 1260, 1176, 1151, 1148, 1095, 1071, 874, 836, 599 $cm^{-1}$
RMN $^1H$ (DMSO $d_6$) : $\delta$ = 2,58 (6H,s) ; 3,93 (3H,s) ; 6,56 (2H, s, échangeables par $CF_3COOD$) ; 7,08 (2H,s) ; 7,20 - 7,25 (2H,m) ; 7,92 - 7,97 (2H,m) ; 11,07 (1H, s, échangeable par $CF_3COOD$).

**Exemple 12 (méthode A1)**

**4-Bromo-N-[3,5-diméthyl-4-[(nitrométhyl)sulfonyl]phényl]benzènesulfonamide**

A un mélange de 2,4 g (9,83 mmoles) de 3,5-diméthyl-4-[(nitrométhyl)sulfonyl] aniline dans 45 ml de tétrahydrofurane anhydre, sont ajoutés successivement 2,0 g (20,0 mmoles) de carbonate de calcium et 3,8 g (14,9 mmoles) de chlorure de 4-bromobenzènesulfonyle. Le milieu réactionnel est agité pendant 20 h à température ambiante, puis est porté à reflux pendant 48 h. Après refroidissement, il est jeté dans 400 ml d'eau et extrait au chlorure de méthylène. Les extraits organiques rassemblés sont séchés sur sulfate de sodium et concentrés à sec sous pression réduite. Le résidu huileux obtenu est purifié par chromatographie sur colonne de silice (éluant : chlorure de méthylène) pour donner 1,1 g (24 %) de 4-bromo-N-[3,5-diméthyl-4-[(nitrométhyl) sulfonyl]phényl] benzènesulfonamide.
F = 164 - 166°C (hexane - acétate d'éthyle)

| Analyse centésimale : $C_{15}H_{15}BrN_2O_6S_2$ (M = 463,32) | | | | | |
|---|---|---|---|---|---|
| | C% | H% | Br % | N% | S% |
| calculé<br>trouvé | 38,88<br>39,07 | 3,26<br>3,38 | 17,25<br>17,00 | 6,05<br>5,93 | 13,84<br>13,70 |

IR : $\bar{\nu}$ = 3258, 1594, 1568, 1471, 1393, 1342, 1166, 1148, 1088, 1069, 740, 632, 609 $cm^{-1}$
RMN $^1H$ (DMSO $d_6$) : δ = 2,64 (6H,s) ; 6,62 (2H, s, échangeables par $CF_3COOD$) ; 7,14 (2H,s) ; 7,99 (4H,s) ; 11,34 (1H, s, échangeable par $CF_3COOD$).

**Exemple 13 (méthode A1)**

**3-Bromo-N-[3,5-diméthyl-4-[(nitrométhyl)sulfonyl]phényl] benzènesulfonamide**

Le composé du titre est obtenu en opérant comme dans l'exemple 12 en utilisant 3,8 g (14,9 mmoies) de chlorure de 3-bromobenzènesulfonyle.
Durée du reflux : 40 h
Rendement: 0,8 g (18 %)
F = 156 - 158°C (hexane - acétate d'éthyle)

| Analyse centésimale : $C_{15}H_{15}BrN_2O_6S_2$ (M = 463,32) | | | | |
|---|---|---|---|---|
| | C % | H % | Br % | N % |
| calculé<br>trouvé | 38,88<br>39,17 | 3,26<br>3,45 | 17,25<br>17,00 | 6,05<br>5,91 |

IR : $\bar{\nu}$ = 3237, 1594, 1565, 1483, 1397, 1358, 1340, 1181, 1167, 1148, 1071, 885, 679, 635, 605 $cm^{-1}$
RMN $^1H$ (DMSO $d_6$) : δ = 2,64 (6H,s) ; 6,62 (2H, s, échangeables par $CF_3COOD$) ; 7,15 (2H,s) ; 7,70 - 7,75 (1H,m) ; 8,04 - 8,07 (2H,m) ; 8,18 - 8,19 (1H,m) ; 11,33 (1H, s, échangeable par $CF_3COOD$).

**Exemple 14 (méthode A2)**

**N-[3,5-Diméthyl-4-[(nitrométhyl)sulfonyl]phényl]-2-(trifluorométhyl) benzènesulfonamide**

Le composé du titre est obtenu en opérant comme dans l'exemple 6 à partir d'une solution de 2,0 g (8,19 mmoles) de 3,5-diméthyl-4-[(nitrométhyl)sulfonyl] aniline dans 38 ml de tétrahydrofurane, de 1,32 ml (16,3 mmoles) de pyridine et de 3,0 g (12,3 mmoles) de chlorure de 2-(trifluorométhyl) benzènesulfonyle.
Durée du chauffage à 40°C : 8 h
Rendement : 0,7 g (19 %)
F = 171 - 173°C (hexane - acétate d'éthyle)

| Analyse centésimale : $C_{16}H_{15}F_3N_2O_6S_2$ (M = 452,42) | | | | | |
|---|---|---|---|---|---|
| | C% | H% | F% | N% | S % |
| calculé | 42,47 | 3,34 | 12,60 | 6,19 | 14,17 |
| trouvé | 42,61 | 3,35 | 12,32 | 6,21 | 14,55 |

IR : $\bar{\nu}$ = 3370, 3028, 2954, 1596, 1562, 1405, 1348, 1340, 1307, 1180, 1166, 1149, 1122 $cm^{-1}$
RMN $^1H$ (DMSO $d_6$) : $\delta$ = 2,42 (6H,s) ; 6,41 (2H, s, échangeables par $CF_3COOD$) ; 6,93 (2H,s) ; 7,82 - 7,91 (2H,m) ; 7,96 - 8,01 (1H,m) ; 8,17 - 8,20 (1H,m) ; 11,38 (1H, s, échangeable par $CF_3COOD$).

**<u>Exemple 15</u> (méthode A2)**

**N-[3,5-Diméthyl-4-[(nitrométhyl)sulfonyl]phényl]-2-naphtalènesulfonamide**

A une solution maintenue sous atmosphère d'azote de 2,0 g (8,19 mmoles) de 3,5-diméthyl-4-[(nitrométhyl) sulfonyl] aniline dans 40 ml de tétrahydrofurane anhydre, sont ajoutés successivement 1,3 ml (16,1 mmoles) de pyridine séchée sur potasse et 2,8 g (12,4 mmoles) de chlorure de 2-naphtalènesulfonyle. Le milieu réactionnel est agité pendant 6 h à température ambiante, puis est abandonné pendant 2 jours avant d'être jeté dans 200 ml d'eau. Le mélange est amené à pH 1 par de l'acide chlorhydrique dilué et est extrait 2 fois au chlorure de méthylène. Les extraits organiques rassemblés sont lavés 2 fois à l'eau, séchés sur sulfate de sodium et concentrés à sec sous pression réduite.
Le résidu solide obtenu est purifié par chromatographie sur colonne de silice (éluant : chlorure de méthylène) pour donner 3,0 g (84 %) de N-[3,5-diméthyl-4-[(nitrométhyl)sulfonyl]phényl-2-naphtalènesulfonamide.
F = 208 - 215°C (acétate d'éthyle) - décomposition vers 215°C

| Analyse centésimale : $C_{19}H_{18}N_2O_6S_2$ (M = 434,47) | | | | |
|---|---|---|---|---|
| | C % | H % | N % | S % |
| calculé | 52,52 | 4,18 | 6,45 | 14,76 |
| trouvé | 52,62 | 4,29 | 6,45 | 14,42 |

IR : $\bar{\nu}$ = 3230, 1557, 1340, 1180, 1163, 1148, 1072, 659, 641 $cm^{-1}$
RMN $^1H$ (DMSO $d_6$) : $\delta$ = 2,54 (6H,s) ; 6,50 (2H, s, échangeables par $CF_3COOD$) ; 7,13 (2H,s) ; 7,76 - 7,81 (2H,m) ; 7,91 - 7,95 (1H,m) ; 8,10 - 8,33 (3H,m) ; 8,77 - 8,78 (1H,m) ; 11,30 (1H, s, échangeable par $CF_3COOD$).

**<u>Exemple 16</u> (méthode A2)**

**N-[3,5-Diméthyl-4-[(nitrométhyl)sulfonyl]phényl](2-nitrophényl) méthanesulfonamide**

Le composé du titre est obtenu en opérant comme dans l'exemple 15 en utilisant 2,9 g (12,3 mmoles) de chlorure de (2-nitrophényl)méthanesulfonyle.
Durée de l'agitation à température ambiante : 26 h
Rendement : 2,6 g (72 %)
F = 189 - 190°C (hexane-acétate d'éthyle)

| Analyse centésimale : $C_{16}H_{17}N_3O_8S_2$ (M = 443,44) | | | | |
|---|---|---|---|---|
| | C % | H % | N % | S % |
| calculé | 43,33 | 3,86 | 9,48 | 14,46 |
| trouvé | 43,67 | 3,82 | 9,60 | 14,32 |

IR : $\bar{\nu}$ = 3243, 1596, 1562, 1531, 1487, 1399, 1361, 1336, 1313, 1160, 1147, 1140, 902, 869, 632 $cm^{-1}$
RMN $^1H$ (DMSO $d_6$) : $\delta$ = 2,58 (6H,s) ; 5,21 (2H,s) ; 6,56 (2H, s, échangeables par $CF_3COOD$) ; 6,98 (2H,s) ; 7,61 - 7,64 (1H,m) ; 7,70 - 7,80 (2H,m) ; 8,10 - 8,13 (1H,m) ; 10,83 (1H, s, échangeable par $CF_3COOD$).

**Exemple 17 (méthode A2)**

**2-Bromo-N-[3,5-diméthyl-4-[(nitrométhyl)sulfonyl]phényl]benzènesulfonamide**

Le composé du titre est obtenu en opérant comme dans l'exemple 15 en utilisant 2,5 g (9,78 mmoles) de chlorure de 2-bromobenzènesulfonyle.

Le milieu réactionnel est agité 20 h à température ambiante avant l'addition de 0,6 g (2,35 mmoles) supplémentaire de chlorure de 2-bromobenzènesulfonyle et la poursuite de l'agitation pendant 4 h à température ambiante.
Rendement : 1,6 g (42 %)
F = 140 - 142°C (hexane-acétate d'éthyle)

| Analyse centésimale : $C_{15}H_{15}BrN_2O_6S_2$ (M = 463,32) | | | | |
|---|---|---|---|---|
| | C % | H % | Br % | N % |
| calculé | 38,88 | 3,26 | 17,25 | 6,05 |
| trouvé | 39,07 | 3,15 | 17,25 | 6,05 |

IR : $\bar{\nu}$ = 3295, 3041, 2969, 1595, 1562, 1478, 1337, 1192, 1188, 1144, 1071, 632; 601 $cm^{-1}$
RMN $^1H$ ($DMSO\ d_6$) : δ = 2,54 (6H,s) ; 6,54 (2H, s, échangeables par $CF_3COOD$) ; 7,03 (2H,s) ; 7,66 - 7,77 (2H,m) ; 7,93 - 7,96 (1H,m) ; 8,33 - 8,37 (1H,m) ; 11,53 (1H, s, échangeable par $CF_3COOD$).

**Exemple 18 (méthode A2)**

**N-[3,5-Diméthyl-4-[(nitrométhyl)sulfonyl]phényl]-4-(méthylsulfonyl)benzènesulfonamide**

A une solution maintenue sous atmosphère d'azote, de 1,6 g (6,55 mmoles) de 3,5-diméthyl-4-[(nitrométhyl) sulfonyl]aniline dans 50 ml de tétrahydrofurane anhydre, sont ajoutés successivement 1,31 ml (16,2 mmoles) de pyridine séchée sur potasse et 2,5 g (9,82 mmoles) de chlorure de 4-(méthylsulfonyl)benzènesulfonyle. Le milieu réactionnel est agité pendant 2 jours à température ambiante avant d'être jeté dans 200 ml d'eau. Le mélange obtenu est amené à pH 3 par addition d'acide chlorhydrique 1N, puis est extrait par 3 fois 200 ml d'acétate d'éthyle. Les extraits organiques rassemblés sont lavés à l'eau, séchés sur sulfate de sodium et concentrés à sec sous pression réduite. Le résidu est purifié par chromatographie sur colonne de silice (éluant: chlorure de méthylène/acétate d'éthyle : 2/1) et recristallisation dans un mélange d'hexane et d'acétate d'éthyie pour donner 0,78 g (26 %) de N-[3,5-diméthyl-4-[(nitrométhyl)sulfonyl]phényl]-4-(méthylsulfonyl)benzènesulfonamide.
F = 192 194°C (hexane acétate d'éthyle)

| Analyse centésimale : $C_{16}H_{18}N_2O_8S_3$ (M = 462,50) | | | | |
|---|---|---|---|---|
| | C % | H % | N % | S% |
| calculé | 41,55 | 3,92 | 6,06 | 20,80 |
| trouvé | 41,39 | 4,02 | 5,90 | 20,76 |

IR : $\bar{\nu}$ = 3281, 1597, 1586, 1555, 1485, 1401, 1395, 1366, 1349, 1328, 1306, 1294, 1289, 1165, 1150, 1090, 1072, 877, 860, 741, 624 $cm^{-1}$
RMN $^1H$ ($DMSO\ d_6$) : δ = 2,49 (6H,s) ; 3,30 (3H,s) ; 6,46 (2H, s, échangeables par $CF_3COOD$) ; 7,02 (2H,s) ; 8,16 (4H, s) ; 11,35 (1H, s, échangeable par $CF_3COOD$).

**Exemple 19 (méthode A2)**

**(E)-N-[3,5-Diméthyl-4-[(nitrométhyl)sulfonyl]phényl]-styrènesulfonamide**

Le composé du titre est obtenu en opérant comme dans l'exemple 18 à partir d'une solution de 2,0 g (8,19 mmoles) de 3,5-diméthyl-4-[(nitrométhyl)sulfonyl]aniline dans 50 ml de tétrahydrofurane, de 1,31 ml (16,2 mmoles) de pyridine et de 2,5 g (12,3 mmoles) de chlorure de trans-β-styrènesulfonyle.

Le milieu réactionnel est agité pendant 3 jours à température ambiante et l'extraction se fait au chlorure de méthylène.
Rendement : 1,5 g (45 %)

F = 174 - 176°C (hexane - acétate d'éthyle)

| Analyse centésimale : $C_{17}H_{18}N_2O_6S_2$ (M = 410,45) | | | | |
|---|---|---|---|---|
| | C% | H% | N% | S% |
| calculé | 49,74 | 4,42 | 6,83 | 15,62 |
| trouvé | 50,06 | 4,45 | 6,84 | 15,35 |

IR : $\bar{\nu}$ = 3235, 1610, 1595, 1577, 1555, 1475, 1390, 1357, 1345, 1323, 1164, 1141, 1067, 889, 872, 744, 627 $cm^{-1}$
RMN $^1H$ (DMSO $d_6$) : δ = 2,67 (6H,s) ; 6,64 (2H, s, échangeables par $CF_3COOD$) ; 7,22 (2H, s) ; 7,58 - 7,63 (4H,m) ; 7,87- 7,97 (3H,m) ; 11,04 (1H, s, échangeable par $CF_3COOD$).

**Exemple 20 (méthode C)**

**1-[3,5-Diméthyl-4-[(nitrométhyl)sulfonyl]phényl]-3-[(2-méthylphényl)sulfonyl]urée**

A une suspension maintenue sous atmosphère d'azote, de 1,3 g (5,32 mmoles) de 3,5-diméthyl-4-[(nitrométhyl)sulfonyl]aniline dans 25,6 ml de chlorure de méthylène anhydre, est ajoutée, goutte à goutte, une solution de 0,77 ml (5,09 mmoles) d'isocyanate d'o-toluènesulfonyle dans 7,7 ml de chlorure de méthylène anhydre. La température du milieu réactionnel s'élève spontanément de 7°C. Un précipité se forme dans la solution primitivement obtenue. L'agitation est poursuivie pendant 1 heure à température ambiante, puis le solide est isolé par filtration et lavé à l'hexane pour donner 1,9 g (Rendement = 86 %) de 1-[3,5-diméthyl-4-[(nitrométhyl)sulfonyl]phényl]-3-[(2-méthylphényl)sulfonyl] urée.
F = 194 - 196°C (hexane - acétate d'éthyle)

| Analyse centésimale : $C_{17}H_{19}N_3O_7S_2$ (M = 441,47) | | | | |
|---|---|---|---|---|
| | C % | H % | N % | S % |
| calculé | 46,25 | 4,34 | 9,52 | 14,52 |
| trouvé | 46,40 | 4,44 | 9,31 | 14,34 |

IR : $\bar{\nu}$ = 3303, 1654, 1587, 1565, 1526, 1455, 1393, 1362, 1341, 1326, 1164, 1153, 1131, 1089, 898, 883, 707, 606 $cm^{-1}$
RMN $^1H$ (DMSO $d_6$) : δ = 2,46 (6H,s) ; 2,58 (3H,s) ; 6,41 (2H, s, échangeables par $CF_3COOD$) ; 7,24 (2H,s) ; 7,38 - 7,43 (2H, m) ; 7,52 - 7,57 (1H,m) ; 7,94 (1H,d, J = 8Hz) ; 9,02 (1H, s, échangeable par $CF_3COOD$) ; 11,14 (1H, pic élargi échangeable par $CF_3COOD$).

**Exemple 21 (méthode C)**

**1-[3,5-Diméthyl-4-[(nitrométhyl)sulfonyl]phényl]-3-[(4-méthylphényl)sulfonyl]urée**

A une suspension maintenue sous atmosphère d'azote, de 2,6 g (10,6 mmoles) 3,5-diméthyl-4-[(nitrométhyl) sulfonyl]aniline dans 52 ml de chlorure de méthylène anhydre, est ajoutée, goutte à goutte, une solution de 1,55 ml (10,2 mmoles) d'isocyanate de p-toluènesulfonyle dans 15,5 ml de chlorure de méthylène anhydre. La température du milieu réactionnel s'élève spontanément de 2°C. L'agitation de la solution obtenue, est poursuivie pendant 30 minutes à température ambiante. Le milieu réactionnel est ensuite concentré à sec sous pression réduite ; le résidu est purifié par chromatographie sur colonne de silice (éluant : chlorure de méthylène, puis mélange chlorure de méthylène/ méthanol : 9/1) et cristallisation dans le chlorure de méthylène, pour donner 2,5 g (57 %) de 1-[3,5-diméthyl-4-[(nitrométhyl)sulfonyl]phényl]-3-[(4-méthylphényl)sulfonyl]urée.
F = 150 - 155°C

| Analyse centésimale : $C_{17}H_{19}N_3O_7S_2$ (M = 441,47) | | | | |
|---|---|---|---|---|
| | C % | H % | N % | S % |
| calculé | 46,25 | 4,34 | 9,52 | 14,52 |
| trouvé | 46,40 | 4,44 | 9,31 | 14,12 |

IR : $\bar{\nu}$ = 1594, 1562, 1337, 1263, 1247, 1159, 1136 $cm^{-1}$

RMN $^1H$ (DMSO $d_6$) : $\delta$ = 2,37 (3H,s) ; 2,55 (6H,s) ; 6,45 (2H, s, échangeables par $CF_3COOD$) ; 7,38 (2H,d, J = 8Hz) ; 7,42 (2H,s) ; 7,83 (2H,d, J = 8Hz) ; 9,12 (1H, s, échangeable par $CF_3COOD$).

**Exemple 22 (méthode A1)**

**N-[4-[3,5-Diméthyl-4-[(nitrométhyl)sulfonyl]phénylsulfamoyl]phényl]acétamide**

A un mélange maintenu sous atmosphère d'azote, de 2,0 g (8,19 mmoles) de 3,5-diméthyl-4-[(nitrométhyl) sulfonyl]aniline et de 1,6 g (16,0 mmoles) de carbonate de calcium dans 40 ml de tétrahydrafurane anhydre, sont ajoutés par petites fractions 2,9 g (12,4 mmoles) de chlorure de N-acétylsulfanilyle. Le milieu réactionnel est agité pendant 6 h à température ambiante, puis est porté à reflux pendant 2 h avant d'être jeté dans 200 ml d'eau. Le mélange est extrait 3 fois au chlorure de méthylène. Les extraits organiques rassemblés sont lavés à l'eau, séchés sur sulfate de sodium et concentrés à sec sous pression réduite. Le résidu huileux obtenu est cristallisé par trituration dans un mélange d'hexane et d'acétate d'éthyle. Ces cristaux sont lavés par un mélange de chlorure de méthylène et de méthanol à température ambiante, puis par un mélange d'hexane et d'acétate d'éthyle à reflux et enfin par de l'éthanol à température ambiante pour donner 0,26 g (7,2 %) de N-[4-[3,5-diméthyl-4-[(nitrométhyl)sulfonyl]phénylsulfamoyl] phényl]acétamide.
F = 209 - 210°C.

| Analyse centésimale : $C_{17}H_{19}N_3O_7S_2$ (M = 441,47) | | | | |
|---|---|---|---|---|
| | C % | H % | N % | S % |
| calculé | 46,25 | 4,34 | 9,52 | 14,52 |
| trouvé | 46,25 | 4,43 | 9,78 | 14,25 |

IR : $\bar{\nu}$ = 3382, 3190, 1676, 1592, 1550, 1536, 1340, 1156 1147 $cm^{-1}$
RMN $^1H$ (DMSO $d_6$) : $\delta$ = 2,10 (3H,s) ; 2,51 (6H,s) ; 6,48 (2H, s, échangeables par $CF_3COOD$) ; 7,01 (2H,s) ; 7,78 - 7,81 (2H,m) ; 7,86 - 7,89 (2H, m) ; 10,39 (1H, s, échangeable par $CF_3COOD$) ; 11,03 (1H, s, échangeable par $CF_3COOD$).

**Exemple 23 (méthode A2)**

**N-[3,5-Diméthyl-4-[(nitrométhyl)sulfonyl]phényl]-2,2,2-trifluoroéthanesulfonamide**

Le composé du titre est obtenu en opérant comme dans l'exemple 18, à partir d'une solution de 1,5 g (6,14 mmoles) de 3,5-diméthyl-4-[(nitrométhyl)sulfonyl]aniline dans 50 ml de tétrahydrofurane, de 0,97 ml (12,0 mmoles) de pyridine et de 1,0 ml (9,04 mmoles) de chlorure de 2,2,2-trifluoroéthanesulfonyle.
L'éluant de chromatographie est le chlorure de méthylène.
Rendement : 1,6 g (68 %).
F = 168 - 170°C (hexane - acétate d'éthyle)

| Analyse centésimale : $C_{11}H_{13}F_3N_2O_6S_2$ (M = 390,35) | | | | | |
|---|---|---|---|---|---|
| | C % | H % | F % | N % | S % |
| calculé | 33,84 | 3,36 | 14,60 | 7,18 | 16,43 |
| trouvé | 34,14 | 3,44 | 14,25 | 7,35 | 16,19 |

IR : $\bar{\nu}$ = 3227, 1601, 1564, 1351, 1333, 1323, 1270 1248, 1157, 1147, 1135, 1089 $cm^{-1}$
RMN $^1H$ (DMSO $d_6$) : $\delta$ = 2,44 (6H,s) ; 4,73 (2H,q, J = 10Hz) ; 6,39 (2H, s, échangeables par $CF_3COOOD$) ; 6,96 (2H, s) ; 11,04 (1H, s, échangeable par $CF_3COOD$).

**Exemple 24 (méthode A1)**

**N-[3,5-Diméthyl-4-[(nitrométhyl)sulfonyl]phényl]-4-fluorobenzènesulfonamide**

A une solution de 2,0 g (8,19 mmoles) de 3,5-diméthyl-4-[(nitrométhyl)sulfonyl]aniline dans 40 ml de tétrahydrofurane anhydre, sont ajoutés 1,65 g (16,5 mmoles) de carbonate de calcium, puis, par petites fractions, 2,4 g (12,3

mmoles) de chlorure de 4-fluorobenzènesulfonyle. Le milieu réactionnel est porté à reflux pendant 50 heures. Après refroidissement, il est jeté dans 200 ml d'eau et extrait par 3 fois 100 ml de chlorure de méthylène. Les extraits organiques rassemblés sont lavés par 100 ml d'eau, séchés sur sulfate de sodium et concentrés à sec sous pression réduite. Le résidu huileux obtenu est purifié par chromatographie sur colonne de silice (éluant : chlorure de méthylène) et recristallisation dans un mélange d'hexane et d'acétate d'éthyle pour donner 1,2 g (36 %) de N-[3,5-diméthyl-4-[(nitrométhyl)sulfonyl]phényl]-4-fluoro-benzènesulfonamide.
F = 152 - 154°C (hexane - acétate d'éthyle)

| Analyse centésimale : $C_{15}H_{15}FN_2O_6S_2$ (M = 402,41) | | | | | |
|---|---|---|---|---|---|
| | C % | H % | F % | N % | S % |
| calculé | 44,77 | 3,76 | 4,72 | 6,96 | 15,93 |
| trouvé | 44,76 | 3,85 | 4,43 | 6,93 | 15,84 |

IR : $\bar{\nu}$ = 1592, 1563, 1478, 1398, 1362, 1341, 1177, 1169, 1159, 1146, 1090, 1071, 877, 865, 841, 666, 635 $cm^{-1}$
RMN $^1H$ (DMSO $d_6$) : δ = 2,68 (6H,s) ; 6,48 (2H, s, échangeables par $CF_3COOD$) ; 7,18 (2H,s) ; 7,61 - 7,69 (2H,m) ; 8,13 - 8,19 (2H,m) ; 11,31 (1H, s, échangeable par $CF_3COOD$).

**Exemple 25 (méthode A2)**

**N-[3,5-Diméthyl-4-[(nitrométhyl)sulfonyl]phényl]-3-fluorobenzènesulfonamide**

Le composé du titre est obtenu comme dans l'exemple 6 à partir d'une solution de 2,0 g (8,19 mmoles) de 3,5-diméthyl-4-[(nitrométhyl)sulfonyl]aniline dans 40 ml de tétrahydrofurane, de 1,29 g (16,3 mmoles) de pyridine et de 2,4 g (12,3 mmoles) de chlorure de 3-fluorobenzènesulfonyle.

Le milieu réactionnel est agité pendant 2 h à température ambiante, puis pendant 6 h à 40°C.
Rendement : 1,3 g (40 %).
F = 135 - 137°C (hexane - acétate d'éthyle)

| Analyse centésimale : $C_{15}H_{15}FN_2O_6S_2$ (M = 402,41) | | | | | |
|---|---|---|---|---|---|
| | C% | H% | F% | N% | S % |
| calculé | 44,77 | 3,76 | 4,72 | 6,96 | 15,93 |
| trouvé | 44,62 | 3,63 | 4,65 | 6,91 | 16,21 |

IR : $\bar{\nu}$ = 3263, 1597, 1557, 1551, 1479, 1395, 1357, 1343, 1320, 1307, 1230, 1168, 1146, 1072, 856, 695, 676, 636, 610 $cm^{-1}$
RMN $^1H$ (DMSO $d_6$) : δ = 2,45 (6H,s) ; 6,43 (2H, s, échangeables par $CF_3COOD$) ; 6,97 (2H,s) ; 7,50 - 7,73 (4H,m) ; 11,16 (1H, s, échangeable par $CF_3COOD$)

**Exemple 26 (méthode A2)**

**N-[3,5-Diméthyl-4-[(nitrométhyl)sulfonyl]phényl]phénylméthane sulfonamide**

Le composé du titre est obtenu en opérant comme dans l'exemple 11, à partir d'une solution de 3,0 g (12,3 mmoles) de 3,5-diméthyl-4-[(nitrométhyl)sulfonyl]aniline dans 57 ml de tétrahydrofurane, de 2,0 ml (24,7 mmoles) de pyridine et de 3,5 g (18,4 mmoles) de chlorure de phénylméthanesulfonyle.

Le milieu réactionnel est agité pendant 20 h à température ambiante, puis pendant 5 h à 40°C. Le produit final est cristallisé dans un mélange d'hexane et de chlorure de méthylène.
Rendement : 0,42 g (8,6 %)
F = 124 - 126°C (hexane - chlorure de méthylène)

| Analyse centésimale : $C_{16}H_{18}N_2O_6S_2$ (M = 398,44) | | | | |
|---|---|---|---|---|
| | C % | H % | N % | S % |
| calculé | 48,23 | 4,55 | 7,03 | 16,09 |
| trouvé | 48,49 | 4,59 | 7,03 | 16,04 |

IR : $\bar{\nu}$ = 3256, 1596, 1566, 1488, 1405, 1359, 1337, 1325, 1312, 1144, 1071, 697, 638 cm-1
RMN $^1H$ (DMSO $d_6$) : $\delta$ = 2,72 (6H,s) ; 4,90 (2H,s) ; 6,69 (2H, s, échangeables par $CF_3COOD$) ; 7,14 (2h,s) ; 7,49 - 7,58 (5H,m) ; 10,70 (1H, s, échangeable par $CF_3COOD$)

**Exemple 27 (méthode A2)**

**4-Bromo-2,5-difluoro-N-[3,5-diméthyl-4-[(nitrométhyl)sulfonyl]phényl]benzènesulfonamide**

A une solution maintenue sous atmosphère d'azote, de 2,0 g (8,19 mmoles) de 3,5-diméthyl-4-[(nitrométhyl) sulfonyl]aniline dans 40 ml de tétrahydrofurane anhydre, sont ajoutés 1,3 g (16,4 mmoles) de pyridine séchée sur potasse puis 3,6 g (12,3 mmoles) de chlorure de 4-bromo-2,5-difluorobenzènesulfonyle. Le milieu réactionnel est agité pendant 7 h 30 à température ambiante, puis pendant 11 h à reflux ; après refroidissement, il est jeté dans 300 ml d'eau et extrait 3 fois au chlorure de méthylène. Les extraits organiques rassemblés sont lavés à l'eau, séchés sur sulfate de sodium et concentrés à sec sous pression réduite. Le résidu solide obtenu est purifié par chromatographie sur colonne de silice (éluant : chlorure de méthylène) et recristallisation dans un mélange d'hexane et d'acétate d'éthyle pour donner 0,24 g (6,2 %) de 4-bromo-2,5-difluoro-N-[3,5-diméthyl-4-[(nitrométhyl)sulfonyl]phényl]benzènesulfonamide, contenant 5 % molaire d'acétate d'éthyle.
F = 167 - 169°C (hexane - acétate d'éthyle)

| Analyse centésimale : $C_{15}H_{13}BrF_2N_2O_6S_2$ (M = 499,31), 5 % $C_4H_8O_2$ | | | | | | |
|---|---|---|---|---|---|---|
| | C% | H% | Br % | F% | N% | S % |
| calculé | 36,24 | 2,68 | 15,86 | 7,54 | 5,56 | 12,73 |
| trouvé | 36,50 | 2,75 | 15,47 | 7,39 | 5,51 | 12,80 |

IR : $\bar{\nu}$ = 3242, 1565, 1480, 1350, 1167 $cm^{-1}$
RMN $^1H$ (DMSO $d_6$) : $\delta$ = 2,42 (6H,s) ; 6,39 (2H, s, échangeables par $CF_3COOD$) ; 6,92 (2H,s) ; 7,97 - 8,03 (2H,m) ; 11,55 (1H, s, échangeable par $CF_3COOD$)

**Exemple 28 (méthode A2)**

**2,5-Difluoro-N-[3,5-diméthyl-4-[(nitrométhyl)sulfonyl]phényl]benzènesuifonamide**

Le composé du titre est obtenu en opérant comme dans l'exemple 15 en utilisant 2,6 g (12,2 mmoles) de chlorure de 2,5-difluorobenzènesulfonyle.
Le milieu réactionnel est agité pendant 20 h à température ambiante et est abandonné pendant 5 jours avant traitement. Le produit final est purifié par chromatographie sur colonne de silice (éluant : chlorure de méthylène) et recristallisation dans le toluène.
Rendement : 1,1 g (32 %)
F = 132 - 134°C (toluène)

| Analyse centésimale : $C_{15}H_{14}F_2N_2O_6S_2$ (M = 420,40) | | | | | |
|---|---|---|---|---|---|
| | C% | H% | F% | N% | S% |
| calculé | 42,85 | 3,36 | 9,04 | 6,66 | 15,25 |
| trouvé | 43,09 | 3,43 | 9,05 | 6,69 | 15,40 |

IR : $\bar{\nu}$ = 1562, 1486, 1353, 1159, 1147, 607 $cm^{-1}$
RMN $^1H$ (DMSO $d_6$) : $\delta$ = 2,47 (6H,s) ; 6,44 (2H, s, échangeables par $CF_3COOD$) ; 6,99 (2H,s) ; 7,49 - 7,57 (1H,m) ; 7,60 - 7,64 (1H,m) ; 7,86 - 7,92 (1H,m)
La matière première, chlorure de 2,5-difluorobenzènesulfonyle, est obtenue selon le mode opératoire suivant :
A un mélange refroidi en dessous de -10°C par un bain de carboglace dans l'éthanol, de 33 ml d'acide chlorhydrique concentré et de 10 ml d'acide acétique, sont ajoutés rapidement 12,9 g (99,9 mmoles) de 2,5-difluoroaniline, puis, goutte à goutte, une solution de 7,4 g (107 mmoles) de nitrite de sodium dans 15 ml d'eau. L'agitation est poursuivie 45 min après la fin de l'addition, à une température comprise entre -10 et -25°C.
Cette solution maintenue vers -25°C, est ajoutée par fractions à un mélange, refroidi à 10°C par un bain de glace, obtenu par saturation de 100 ml d'acide acétique par de l'anhydride sulfureux et addition de 2,5 g (25,3 mmoles)

de chlorure cuivreux. Il se produit un important dégagement d'azote. Le milieu réactionnel est laissé revenir à température ambiante et maintenu ainsi pendant 2 h, avant d'être jeté dans 450 ml d'un mélange d'eau et de glace ; il est extrait à l'éther éthylique. Les extraits éthérés rassemblés sont lavés par une solution aqueuse saturée de bicarbonate de soude, séchés sur sulfate de sodium et concentrés. Le résidu huileux obtenu est distillé sous pression réduite pour donner 14,3 g (87 %) d'un liquide constitué essentiellement de chlorure de 2,5-difluorobenzènesulfonyle que l'on utilise dans l'étape suivante sans autre purification.
Eb = 65 - 70°C sous 0,5 mm de mercure.
IR: $\bar{\nu}$ = 3118, 3087, 1488, 1410, 1384, 1301, 1255, 1202, 1188, 1152, 1119, 1052, 881, 832, 772, 692, 689, 605, 599 $cm^{-1}$
RMN $^1H$ ($CDCl_3$) : $\delta$ = 7,28 - 7,32 (1H,m) ; 7,38 - 7,42 (1H,m) ; 7,58 - 7,63 (1H,m)

**<u>Exemple 29</u> (méthode A2)**

**2,3-Difluoro-N-[3,5-diméthyl-4-[(nitrométhyl)sulfonyl]phényl]benzènesulfonamide**

Le composé du titre est obtenu en opérant comme dans l'exemple 15 en utilisant 2,6 g (12,2 mmoles) de chlorure de 2,3-difluorobenzènesulfonyle.
Le milieu réactionnel est agité pendant 20 h à température ambiante, à l'abri de la lumière et abandonné pendant 3 jours, avant traitement. Le produit final est purifié par chromatographie sur colonne de silice (éluant : chlorure de méthylène) et recristallisation dans le toluène.
Rendement : 1,1 g (32 %)
F = 166 - 168°C (toluène)

| Analyse centésimale : $C_{15}H_{14}F_2N_2O_6S_2$ (M = 420,40) | | | | | |
|---|---|---|---|---|---|
| | C% | H% | F % | N % | S% |
| calculé | 42,85 | 3,36 | 9,04 | 6,66 | 15,25 |
| trouvé | 43,07 | 3,51 | 8,98 | 6,61 | 15,01 |

IR : $\bar{\nu}$ = 3292, 1600, 1557, 1494, 1363, 1348, 1329, 1276, 1199, 1161, 1140, 636 $cm^{-1}$
RMN $^1H$ (DMSO $d_6$) : $\delta$ = 2,29 (6H,s) ; 6,28 (2H, s, échangeables par $CF_3COOD$) ; 6,81 (2H,s) ; 7,24 - 7,29 (1H,m) ; 7,61 - 7,67 (2H,m) ; 11,44 (1H, s, échangeable par $CF_3COOD$).
La matière première, chlorure de 2,3-difluorobenzenesulfonyle, est obtenue selon le mode opératoire décrit dans l'exemple 28, en utilisant d'une part 22 ml d'acide chlorhydrique concentré, 7,7 ml d'acide acétique, 10,0 g (77,5 mmoles) de 2,3-difluoroaniline et 5,8 g (84,1 mmoles) de nitrite de sodium dans 12 ml d'eau et d'autre part, 77 ml d'acide acétique saturé par de l'anhydride sulfureux et 1,9 g (19,2 mmoles) de chlorure cuivreux.
Rendement : 8,5 g (52 %)
Eb = 60 - 80°C sous 0,5 mm de mercure
IR : $\bar{\nu}$ = 1606, 1493, 1388, 1280, 1238, 1203, 1171, 1148, 907, 824, 789, 711, 650 $cm^{-1}$
RMN $^1H$ ($CDCl_3$) : $\delta$ = 7,37 - 7,42 (1H,m) ; 7,61 - 7,65 (1H,m) ; 7,76 - 7,81 (1H,m)

**<u>Exemple 30</u> (méthode A2)**

**3,5-Difluoro-N-[3,5-diméthyl-4-[(nitrométhyl)sulfonyl]phényl]benzènesulfonamide**

Le composé du titre est obtenu en opérant comme dans l'exemple 15 en utilisant 2,6 g (12,2 mmoles) de chlorure de 3,5-difluorobenzènesulfonyle que l'on additionne en solution dans 20 ml de tétrahydrofurane.
Le milieu réactionnel est agité pendant 20 h à température ambiante, à l'abri de la lumière et est abandonné pendant 3 jours, avant traitement. Le produit final est purifié par chromatographie sur colonne de silice (éluant : chlorure de méthylène) et recristallisation dans le toluène.
Rendement : 1,8 g (52 %)
F = 187 - 190°C (tolène)

| Analyse centésimale : $C_{15}H_{14}F_2N_2O_6S_2$ (M = 420,40) | | | | | |
|---|---|---|---|---|---|
| | C % | H % | F % | N % | S % |
| calculé | 42,85 | 3,36 | 9,04 | 6,66 | 15,25 |
| trouvé | 43,21 | 3,44 | 8,85 | 6,50 | 15,01 |

IR : $\bar{\nu}$ = 3343, 2979, 1607, 1595, 1555, 1474, 1446, 1401, 1363, 1346, 1316, 1295, 1252, 1196, 1160, 1127, 1070, 993, 801, 668, 637, 616 $cm^{-1}$
RMN $^1H$ (DMSO $d_6$) : $\delta$ = 2,45 (6H,s) ; 6,42 (2H, s, échangeables par $CF_3COOD$) ; 6,97 (2H,s) ; 7,57 - 7,64 (3H,m) ; 11,21 (1H, s, échangeable par $CF_3COOD$) La matière première, chlorure de 3,5-difluorobenzènesulfonyle, est obtenue selon le mode opératoire décrit dans l'exemple 28, en utilisant d'une part 22 ml d'acide chlorhydrique concentré, 7,7 ml d'acide acétique, 10,0 g (77,5 mmoles) de 3,5-difluoroaniline et 5,8 g (84,1 mmoles) de nitrite de sodium dans 15 ml d'eau ; et d'autre part, 77 ml d'acide acétique saturé par de l'anhydride sulfureux et 1,9 g (19,2 mmoles) de chlorure cuivreux.
Rendement : 7,1 g (43 %) - solide blanc
Eb = 60 - 120°C sous 0,9 mm de mercure
IR : $\bar{\nu}$ = 3100, 1608, 1592, 1446, 1375, 1361, 1301, 1178, 1133, 1080, 992, 881, 866, 662, 611 $cm^{-1}$
RMN $^1H$ ($CDCl_3$) : $\delta$ = 7,04 - 7,11 (1H,m) ; 7,46 - 7,48 (2H,m)

**<u>Exemple 31</u> (méthode A2)**

**N-[3,5-Diméthyl-4-[(nitrométhyl)sulfonyl]phényl]-1-napthalènesulfonamide**

Le composé du titre est obtenu en opérant comme dans l'exemple 15 en utilisant 2,8 g (12,4 mmoles) de chlorure de 1-napthalènesulfonyle.

Le milieu réactionnel est agité pendant 26 h à température ambiante. Le produit final est purifié par chromatographie sur colonne de silice (éluant : chlorure de méthylène) et recristallisation dans le toluène.
Rendement : 1,4 g (39 %)
F = 170- 172°C (toluène)

| Analyse centésimale : $C_{19}H_{18}N_2O_6S_2$ (M = 434,47) | | | | |
|---|---|---|---|---|
| | C % | H % | N % | S % |
| calculé | 52,52 | 4,18 | 6,45 | 14,76 |
| trouvé | 52,65 | 3,89 | 6,51 | 14,72 |

IR : $\bar{\nu}$ = 3267, 1595, 1560, 1330, 1181, 1163, 1136, 1070, 771, 636, 630, 600 $cm^{-1}$
RMN $^1H$ (DMSO $d_6$) : $\delta$ = 2,44 (6H,s) ; 6,42 (2H, s, échangeables par $CF_3COOD$) ; 6,95 (2H,s) ; 7,68 - 7,83 (3H,m) ; 8,13 (1H,d, J = 8,0 Hz) ; 8,31 (1H,d, J = 8,2 Hz) ; 8,46 (1H,d, J = 7,3 Hz) ; 8,70 (1H,d, J = 8,5 Hz) ; 11,53 (1H, s, échangeable par $CF_3COOD$)

**<u>Exemple 32</u> (méthode A2)**

**N-[3,5-Diméthyl-4-[(nitrométhyl)sulfonyl]phényl]-2-thiophènesulfonamide**

Le composé du titre est obtenu en opérant comme dans l'exemple 6 à partir d'un mélange de 3,0 g (12,3 mmoles) de 3,5-diméthyl-4-[(nitrométhyl)sulfonyl]aniline, de 60 ml de tétrahydrofurane et de 1,95 ml (24,1 mmoles) de pyridine auquel on ajoute rapidement 2,8 g (15,3 mmoles) de chlorure de 2-thiophènesulfonyle.

Le milieu réactionnei est agité pendant 24 h à température ambiante et chauffé à 50°C pendant 4 h. Le produit final est purifié par chromatographie sur colonne de silice (éluant : chlorure de méthylène) et recristallisation dans le toluène.
Rendement : 0,8 g (17 %)
F = 158 - 159°C (toluène)

| Analyse centésimale : $C_{13}H_{14}N_2O_6S_3$ (M = 390,44) | | | | |
|---|---|---|---|---|
| | C % | H % | N % | S % |
| calculé | 39,99 | 3,61 | 7,18 | 24,63 |
| trouvé | 40,07 | 3,68 | 7,29 | 24,46 |

IR : $\bar{\nu}$ = 1598, 1552, 1345, 1151, 600 $cm^{-1}$
RMN $^1H$ (DMSO $d_6$) : $\delta$ = 2,36 (6H,s) ; 6,33 (2H, s, échangeables par $CF_3COOD$) ; 6,90 (2H,s) ; 7,02 - 7,04 (1H,m) ; 7,63 - 7,65 (1H,m) ; 7,83 - 7,85 (1H,m) ; 11,09 (1H, s, échangeable par $CF_3COOD$)

**Exemple 33 (méthode B)**

**N-[4-[(Dichloronitrométhyl)sulfonyl]-3,5-diméthyl-phényl]benzènesulfonamide**

Un mélange de 0,96 g (2,50 mmoles) de N-[3,5-diméthyl-4-[(nitrométhyl)sulfonyl]phényl]benzène sulfonamide, de 0,66 g (4,94 mmoles) de N-chlorosuccinimide et de 10 mg (0,06 mmoles) de 2,2'-azobisisobutyronitrile dans 20 ml de tétrachlorure de carbone est porté à reflux pendant 6 h. Après refroidissement, la partie insoluble est éliminée par filtration et la solution est concentrée à sec sous pression réduite. Le résidu pâteux obtenu est purifié par chromatographie sur colonne de silice (éluant : chlorure de méthylène) et cristallisation dans un mélange d'hexane et de chlorure de méthylène pour donner 0,28 g (25 %) de N-[4-[(dichloronitrométhyl)sulfonyl]-3,5-diméthyl-phényl]benzènesulfonamide.
F = 112 - 114°C (hexane - chlorure de méthylène)

| Analyse centésimale : $C_{15}H_{14}Cl_2N_2O_6S_2$ (M = 453,31) | | | | | |
|---|---|---|---|---|---|
| | C % | H % | Cl % | N % | S % |
| calculé | 39,74 | 3,11 | 15,64 | 6,18 | 14,14 |
| trouvé | 40,24 | 3,20 | 15,71 | 6,29 | 13,70 |

IR : $\bar{\nu}$ = 3218, 1580, 1466, 1360, 1329, 1310, 1186, 1161, 1091, 748, 618, 609, 601 $cm^{-1}$
RMN $^1H$ (DMSO $d_6$) : δ = 2,38 (6H,s) ; 7,00 (2H,s) ; 7,49 - 7,59 (3H,m) ; 7,81 - 7,84 (2H,m) ; 11,29 (1H, s, échangeable par $CF_3COOD$)

**Exemple 34 (méthode A2)**

**N-[3,5-Diméthyl-4-[(nitrométhyl)sulfonyl]phényl]-2-fluorobenzènesulfonamide**

A une solution maintenue sous atmosphère d'azote, de 2,0 g (8,19 mmoles) de 3,5-diméthyl-4-[(nitrométhyl) sulfonyl]aniline dans 40 ml de tétrahydrofurane anhydre, sont ajoutés successivement et rapidement 1,29 g (16,3 mmoles) de pyridine séchée sur potasse et 2,4 g (12,3 mmoles) de chlorure de 2-fluorobenzènesulfonyle. Le mélange est agité pendant 2 h à température ambiante, puis est porté à 40°C pendant 12 h. Après refroidissement, le milieu réactionnel est jeté dans un mélange de 100 ml d'eau et 100 g de glace, est acidifié par de l'acide chlorhydrique N jusqu'à pH 2, puis est extrait par 3 fois 100 ml de chlorure de méthylène. Les extraits organiques rassemblés sont lavés par 100 ml d'eau, séchés sur sulfate de sodium et concentrés à sec sous pression réduite. Le résidu pâteux obtenu est purifié par chromatographie sur colonne de silice (éluant : chlorure de méthylène) et recristallisation dans un mélange d'hexane et d'acétate d'éthyle pour donner 1,5 g (Rdt = 46 %) de N-[3,5-diméthyl-4-[(nitrométhyl)sulfonyl] phényl]-2-fluoro-benzènesulfonamide.
F = 175 - 177°C (hexane - acétate d'éthyle)

| Analyse centésimale : $C_{15}H_{15}FN_2O_6S_2$ (M = 402,41) | | | | | |
|---|---|---|---|---|---|
| | C% | H% | F% | N% | S % |
| calculé | 44,77 | 3,76 | 4,72 | 6,96 | 15,93 |
| trouvé | 44,99 | 3,61 | 4,66 | 6,95 | 15,81 |

IR : $\bar{\nu}$ = 3236, 1598, 1583, 1557, 1475, 1390, 1350, 1329, 1191, 1175, 1160, 1149, 1124, 1077, 1068, 890, 881, 875, 778, 623, 600 $cm^{-1}$
RMN $^1H$ (DMSO $d_6$) : δ = 2,56 (6H,s) ; 6,55 (2H, s, échangeables par $CF_3COOD$) ; 7,08 (2H,s) ; 7,51 - 7,56 (2H,m) ; 7,84 - 7,87 (1H,m) ; 8,11 - 8,16 (1H,m) ; 11,55 (1H, s, échangeable par $CF_3COOD$)

**Exempte 35 (méthode A2)**

**N-[3,5-Diméthyl-4-[(nitrométhyl)sulfonyl]phényl]-4-(trifluorométhyl)benzènesulfonamide**

Le composé du titre est obtenu en opérant comme dans l'exemple 34 en utilisant 3,0 g (12,3 mmoles) de chlorure de 4-(trifluorométhyl)benzènesulfonyle.
Le milieu réactionnel est agité pendant 2 h à température ambiante puis est chauffé à 40°C pendant 20 h.

Rendement : 1,5 g (35 %)
F = 187 - 188°C (hexane - acétate d'éthyle)

| Analyse centésimale : $C_{16}H_{15}F_3N_2O_6S_2$ (M = 452,42) | | | | | |
|---|---|---|---|---|---|
| | C % | H % | F % | N % | S % |
| calculé | 42,47 | 3,34 | 12,60 | 6,19 | 14,17 |
| trouvé | 42,73 | 3,39 | 12,42 | 6,13 | 14,00 |

IR : $\bar{\nu}$ = 3252, 1596, 1563, 1408, 1347, 1324, 1166, 1140, 1131, 1092, 1062, 751, 638 $cm^{-1}$
RMN $^1H$ (DMSO $d_6$) : δ = 2,42 (6H,s) ; 6,40 (2H, s, échangeables par $CF_3COOD$) ; 6,95 (2H,s) ; 7,95 (2H,d, J = 8,4 Hz) ; 8,05 (2H,d, J = 8,3 Hz) ; 11,26 (1H, s, échangeable par $CF_3COOD$).

| | C % | H % | F % | N % | S % |
|---|---|---|---|---|---|
| calculé | 37,98 | 2,34 | 20,03 | 5,91 | 13,52 |
| trouvé | 37,94 | 2,38 | 19,65 | 5,75 | 13,40 |

IR : $\bar{\nu}$ = 1563, 1522, 1503, 1330, 1170, 1142, 992 $cm^{-1}$
RMN $^1H$ (DMSO $d_6$) : δ = 2,49 (6H,s) ; 6,50 (2H, s, échangeables par $CF_3COOD$) ; 7,03 (2H,s) ; 12,1 (pic élargi échangeable par $CF_3COOD$)

**Exemple 38 (méthode A2)**

**N-[3,5-Diméthyl-4-[(nitrométhyl)sulfonyl]phényl]-2-dibenzofuransulfonamide**

Le composé du titre est obtenu en opérant comme dans l'exemple 6 à partir d'une solution de 2,0 g (8,19 mmoles) de 3,5-diméthyl-4-[(nitrométhyl)sulfonyl]aniline dans 38 ml de tétrahydrofurane, de 1,3 ml (16,1 mmoles) de pyridine et de 3,3 g (12,3 mmoles) de chlorure de 2-dibenzofuransulfonyle [préparé selon M. JANCZEWSKI et H. MAZIARCZYK, Rocz.chem 47 (11), 2055-2069 (1973)].
Le milieu réactionnel est agité pendant 20 h à température ambiante puis est chauffé à 45°C pendant 4 h. Le produit final est purifié par chromatographie sur colonne de silice (éluant : chlorure de méthylène) et recristallisation dans un mélange d'hexane et d'acétate d'éthyle.
Rendement : 0,48 g (12 %)
F = 214 - 215°C (hexane - acétate d'éthyle) (décomp.)

| Analyse centésimale : $C_{21}H_{18}N_2O_7S_2$ (M = 474,49) | | | | |
|---|---|---|---|---|
| | C % | H % | N % | S % |
| calculé | 53,15 | 3,82 | 5,90 | 13,51 |
| trouvé | 52,88 | 3,91 | 5,92 | 13,46 |

IR : $\bar{\nu}$ = 3284, 1596, 1571, 1479, 1471, 1446, 1400, 1361, 1336, 1324, 1310, 1205, 1199, 1160, 1139, 1129, 1069, 884, 852, 755, 637, 611 $cm^{-1}$
RMN $^1H$ (DMSO $d_6$) : δ = 2,41 (6H,s) ; 6,36 (2H, s, échangeables par $CF_3COOD$) 7,00 (2H,s) ; 7,43 (1H,t, J = 7,2 Hz) ; 7,53 - 7,59 (1H,m) ; 7,72 (1H,d, J = 8,3 Hz); 7,88 (1H,d, J = 8,7 Hz) ; 7,95 - 7,99 (1H,m) ; 8,32 (1H,d, J = 7,3 Hz) ; 8,82 (1H,d, J = 1,9 Hz) ; 11,10 (1H, s, échangeable par $CF_3COOD$).

**Exemple 36 (méthode A2)**

**N-[3,5-Diméthyl-4-[(nitrométhyl)sulfonyl]phényl]-4-(trifluorométhoxy)benzène sulfonamide**

Le composé du titre est obtenu en opérant comme dans l'exemple 11 à partir d'une solution de 3,1 g (12,7 mmoles) de 3,5-diméthyl-4-[(nitrométhyl)sulfonyl]aniline dans 62,4 ml de tétrahydrofurane, de 2,05 g (25,9 mmoles) de pyridine et de 5,0 g (19,2 mmoles) de chlorure de 4-(trifluorométhoxy)benzènesulfonyle
Rendement : 1,8 g (29 %)
F = 180 - 182°C (hexane - acétate d'éthyle)

| Analyse centésimale : $C_{16}H_{15}F_3N_2O_7S_2$ (M = 468,42) | | | | | |
|---|---|---|---|---|---|
| | C % | H % | F % | N % | S % |
| calculé | 41,02 | 3,23 | 12,17 | 5,98 | 13,69 |
| trouvé | 41,06 | 3,29 | 11,81 | 6,13 | 13,41 |

IR : $\bar{\nu}$ = 1595, 1559, 1347, 1327, 1266, 1218, 1164, 1138 $cm^{-1}$
RMN $^1H$ (DMSO $d_6$) : $\delta$ = 2,34 (6H,s) ; 6,31 (2H, s, échangeables par $CF_3COOD$) ; 6,85 (2H,s) ; 7,47 (2H,d, J = 8,2 Hz) ; 7,87 - 7,92 (2H,m) ; 11,07 (1H, s, échangeable par $CF_3COOD$).

**Exemple 37 (méthode A1)**

**N-[3,5-Diméthyl-4-[(nitrométhyl)sulfonyl]phényl]-2,3,4,5,6-pentafluorobenzènesulfonamide**

Le composé du titre est obtenu en opérant comme dans l'exemple 5 à partir d'une solution de 1,3 g (5,32 mmoles) de 3,5-diméthyl-4-[(nitrométhyl)sulfonyl]aniline dans 50 ml de tétrahydrofurane, de 1,1 g (11,0 mmoles) de carbonate de calcium et de 2,1 g (7,88 mmoles) de chlorure de pentafluorobenzènesulfonyle. Le milieu réactionnel est agité pendant 68 h à température ambiante. Le produit final est purifié par chromatographie sur colonne de silice (éluant: chlorure de méthylène).
Rendement : 0,4 g (16 %)
F = 128 - 130°C (hexane - chlorure de méthylène)
Analyse centésimale : $C_{15}H_{11}F_5N_2O_6S_2$ (M = 474,37)

**Exemple 39 (méthode A2)**

**N-[3,5-Diméthyl-4-[(nitrométhyl)sulfonyl]phényl]-4-biphénylsulfonamide**

Le composé du titre est obtenu en opérant comme dans l'exemple 6 à partir d'une solution de 2,0 g (8,19 mmoles) de 3,5-diméthyl-4-[(nitrométhyl)sulfonyl]aniline dans 38 ml de tétrahydrofurane, de 1,3 ml (16,1 mmoles) de pyridine et de 3,1 g (12,3 mmoles) de chlorure de 4-biphénylsulfonyle.
Le milieu réactionnel est agité pendant 20 h à température ambiante et chauffé à 40°C pendant 4 h. Le produit final est purifié par chromatographie sur colonne de silice (éluant : chlorure de méthylène) et recristallisation dans un mélange d'hexane et d'acétate d'éthyle.
Rendement : 0,68 g (18 %)
F = 206 - 208°C (hexane - acétate d'éthyle) (décomp.)

| Analyse centésimale : $C_{21}H_{20}N_2O_6S_2$ (M = 460,51) | | | | |
|---|---|---|---|---|
| | C % | H % | N % | S % |
| calculé | 54,77 | 4,38 | 6,08 | 13,92 |
| trouvé | 54,90 | 4,43 | 6,05 | 13,58 |

IR : $\bar{\nu}$ = 3237, 1594, 1564, 1557, 1479, 1346, 1156, 1068, 764, 674, 626, 604 $cm^{-1}$
RMN $^1H$ (DMSO $d_6$) : $\delta$ = 2,53 (6H,s) ; 6,49 (2H, s, échangeables par $CF_3COOD$) ; 7,08 (2H,s) ; 7,48 - 7,57 (3H,m) ; 7,75 - 7,78 (2H,m) ; 7,94 - 8,04 (4H,m) ; 11,20 (1H, s, échangeable par $CF_3COOD$).

**Exemple 40 (méthode A2)**

**N-[3,5-Diméthyl-4-[(nitrométhyl)sulfonyl]phényl]méthanesulfonamide**

A une solution maintenue sous atmosphère d'azote de 2,7 g (11,0 mmoles) de 3,5-diméthyl-4-[(nitrométhyl) sulfonyl]aniline et de 1,6 g (20,2 mmoles) de pyridine séchée sur potasse, dans 50 ml de tétrahydrofurane anhydre, est ajoutée, goutte à goutte, une solution de 1,7 g (14,8 mmoles) de chlorure de méthanesulfonyle dans 5 ml de tétrahydrofurane anhydre. Le mélange est agité pendant 6 h 30 à température ambiante, puis est porté à reflux pendant 4 h. On ajoute alors 1,1 g (9,60 mmoles) de chlorure de méthanesulfonyle et poursuit le reflux pendant 1 h 30. On rajoute à nouveau 1,1 g (9,60 mmoles) de chlorure de méthanesulfonyle et poursuit le reflux pendant 4 h. Après

refroidissement, le milieu réactionnel est jeté dans l'eau glacée et extrait au chlorure de méthylène. Les extraits organiques rassemblés sont séchés sur sulfate de sodium et concentrés à sec sous pression réduite. Le résidu obtenu est cristallisé dans un mélange refroidi d'hexane et de chlorure de méthylène et est purifié par recristallisation dans un mélange d'hexane et d'acétate d'éthyle pour donner 1,1 g (Rendement = 22 %) de N-[3,5-diméthyl-4-[(nitrométhyl) sulfonyl]phényl]méthanesulfonamide contenant 14 % molaire d'acétate d'éthyle.
F = 138 - 140°C (hexane - acétate d'éthyle)

| Analyse centésimale : $C_{10}H_{14}N_2O_6S_2$ (M = 322,35), 14 % $C_4H_8O_2$ | | | | |
|---|---|---|---|---|
| | C % | H % | N % | S % |
| calculé | 38,18 | 4,61 | 8,32 | 19,05 |
| trouvé | 37,90 | 4,55 | 8,12 | 18,62 |

IR : $\bar{\nu}$ = 3269, 1598, 1569, 1344, 1339, 1309, 1142 $cm^{-1}$
RMN $^1H$ (DMSO $d_6$) : δ = 2,58 (6H,s) ; 3,23 (3H,s) ; 3,5 (1H, pic élargi échangeable par $CF_3COOD$) ; 6,52 (2H, s, échangeables par $CF_3COOD$) ; 7,09 (2H,s).

**Exemple 41 (méthode C)**

**1-[3,5-Diméthyl-4-[(nitrométhyl)sulfonyl]phényl]-3-(phénylsulfonyl)urée**

Le composé du titre est obtenu en opérant comme dans l'exemple 20 à partir d'une suspension de 2,8 g (11,5 mmoles) de 3,5-diméthyl-4-[(nitrométhyl)sulfonyl]aniline dans 56 ml de chlorure de méthylène et d'une solution de 2,0 g (10,9 mmoles) d'isocyanate de benzènesulfonyle dans 15 ml de chlorure de méthylène.
Dans le produit final, l'acétate d'éthyle résiduel est éliminé par chauffage à reflux dans le chlorure de méthylène pendant 2 h.
Rendement : 4,5 g (96 %)
F = 140 - 142°C (hexane - acétate d'éthyle)

| Analyse centésimale : $C_{16}H_{17}N_3O_7S_2$ (M = 427,44) | | | | |
|---|---|---|---|---|
| | C % | H % | N % | S % |
| calculé | 44,96 | 4,01 | 9,83 | 15,00 |
| trouvé | 45,15 | 4,16 | 10,15 | 14,71 |

IR : $\bar{\nu}$ = 1658, 1587, 1567, 1529, 1463, 1326, 1152, 1086, 899 $cm^{-1}$
RMN $^1H$ (DMSO $d_6$) : δ = 2,31 (6H,s) ; 6,26 (2H, s, échangeables par $CF_3COOD$) ; 7,09 (2H,s) ; 7,41 - 7,54 (3H,m) ; 7,76 - 7,79 (2H,m) ; 9,06 (1H, s, échangeable par $CF_3COOD$) ; 10,96 (1H, pic élargi échangeable par $CF_3COOD$)

**Exemple 42 (méthode A2)**

**N-[3,5-Diméthyl-4-[(nitrométhyl)sulfonyl]-3-dibenzofurane sulfonamide**

Obtenu en opérant comme dans l'exemple 6 à partir d'une solution de 2,0 g (8,19 mmoles) de 3,5-diméthyl-4-[(nitrométhyl)sulfonyl]aniline dans 40 ml de tétrahydrofurane, de 1,29 g (16,3 mmoles) de pyridine et de 3,0 g (12,3 mmoles) de chlorure de 3-dibenzofuransulfonyle [préparé selon M. JANCZEWSKI et H. MAZIARCZYK, Rocz. Chem. 48 (11), 1907-1919 (1974)].
Le milieu réactionnel est agité pendant 2 h à température ambiante puis est chauffé à 40°C pendant 24 h. Le produit final est purifié par chromatographie sur colonne de silice (éluant : chlorure de méthylène) et recristallisation dans un mélange d'hexane et d'acétate d'éthyle.
Rdt:2,1 g (54%)
F = 206 - 208°C (hexane - acétate d'éthyle)

| Analyse centésimale : $C_{21}H_{18}N_2O_7S_2$ (M = 474,49) | | | |
|---|---|---|---|
| | C % | H % | N % |
| calculé | 53,15 | 3,82 | 5,90 |

(suite)

| Analyse centésimale : $C_{21}H_{18}N_2O_7S_2$ (M = 474,49) | | | |
|---|---|---|---|
| | C % | H % | N % |
| trouvé | 53,34 | 3,95 | 5,68 |

IR : $\bar{\nu}$ = 3274, 1592, 1561, 1462, 1343, 1324, 1201, 1180, 1158, 1139, 1068, 852, 745, 705, 653, 632 $cm^{-1}$
RMN $^1H$ (DMSO $d_6$) : δ = 2,52 (6H,s) ; 6,46 (2H, s, échangeables par $CF_3COOD$) ; 7,09 (2H,s) ; 7,53 - 7,55 (1H,m) ; 7,69 - 7,72 (1H,m) ; 7,84 (1H,d, J = 8,3 Hz) ; 7,96 - 8,00 (1H,m) ; 8,28 - 8,33 (2H,m) ; 8,44 (1H,d, J = 8,1 Hz) ; 11,25 (1H, s, échangeable par $CF_3COOD$).

**Exempte 43 (méthode A1)**

**N-[2-Chloro-4-[3,5-diméthyl-4-[(nitrométhyl)sulfonyl]phénylsulfamoyl]phényl]acétamide**

Le composé du titre est obtenu en opérant comme dans l'exemple 22 en utilisant 3,3 g (12,3 mmoles) de chlorure de 4-acetamido-3-chlorobenzènesulfonyle.

Le milieu réactionnel est agité pendant 7 h 30 à température ambiante puis est chauffé à reflux pendant 7 h 30. Le résidu solide obtenu après concentration sous pression réduite est trituré dans l'hexane, puis lavé par un mélange d'hexane et d'acétate d'éthyle et enfin lavé à l'éthanol.
Rendement : 0,1 g (2,6 %)
F = 209 - 211°C

| Analyse centésimale : $C_{17}H_{18}ClN_3O_7S_2$ (M = 475,92) | | | | | |
|---|---|---|---|---|---|
| | C% | H% | Cl% | N% | S% |
| calculé | 42,90 | 3,81 | 7,45 | 8,83 | 13,47 |
| trouvé | 42,75 | 3,91 | 7,75 | 8,77 | 13,01 |

IR : $\bar{\nu}$ = 3402, 3276, 1722, 1583, 1550, 1514, 1349, 1174, 1163, 631 $cm^{-1}$
RMN $^1H$ (DMSO $d_6$) : δ = 2,04 (3H,s) ; 2,39 (6H,s) ; 6,36 (2H, s, échangeables par $CF_3COOD$) ; 6,90 (2H,s) ; 7,74 (1H, dd, J = 8,7 et 2,2 Hz) ; 7,86 (1H,d, J = 2,2 Hz) ; 8,01 (1H,d, J = 8,7 Hz) ; 9,64 (1H, s, échangeable par $CF_3COOD$)

**Exemple 44 (méthode A2)**

**N-[3,5-Diméthyl-4-[(nitrométhyl)sulfonyl]phényl]-2-méthoxybenzènesulfonamide**

Le composé du titre est obtenu en opérant comme dans l'exemple 18 à partir d'une solution de 2,0 g (8,19 mmoles) de 3,5-diméthyl-4-[(nitrométhyl)sulfonyl]aniline dans 50 ml de tétrahydrofurane, de 1,31 ml (16,2 mmoles) de pyridine et de 2,53 g (12,2 mmoles) de chlorure de 2-méthoxybenzènesulfonyle [préparé selon H. MEERWEIN et al., Chem. Ber. 90, 841-852 (1957)]

Le produit final est purifié par chromatographie sur colonne de silice (éluant : chlorure de méthylène).
Rendement : 1,8 g (53 %)
F = 172 - 174°C (hexane - acétate d'éthyle)

| Analyse centésimale : $C_{16}H_{18}N_2O_7S_2$ (M = 414,44) | | | | |
|---|---|---|---|---|
| | C % | H % | N % | S % |
| calculé | 46,37 | 4,38 | 6,76 | 15,47 |
| trouvé | 46,34 | 4,29 | 6,74 | 15,29 |

IR : $\bar{\nu}$ = 3261, 1595, 1559, 1486, 1335, 1323, 1282, 1166, 1157, 1132, 1072, 600 $cm^{-1}$
RMN $^1H$ (DMSO $d_6$) ; δ = 2,59 (6H,s) ; 3,98 (3H,s) ; 6,57 (2H, s, échangeables par $CF_3COOD$) ; 7,10 (2H,s) ; 7,23 - 7,29 (1H,m) ; 7,34 (1H,d, J = 8,1 Hz) ; 7,74 - 7,80 (1H,m) ; 8,06 - 8,09 (1H,m) ; 10,99 (1H, s, échangeable par $CF_3COOD$).

**<u>Exemple 45</u> (méthode A2)**

**N-[3,5-Diméthyl-4-[(nitrométhyl)sulfonyl]phényl]-2-méthyl-benzène sulfonamide**

Obtenu en opérant comme dans l'exemple 27, à partir d'une solution de 2,6 g (10,6 mmoles) de 3,5-diméthyl-4-[(nitrométhyl)sulfonyl]aniline dans 65 ml de tétrahydrofurane, de 1,69 ml (20,9 mmoles) de pyridine et de 3 g (15,7 mmoles) de chlorure d'ortho-toluenesulfonyle.

On agite à température ambiante pendant 24 heures ; on chauffe ensuite 8 heures à 40° C, puis on laisse 24 heures à température ambiante.
Rendement : 0,29 g (6,8 %)
F = 118 - 120° C (hexane - acétate d'éthyle)

| Analyse centésimale : $C_{16}H_{18}N_2O_6S_2$ (M = 398,44) | | | | |
|---|---|---|---|---|
| | C% | H% | N% | S% |
| calculé<br>trouvé | 48,23<br>48,37 | 4,55<br>4,40 | 7,03<br>7,10 | 16,09<br>15,77 |

La RMN indique la présence d'isomère ortho méthyl (environ 50 %) mélangé aux isomères meta et para (environ 50 % pour les deux) provenant du fait que le chlorure d'ortho-toluenesulfonyle commercial (TCI) est en fait un mélange d'isomères (comme l'indique son spectre RMN).

**<u>Exemple 46</u> (méthode A2)**

**N-[3,5-Diméthyl-4-[(nitrométhyl)sulfonyl]phényl]-3-méthoxy-benzène sulfonamide**

Obtenu en opérant comme dans l'exemple 2, à partir de 2 g (8,18 mmoles) de 3,5-diméthyl-4-[(nitrométhyl) sulfonyl]aniline, de 50 ml de tétrahydrofurane, de 1,3 ml (16,1 mmole) de pyridine et de 2,5 g (12,3 mmoles) de chlorure de 3-méthoxy-benzènesulfonyle (préparé selon M. LUDWIG et Coll, Collect. Czech. Chem. Commun. 1984, <u>49</u> (5), 1182).
Rendement : 0,83 g (24,7 %)
F = 166 - 168° C (hexane - acétate d'éthyle)

| Analyse centésimale : $C_{16}H_{18}N_2O_7S_2$ (M = 414,44) | | | | |
|---|---|---|---|---|
| | C % | H % | N % | S % |
| calculé<br>trouvé | 46,37<br>46,37 | 4,38<br>4,48 | 6,76<br>6,70 | 15,47<br>15,31 |

RMN $^1H$ (DMSO $d_6$) : $\delta$ = 3,85 (3H,s) ; 6,5 (2H, s, échangeables avec $CF_3COOD$) ; 7,05 (2H,s) ; 7,25 à 7,65 (4H, m) ; 11,1 (1H, s, échangeable avec $CF_3COOD$).

**<u>Exemple 47</u> (méthode A1)**

**N-[3,5-Diméthyl-4-[(nitrométhyl)sulfonyl]phényl]-3-pyridinesulfonamide**

Obtenu en opérant comme dans l'exemple 5, à partir de 1,7 g (7 mmoles) de 3,5-diméthyl-4-[(nitrométhyl) sulfonyl]aniline, de 40 ml de tétrahydrofurane, de 2,8 g (28 mmoles) de $CaCO_3$ et de 1,9 g (10,5 mmoles) de chlorure de 3-pyridinesulfonyle (préparé selon FUERST et Coll, J. Prakt. Chem., 1967, <u>36</u>, 160) en agitant 5 heures à température ambiante.

Le produit brut est chromatographié sur silice avec un mélange $CH_2Cl_2$-MeOH (98:2) puis recristallisé dans un mélange hexane - acétate d'éthyle.
Rendement : 0,11 g (4 %)
F = 191 - 192° C (hexane - acétate d'éthyle)

| Analyse centésimale : $C_{14}H_{15}N_3O_6S_2$ (M = 385,42) | | | | |
|---|---|---|---|---|
| | C% | H% | N% | S% |
| calculé | 43,63 | 3,92 | 10,90 | 16,64 |
| trouvé | 43,62 | 3,99 | 10,91 | 16,76 |

RMN $^1H$ (DMSO $d_6$) : δ = 6,5 (2H, s, échangeables avec $CF_3COOD$) ; 7,03 (2H,s) ; 7,64 (1H,dd) ; 8,28 (1H,dt) ; 8,85 (1H,dd) ; 9,06 (1H,d) ; 11,3 (1H, s, échangeable avec $CF_3COOD$).

**Exemple 48 (méthode A2)**

**N-[3,5-Diméthyl-4-[(nitrométhyl)sulfonyl]phényl]-(2-trifluorométhylphényl) méthanesulfonamide**

Obtenu en opérant comme dans l'exemple 2, à partir de 3 g (12,28 mmoles) de 3,5-diméthyl-4-[(nitrométhyl) sulfonyl]aniline, de 57 ml de tétrahydrofurane anhydre, de 2 ml (24,7 mmoles) de pyridine et de 4,8 g (18,5 mmoles) de chlorure de (2-trifluorométhylphényl)méthanesulfonyle (préparé selon HAMER et

Coll., J. Pharm. Sci., 1975, 64, 1961). Le produit obtenu est recristallisé dans un mélange hexane - acétate d'éthyle.
Rendement : 1,37 g (24 %)
F = 146 - 148° C (hexane - acétate d'éthyle)

| Analyse centésimale : $C_{17}H_{17}F_3N_2O_6S_2$ | | | | | |
|---|---|---|---|---|---|
| | C% | H% | F% | N% | S% |
| calculé | 43,77 | 3,67 | 12,22 | 6,01 | 13,75 |
| trouvé | 43,72 | 3,90 | 11,97 | 6,03 | 13,65 |

RMN $^1H$ (DMSO $d_6$) : δ = 2,3 (6H,s) ; 4,6 (2H,s) ; 6,27 (2H, s, échangeables avec $CF_3COOD$) ; 6,71 (2H,s) ; 7,4 à 7,6 (4H,m) ; 10,6 (1H, s, échangeable avec $CF_3COOD$)

**Exemple 49 (méthode A2)**

**N-[3,5-Diméthyl-4-[(nitrométhyl)sulfonyl]phényl]-(3-fluorophényl)methane sulfonamide**

Obtenu en opérant comme dans l'exemple 2, à partir de 3 g (12,28 mmoles) de 3,5-diméthyl-4-[(nitrométhyl) sulfonyl]aniline, de 57 ml de tétrahydrofurane anhydre, de 2 ml (24,7 mmoles) de pyridine et de 3,85 g (18,4 mmoles) de chlorure de (3-fluorophényl)méthanesulfonyle (préparé selon US 3 471 474). Le produit est recristallisé dans un mélange hexane - acétate d'éthyle.
Rendement : 0,45 g (8,8 %)
F = 172 - 174° C (hexane - AcOEt)

| Analyse centésimale : $C_{17}H_{17}FN_2O_6S_2$ (M = 416,45) | | | | | |
|---|---|---|---|---|---|
| | C % | H % | F % | N % | S % |
| calculé | 46,15 | 4,11 | 4,56 | 6,73 | 15,40 |
| trouvé | 45,96 | 4,05 | 4,62 | 6,73 | 15,44 |

RMN $^1H$ (DMSO $d_6$) : δ = 2,28 (6H,s) ; 4,5 (2H,s) ; 6,24 (2H, s, échangeables avec $CF_3COOD$) ; 6,71 (2H,s) ; 6,85 à 7,0 (3H,m) ; 7,11 à 7,21 (1H,m) ; 10,25 (1H, s, échangeable avec $CF_3COOD$)

**Exemple 50 (méthode A2)**

**N-[3[[3,5-Diméthyl-4-[(nitrométhyl)sulfonyl]phénylamino]sulfonyl]phényl] acétamide**

Obtenu en opérant comme dans l'exemple 2, à partir de 2 g (8,18 mmoles) de 3,5-diméthyl-4-[(nitrométhyl) sulfonyl]aniline, de 50 ml de tétrahydrofurane anhydre, de 1,3 ml (16,1 mmoles) de pyridine, et d'une solution de 3,8

g (16,2 mmoles) de chlorure de 3-acétylamino-benzènesulfonyle (préparé selon BARCO et Coll, Synthesis, 1974, 877) dans 50 ml de tétrahydrofurane anhydre.

Après évaporation du solvant d'extraction, le résidu est chromatographié sur silice avec un mélange d'hexane et d'acétate d'éthyle (1:1) comme éluant, puis recristallisé deux fois dans le chlorure de méthylène.

Rendement : 0,775 g (21,4 %)

F = 108 - 110° C ($CH_2Cl_2$)

| Analyse centésimale : $C_{17}H_{19}N_3O_7S_2$ (M = 441,48) | | | | |
|---|---|---|---|---|
| | C% | H% | N% | S% |
| calculé | 46,25 | 4,34 | 9,52 | 14,53 |
| trouvé | 46,15 | 4,5 | 9,29 | 14,17 |

RMN $^1H$ (DMSO $d_6$) : δ = 2,08 (3H,s) ; 2,48 (6H,s) ; 6,45 (2H, s, échangeables avec $CF_3COOD$) ; 7,0 (2H,s) ; 7,5 à 7,7 (3H,m) ; 8,36 (1H,s) ; 10,3 (1H, s, échangeable avec $CF_3COOD$) ; 11,15 (1H, s échangeable avec $CF_3COOD$)

**Exemple 51 (méthode A2)**

**N-[3,5-Diméthyl-4-[(nitrométhyl)sulfonyl]phényl]-(2-méthylphényl)methane sulfonamide**

Obtenu en opérant comme dans l'exemple 2, à partir de 2 g (8,18 mmoles) de 3,5-diméthyl-4-[(nitrométhyl) sulfonyl]aniline, de 38 ml de tétrahydrofurane anhydre, de 1,3 ml (16,1 mmoles) de pyridine, et de 2,5 g (12,2 mmoles) de chlorure de (2-méthylphényl)méthanesulfonyle.

Après évaporation du solvant d'extraction, le résidu est chromatographié sur silice une première fois en utilisant le chlorure de méthylène comme éluant, puis une deuxième fois en utilisant un mélange hexane - acétate d'éthyle (1:1).

Enfin le produit est recristallisé dans un mélange hexane - acétate d'éthyle

Rendement : 0,19 g (5,6 %)

F = 167 - 169° C (hexane - AcOEt)

| Analyse centésimale : $C_{17}H_{20}N_2O_6S_2$ (M = 412,49) | | | |
|---|---|---|---|
| | C % | H % | N % |
| calculé | 49,50 | 4,89 | 6,79 |
| trouvé | 49,73 | 4,90 | 6,55 |

RMN $^1H$ (DMSO $d_6$) : δ = 2,3 (3H,s) ; 2,5 (6H,s) ; 4,68 (2H,s) ; 6,43 (2H, s, échangeables avec $CF_3COOD$) ; 6,91 (2H, s) ; 7,1 à 7,3 (4H,m) ; 10,5 (1H, s, échangeable avec $CF_3COOD$)

La matière première, le chlorure de (2-méthylphényl)méthanesulfonyle est préparée à partir d'alpha-chloro ortho-xylène en suivant le procédé de J. NAKAYAMA et Coll, Tetrahedron Letters, 1984, 25 (40) 4553.

Rendement.: 31 %

F = 46 - 48° C

RMN $^1H$ ($CDCl_3$) : δ = 2,4 (3H,s) ; 4,9 (2H,s) ; 7,15 à 7,40 (4H,m)

**Exemple 52 (méthode A2)**

**4-Bromo-N-[3,5-diméthyl-4-[(nitrométhyl)sulfonyl]phényl]-(2-fluorophényl) méthanesulfonamide**

Obtenu en opérant comme dans l'exemple 2, à partir de 3 g (12,28 mmoles) de 3,5-diméthyl-4-[(nitrométhyl) sulfonyl]aniline, 57 ml de tétrahydrofurane anhydre, 2 ml (24,7 mmoles) de pyridine, et 5,3 g (19,78 mmoles) de chlorure de (4-bromo-2-fluorophényl)méthanesulfonyle. Après évaporation du solvant, le résidu est chromatographié deux fois sur silice en éluant avec $CH_2Cl_2$ et recristallisé deux fois dans un mélange hexane - acétate d'éthyle.

Rendement: 1,1 g (18 %)

F = 152 - 154° C (hexane - AcOEt)

| Analyse centésimale : $C_{16}H_{16}BrFN_2O_6S_2$ (M = 495,35) | | | | | | |
|---|---|---|---|---|---|---|
| | C% | H% | Br% | F% | N% | S% |
| calculé | 38,80 | 3,26 | 16,13 | 3,84 | 5,66 | 12,95 |
| trouvé | 38,64 | 3,22 | 16,15 | 3,30 | 5,50 | 12,86 |

RMN $^1H$ (DMSO $d_6$) : δ = 2,65 (6H,s) ; 4,85 (2H,s) ; 6,60 (2H, s, échangeables avec $CF_3COOD$) ; 7,08 (2H,s) ; 7,45 à 7,74 (3H,m) ; 10,8 (1H, s, échangeable avec $CF_3COOD$)

La matière première, le chlorure de (4-bromo-2-fluorophényl)méthanesulfonyle est préparée à partir du bromure de 4-bromo-2-fluorobenzyle en suivant le procédé de J. NAKAYAMA et Coll., Tetrahedron Letters, 1984, 25 (40), 4553.

Rendement : 90 %

Huile purifiée par chromatographie rapide sur $SiO_2$ en éluant avec $CH_2Cl_2$.

RMN $^1H$ ($CDCl_3$) : δ = 4,8 (2H,s) ; 7,2 à 7,4 (4H,m)

**Exemple 53 (méthode A2)**

**N-[3,5-Diméthyl-4-[(nitrométhyl)sulfonyl]phényl]-cyclohexylméthane sulfonamide**

A un mélange refroidi à -20° C sous atmosphère d'aozte de 2 g (8,18 mmoles) de 3,5-diméthyl-4-[(nitrométhyl) sulfonyl]aniline, 50 ml de tétrahydrofurane anhydre et 1,3 ml (16,1 mmoles) de pyridine, on ajoute goutte à goutte 3,2 g (16,1 mmoles) de chlorure de cyclohexyl-méthanesulfonyle (préparé selon J.F. KING et Coll., J. Am. Chem. Soc., 1992, 114 (5), 1743) et on agite 6 heures à -20° C. On laisse une nuit à -20° C puis on laisse revenir à température ambiante et on agite 24 heures à température ambiante puis on chauffe 13 heures à reflux. On refroidit, verse dans l'eau, acidifie à pH = 3 avec HCl 1N, extrait avec $CH_2Cl_2$, lave à l'eau, sèche sur $Na_2SO_4$, filtre et évapore sous vide.

Le résidu est purifié par chromatographie sur silice en éluant avec $CH_2Cl_2$ puis recristallisé dans un mélange hexane - acétate d'éthyle.

Rendement : 0,085 g (2,5 %)

F = 126 - 128°C

| Analyse centésimale : $C_{16}H_{24}N_2O_6S_2$ + 17 % $C_6H_{14}$ (M = 404,51 + 0,17 x 86,18) | | | | |
|---|---|---|---|---|
| | C% | H% | N% | S% |
| calculé | 48,77 | 6,34 | 6,68 | 15,30 |
| trouvé | 48,53 | 6,20 | 6,66 | 14,91 |

RMN $^1H$ (DMSO $d_6$) : δ = 1,0 à 1,3 (5H,m) ; 1,5 à 1,7 (3H,m) ; 1,75 à 1,90 (3H,m) ; 2,5 (6H,s) ; 3,15 à 3,22 (2H,m) ; 6,48 (2H, s, échangeables avec $CF_3COOD$) ; 7,03 (2H,s) ; 10,5 (1H, s, échangeable avec $CF_3COOD$)

**Exemple 54**

**Sel de sodium du N-[3,5-diméthyl-4-[(nitrométhyl)sulfonyl]phényl]-benzène sulfonamide**

Un mélange de 0,34 g (0,884 mmole) de N-[3,5-diméthyl-4-[(nitrométhyl)sulfonyl]phényl]-benzènesulfonamide, et de 0,149 g (1,77 mmole) de bicarbonate de sodium dans 15 ml d'eau distillée est agitée 24 heures à température ambiante.

La solution obtenue est évaporée sous vide poussé à température ≤ 30° C. Le résidu solide est lavé à l'éther, séché sous vide et recristallisé dans un mélange méthanol-éther.

Rendement : 0,22 g (58 %)

F = 253 - 255° C (MeOH - $Et_2O$)

| Analyse centésimale : $C_{15}H_{14}N_2Na_2O_6S_2$ + 0,65 $H_2O$ (M = 440,11) | | | | | |
|---|---|---|---|---|---|
| | C% | H% | N% | Na % | S % |
| calculé | 40,94 | 3,50 | 6,37 | 10,45 | 14,57 |
| trouvé | 41,18 | 3,43 | 6,46 | 10,52 | 14,25 |

RMN $^1H$ (DMSO $d_6$) : $\delta$ = 2,44 (6H,s) ; 6,98 (2H,s) ; 7,05 (1H, s, échangeable avec $CF_3COOD$) ; 7,46 à 7,65 (3H,m) ; 7,8 à 7,9 (2H, m)

**Exemple 55**

**Sel de calcium du N-[3,5-diméthyl-4-[(nitrométhyl)sulfonyl]phényl]benzène sulfonamide**

Un mélange de 0,34 g (0,88 mmole) de N-[3,5-diméthyl-4-[(nitrométhyl)sulfonyl]phényl]-benzènesulfonamide, et de 0,033 g (0,88 mmole) d'hydroxyde de calcium dans 20 ml d'eau déionisée est agité 1 heure à 25° C puis 15 min à 70° C. La solution obtenue est filtrée à chaud et évaporée sous vide. Le solide résiduel est recristallisé dans un mélange méthanol - éther.
Rendement : 0,08 g (22 %)
F = 195 - 197° C (MeOH - $Et_2O$)

| Analyse centésimale : $C_{30}H_{30}CaN_4O_{12}S_4 + 2H_2O$ (M = 842,93) | | | | | |
|---|---|---|---|---|---|
| | C % | H % | Ca % | N % | S % |
| calculé | 42,75 | 4,06 | 4,75 | 6,65 | 15,21 |
| trouvé | 42,52 | 4,17 | 4,74 | 6,40 | 14,81 |

RMN $^1H$ (DMSO $d_6$) : $\delta$ = 2,43 (6H,s) ; 6,5 (1H, s, échangeable avec $CF_3COOD$) ; 6,7 (2H,s) ; 7,4 à 7,6 (3H,m) ; 7,7 à 7,8 (2H, m) ; 10,5 (1H, s, échangeable avec $CF_3COOD$)

**Exempte 56 (méthode F1)**

**N,N'-Bis[3,5-diméthyl-4-[(nitrométhyl)sulfonyl]phényl]-1,8-octanediamide**

A un mélange de 2 g (8,18 mmoles) de 3,5-diméthyl-4-[(nitrométhyl)sulfonyl] aniline, 50 ml de tétrahydrofurane anhydre, et de 1,64 g (16,3 mmoles) de carbonate de calcium maintenus sous atmosphère d'azote, on ajoute 0,8637 g (4,09 mmoles) de chlorure de suberoyle et on agite une nuit à température ambiante. On verse dans l'eau, acidifie à pH = 3 avec HCl dilué, et filtre un insoluble. Le filtrat est extrait à l'acétate d'éthyle, on lave la phase organique à l'eau, sèche sur sulfate de sodium, filtre et évapore sous vide. Le résidu obtenu est réuni avec le premier insoluble, et on recristallise dans un mélange éthanol - DMF.
Rendement : 0,79 g (15,4 %)
F = 235 - 238° C (EtOH - DMF)

| Analyse centésimale : $C_{26}H_{34}N_4O_{10}S_2$ (M = 626,68) | | | | |
|---|---|---|---|---|
| | C% | H% | N% | S % |
| calculé | 49,83 | 5,47 | 8,94 | 10,23 |
| trouvé | 49,96 | 5,60 | 9,16 | 9,86 |

IR: $\bar{\nu}$ = 3363 et 1667 $cm^{-1}$
RMN $^1H$ (DMSO $d_6$) : $\delta$ = 1,25 à 1,35 (4H,m) ; 1,5 à 1,6 (4H,m) ; 2,3 (4H,t) ; 2,48 (12H,s) ; 6,42 (4H, s, échangeables avec $CF_3COOD$) ; 7,5 (4H,s) ; 10,2 (2H, s, échangeables avec $CF_3COOD$)

**Exemple 57 (méthode F1)**

**N,N'-Bis[3,5-diméthyl-4-[(nitrométhyl)sulfonyl]phényl]-1,5-pentanediamide**

A un mélange de 2 g (8,18 mmoles) de 3,5-diméthyl-4-[(nitrométhyl)sulfonyl] aniline, 50 ml de tétrahydrofurane anhydre et 1,64 g (16,3 mmoles) de carbonate de calcium maintenu sous atmosphère d'azote, on ajoute 0,691 g (4,09 mmoles) de dichlorure de glutaryle et on agite 24 heures à température ambiante. On verse dans l'eau, acidifie à pH = 3 avec HCl dilué, extrait à l'acétate d'éthyle, lave à l'eau, sèche la phase organique sur $Na_2SO_4$, filtre et évapore sous vide. Le solide obtenu est recristallisé dans un mélange hexane - acétate d'éthyle.
Rendement : 0,49 g (20 %)
F = 207 - 210° C (hexane - AcOEt)

| Analyse centésimale : $C_{23}H_{28}N_4O_{10}S_2$ (M = 584,606) | | | | |
|---|---|---|---|---|
| | C % | H % | N % | S % |
| calculé<br>trouvé | 47,25<br>47,06 | 4,83<br>4,87 | 9,58<br>9,46 | 10,97<br>10,65 |

IR : $\bar{\nu}$ = 3300 et 1684 $cm^{-1}$
RMN $^1H$ (DMSO $d_6$) : $\delta$ = 1,63 (2H,q) ; 2,18 (4H,t) ; 2,28 (12H,s) ; 6,22 (4H, s, échangeables avec $CF_3COOD$) ; 7,3 (4H,s) ; 10,04 (2H, s, échangeables avec $CF_3COOD$)

**Exemple 58 (méthode G1)**

**N,N'-Bis[3,5-diméthyl-4-[(nitrométhyl)sulfonyl]phényl]-1,3-benzène disulfonamide**

A un mélange de 2 g (8,18 mmoles) de 3,5-diméthyl-4-[(nitrométhyl)sulfonyl]aniline, 38 ml de tétrahydrofurane anhydre et 1,63 g (16,1 mmoles) de carbonate de calcium, maintenu sous atmosphère d'azote, on ajoute en une fois 1,1 g (4,09 mmoles) de chlorure de 1,3-benzènedisulfonyle. On agite 24 heures à température ambiante puis 40 heures à 50° C.

On refroidit à l'ambiante, verse dans l'eau, extrait par $CH_2Cl_2$, lave à l'eau, sèche sur $Na_2SO_4$, filtre et évapore sous vide. Le résidu est purifié par chromatographie sur silice (à une température de -30° C) en éluant d'abord avec $CH_2Cl_2$ pour éliminer le produit de départ, puis avec un mélange $CH_2Cl_2$ + AcOEt (3:1) pour obtenir le produit attendu, que l'on recristallise une fois dans un mélange hexane - $CH_2Cl_2$ puis une deuxième fois dans un mélange AcOEt - hexane - $CH_2Cl_2$.
Rendement : 0,4 g (14 %)
F = 191 - 192° C (AcOEt - hexane - $CH_2Cl_2$)

| Analyse centésimale : $C_{24}H_{26}N_4O_{12}S_4$ + 0,18 $CH_3$-COOEt (M = 706,62) | | | | |
|---|---|---|---|---|
| | C % | H % | N % | S % |
| calculé<br>trouvé | 42,02<br>42,22 | 3,91<br>3,99 | 7,93<br>7,96 | 18,15<br>17,75 |

IR : $\bar{\nu}$ = 3245, 1595 et 1559 $cm^{-1}$
RMN $^1H$ (DMSO $d_6$) : $\delta$ = 2,54 (12H,s) ; 6,51 (4H, s, échangeables avec $CF_3COOD$) ; 7,06 (4H,s) ; 7,9 (1H,t) ; 8,2 (2H, d) ; 8,48 (1H,s) ; 11,4 (2H, s, échangeables avec $CF_3COOD$)

**Exemple 59 (méthode F1)**

**N,N'-Bis[3,5-diméthyl-4-[(nitrométhyl)sulfonyl]phényl]-éthanediamide**

A un mélange de 2 g (8,18 mmoles) de 3,5-diméthyl-4-[(nitrométhyl)sulfonyl]aniline, 50 ml de tétrahydrofurane anhydre, et 1,63 g (16,2 mmoles) de carbonate de calcium, maintenu sous atmosphère d'azote, on ajoute 0,35 ml (4,09 mmoles) de chlorure d'oxalyle. On agite 3 jours à température ambiante et verse dans l'eau. Le précipité obtenu est filtré, lavé à l'eau, séché sous vide et recristallisé dans un mélange éthanoldiméthylformamide.
Rendement : 1,62 g (73 %)
F = 246 - 248° C (EtOH - DMF)

| Analyse centésimale : $C_{20}H_{22}N_4O_{10}S_2$ (M = 542,528) | | | | |
|---|---|---|---|---|
| | C % | H % | N % | S % |
| calculé<br>trouvé | 44,27<br>44,35 | 4,09<br>4,23 | 10,33<br>10,34 | 11,82<br>11,62 |

IR : $\bar{\nu}$ = 3340 et 1700 $cm^{-1}$
RMN $^1H$ (DMSO $d_6$) : $\delta$ = 2,58 (12H,s) ; 6,56 (4H, s, échangeables avec $CF_3COOD$) ; 7,84 (4H,s) ; 11,2 (2H, s, échangeables avec $CF_3COOD$)

**Exemple 60 (méthode F1)**

**N,N'-Bis[4-[(nitrométhyl)sulfonyl]phényl]-1,5-pentanediamide**

A un mélange de 2 g (9,25 mmoles) de 4-[(nitrométhyl)sulfonyl]aniline (préparée selon US 5 153 227), 50 ml de tétrahydrofurane anhydre, 1,85 g (18,5 mmoles) de carbonate de calcium, maintenu sous atmosphère d'azote, on ajoute 0,78 g (4,63 mmoles) de dichlorure de glutaryle. On agite 4 jours à température ambiante, puis on chauffe 24 heures à 40° C et 16 heures à 60° C. On refroidit, verse dans un mélange eau + glace et agite 4 heures à 0° C. On filtre le précipité obtenu, qui est lavé à l'eau, et dissout dans de l'acétate d'éthyle. On lave la phase organique à l'eau, sèche sur $Na_2SO_4$, filtre et évapore sous vide. Le solide obtenu est recristallisé dans l'éthanol.
Rendement : 0,88 g (37 %)
F = 203 - 205° C (EtOH)

| Analyse centésimale : $C_{19}H_{20}N_4O_{10}S_2$ (M = 528,52) | | | | |
|---|---|---|---|---|
| | C % | H % | N % | S % |
| calculé<br>trouvé | 43,18<br>43,20 | 3,81<br>3,94 | 10,60<br>10,42 | 12,13<br>11,92 |

RMN $^1H$ (DMSO $d_6$) : δ = 1,9 à 2,1 (2H,m) ; 2,55 (4H,t) ; 6,6 (4H, s, échangeables avec $CF_3COOD$) ; 7,95 (8H,s) ; 10,56 (2H, s, échangeables avec $CF_3COOD$)

**Exemple 61 (méthode F1)**

**N,N'-Bis[3,5-diméthyl-4-[(nitrométhyl)sulfonyl]phényl]-1,4-butanediamide**

A un mélange de 2 g (8,18 mmoles) de 3,5-diméthyl-4[(nitrométhyl)sulfonyl]aniline, 50 ml de tétrahydrofurane anhydre, 1,6 g (16,3 mmoles) de carbonate de calcium, maintenu sous atmosphère d'azote, on ajoute 0,64 g (4,14 mmoles) de chlorure de succinyle et on agite 6 jours à température ambiante. On verse dans un mélange glace + eau, acidifie à pH = 3 avec HCl N, filtre le précipité obtenu, lave à l'eau et sèche sous vide. Le solide obtenu est lavé avec de l'acétate d'éthyle à chaud puis recristallisé dans l'éthanol.
Rendement : 0,115 g (4,8 %)
F = 226 - 228° C (EtOH)

| Analyse centésimale : $C_{22}H_{26}N_4O_{10}S_2$ + 0,06 EtOH + 0,5 $H_2O$ (M = 580,199) | | | | |
|---|---|---|---|---|
| | C% | H% | N% | S % |
| calculé<br>trouvé | 45,63<br>46,03 | 4,74<br>4,63 | 9,62<br>9,63 | 11,01<br>10,53 |

RMN $^1H$ (DMSO $d_6$) : δ = 2,5 (6H,s) ; 2,7 (4H,s) ; 6,44 (4H, s, échangeables avec $CF_3COOD$) ; 7,5 (4H,s) ; 10,4 (2H, s, échangeables avec $CF_3COOD$)

**Exemple 62 (méthode F1)**

**N,N'-Bis[3,5-diméthyl-4-[(nitrométhyl)sulfonyl]phényl]-1,3-propanediamide**

A un mélange de 2 g (8,18 mmoles) de 3,5-diméthyl-4-[(nitrométhyl)sulfonyl]aniline, 50 ml de tétrahydrofurane anhydre, 1,6 g (16,3 mmoles) de carbonate de calcium, maintenu sous atmosphère d'azote, on ajoute 0,58 g (4,14 mmoles) de dichlorure de malonyle et on agite 24 heures à température ambiante. On verse sur un mélange glace + eau, acidifie à pH = 3 avec HCl N, extrait à l'acétate d'éthyle, lave à l'eau, sèche sur $Na_2SO_4$, filtre et évapore sous vide. Le solide obtenu est recristallisé dans un mélange hexane - acétate d'éthyle.
Rendement : 0,2 g (9,8 %)
F = 197 - 199° C (Hexane - AcOEt)

| Analyse centésimale : $C_{21}H_{24}N_4O_{10}S_2$ + 0,5 $H_2O$ + 0,1 AcOEt (M = 574,396) | | | | |
|---|---|---|---|---|
| | C % | H % | N % | S % |
| calculé<br>trouvé | 44,75<br>44,88 | 4,53<br>4,56 | 9,75<br>9,62 | 11,17<br>10,78 |

RMN $^1H$ (DMSO $d_6$) : δ = 2,6 (12H,s) ; 3,65 (2H,s) ; 6,55 (4H, s, échangeables avec $CF_3COOD$) ; 7,6 (4H,s) ; 10,6 (2H, s, échangeables avec $CF_3COOD$)

**Exemple 63 (méthode H)**

**N,N'-Bis[3,5-diméthyl-4-[(nitrométhyl)sulfonyl]phényl]-urée**

A un mélange de 2 g (8,18 mmoles) de 3,5-diméthyl-4-[nitrométhyl)sulfonyl]aniline, 60 ml de tétrahydrofurane anhydre, 0,54 g (5,39 mmoles) de carbonate de calcium, maintenu sous atmosphère d'azote, on ajoute goutte à goutte 0,5 ml (4,09 mmoles) de chloroformiate de trichlorométhyle. On agite 24 heures à température ambiante, chauffe 8 heures à reflux, et laisse 4 jours à température ambiante. On verse dans l'eau, filtre le précipité obtenu, sèche et recristallise dans l'éthanol.
Rendement : 0,48 g (20 %)
F = 224 - 226° C (EtOH)

| Analyse centésimale : $C_{19}H_{22}N_4O_9S_2$ + 0,3 EtOH + 0,3 $H_2O$ (M = 533,765) | | | | |
|---|---|---|---|---|
| | C% | H% | N% | S % |
| calculé<br>trouvé | 44,11<br>44,43 | 4,61<br>4,43 | 10,50<br>10,75 | 12,02<br>11,76 |

RMN $^1H$ (DMSO $d_6$) : δ = 2,53 (12H,s) ; 6,49 (4H, s, échangeables avec $CF_3COOD$) ; 7,4 (4H,s) ; 9,33 (2H, s, échangeables avec $CF_3COOD$)

**Exemple 64 (méthode G2)**

**N,N'-Bis[3,5-diméthyl-4-[(nitrométhyl)sulfonyl]phényl]-1,3-benzènedi méthanesulfonamide**

A un mélange de 4 g (16,3 mmoles) de 3,5-diméthyl-4-[(nitrométhyl)sulfonyl]aniline, 76 ml de tétrahydrofurane anhydre, 5,2 ml (64,3 mmoles) de pyridine, maintenu sous atmosphère d'azote, on ajoute en une fois 2,5 g (8,2 mmoles) de dichlorure de 1,3-benzènediméthanesulfonyle (préparé selon J. LICHTENBERGER et Coll., Bull. Soc. Chim. Fr. 1961, 369) et on agite une nuit à température ambiante. On verse dans 500 ml d'eau, acidifie avec HCl N, extrait avec $CH_2Cl_2$, lave à l'eau, sèche sur $Na_2SO_4$, filtre et évapore sous vide. Le résidu est purifié par chromatographie sur silice en éluant avec $CH_2Cl_2$, puis recristallisé dans un mélange $CH_2Cl_2$ - hexane et chromatographié à nouveau sur silice en éluant avec un mélange AcOEt - hexane (1:1).
Rendement : 0,35 g (6 %)
F = 124-130° C (AcOEt - hexane)

| Analyse centésimale : $C_{26}H_{30}N_4O_{12}S_4$ + 0,3 AcOEt + 0,2 $H_2O$ (M = 748,847) | | | | |
|---|---|---|---|---|
| | C% | H% | N% | S % |
| calculé<br>trouvé | 43,63<br>43,96 | 4,41<br>4,44 | 7,48<br>7,27 | 17,13<br>17,05 |

RMN $^1H$ (DMSO $d_6$) : δ = 2,5 (12H,s) ; 4,6 (4H,s) ; 6,48 (4H, s, échangeables avec $CF_3COOD$) ; 6,95 (4H,s) ; 7,2 à 7,4 (4H,m) ; 10,5 (2H, s, échangeables avec $CF_3COOD$)

## Revendications

1. Composés de formule 1

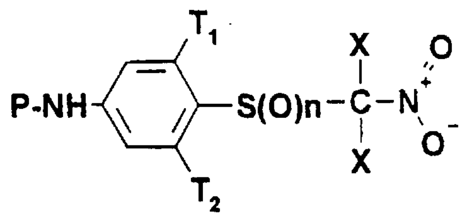

(1)

dans laquelle :

P représente

le radical (i) : $-(CO-NH)_m-SO_2-R$;

le radical (ii) :

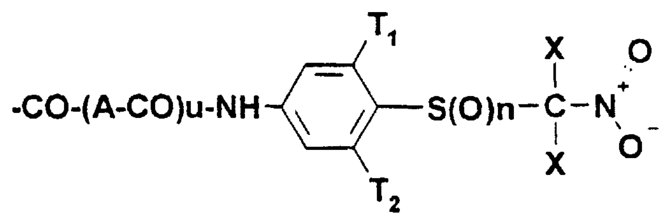

ou bien le radical (iii)

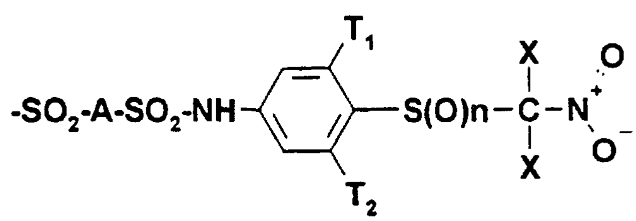

R représente un radical choisi parmi phényle, benzyle, diphénylméthyle, naphtyle, cycloalkylalkyle dans lequel la partie alkyle est en $C_1$-$C_4$ et la partie cycloalkyle est en $C_3$-$C_7$, et styryle, ledit radical étant éventuellement substitué par un ou plusieurs groupes Z identiques ou différents, ou bien

R représente un radical hétérocyclique aromatique en $C_3$-$C_5$ comprenant 1 ou 2 hétéroatomes choisis parmi O, S et N, ledit radical étant éventuellement substitué par un ou plusieurs groupes Z identiques ou différents et étant éventuellement condensé à 1 ou 2 noyaux phényle éventuellement substitués par un ou plusieurs groupes Z identiques ou différents, ou bien

R représente alkyle en $C_1$-$C_4$ éventuellement substitué par un ou plusieurs atomes d'halogène identiques ou différents, cycloalkyle en $C_3$-$C_7$ ou ($C_3$-$C_7$)-cycloalkyle-($C_1$-$C_4$)alkyle;

Z est choisi parmi un atome d'halogène, un groupe alkyle en $C_1$-$C_4$, alcoxy en $C_1$-$C_4$, nitro, cyano, trifluorométhyle, trifluorométhoxy, ($C_2$-$C_5$)alcanoylamino, ($C_1$-$C_4$)alkylsulfonyle, ($C_1$-$C_4$)alkylthio et phényle;

X représente un atome d'hydrogène ou d'halogène;

m est 0 ou 1;

n est 0, 1 ou 2;

$T_1$ et $T_2$ représentent indépendamment l'un de l'autre un atome d'hydrogène ou un groupe alkyle en $C_1$-$C_4$;

u est 0 ou 1;

A représente alkylène en $C_1$-$C_8$ ou le groupe

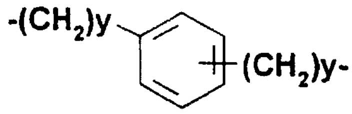

y étant un entier choisi parmi 0, 1, 2, 3 et 4 ; étant entendu que lorsque P représente le radical (ii), A peut également représenter une liaison;

leurs formes tautomères, et leurs sels d'addition avec des bases pharmaceutiquement acceptables.

2. Composés de formule 1 selon la revendication 1, **caractérisé en ce que**

P représente $-(CO-NH)_m-SO_2-R$;
R représente un radical choisi parmi phényle, diphénylméthyle, naphtyle et styryle, ledit radical étant éventuellement substitué par un ou plusieurs groupes
Z identiques ou différents, ou bien
R représente un radical hétérocyclique aromatique en $C_3$-$C_5$ comprenant 1 ou 2 hétéroatomes choisis parmi O, S et N, ledit radical étant éventuellement substitué par un ou plusieurs groupes Z identiques ou différents et étant éventuellement condensé à 1 ou 2 noyaux phényle éventuellement substitués par un ou plusieurs groupes Z identiques ou différents; ou bien
R représente alkyle en $C_1$-$C_4$ éventuellement substitué par un ou plusieurs atomes d'halogène identiques ou différents, cycloalkyle en $C_3$-$C_7$ ou ($C_3$-$C_7$)-cycloalkyle-($C_1$-$C_4$)alkyle;
Z, X, m et n étant tels que définis à la revendication 1,

leurs formes tautomères, et leurs sels d'addition avec des bases pharmaceutiquement acceptables.

3. Composés de formule 1 selon la revendication 1, **caractérisés en ce que** :

P représente $-(CO-NH)_m-SO_2-R$;
R représente phényle; phényle substitué par un ou plusieurs groupes Z identiques ou différents; benzyle; benzyle substitué par un ou plusieurs groupes Z identiques ou différents; alkyle en $C_1$-$C_4$ éventuellement substitué par un ou plusieurs atomes d'halogène identiques ou différents; cycloalkyle en $C_3$-$C_7$; ($C_3$-$C_7$)cycloalkyle-($C_1$-$C_4$)alkyle; styryle; thiényle; pyridyle; naphtyle; dibenzofuranyle; ou diphénylméthyle;
Z est choisi parmi un atome d'halogène, un groupe alkyle en $C_1$-$C_4$, alkoxy en $C_1$-$C_4$, nitro, trifluorométhyle, trifluorométhoxy, ($C_2$-$C_5$)alcanoylamino, ($C_1$-$C_4$)alkylsulfonyle et phényle;
X, m et n étant tels que définis à la revendication 1,

leurs formes tautomères, et leurs sels d'addition avec des bases pharmaceutiquement acceptables.

4. Composés de formule 1 selon l'une quelconque des revendications 1 et 3, **caractérisés en ce que** :

P représente $-(CO-NH)_m-SO_2-R$;
R représente phényle; phényle substitué par un ou plusieurs groupes Z identiques ou différents; benzyle; benzyle substitué par un ou plusieurs groupes Z identiques ou différents; méthyle; cycloalkyle en $C_3$-$C_7$; ($C_3$-$C_7$)cycloalkyle-($C_1$-$C_4$)alkyle; styryle; thiényle; pyridyle, naphtyle; dibenzofuranyle; diphénylméthyle ou 2,2,2-trifluoroéthyle;
Z est choisi parmi fluoro, chloro, bromo, méthyle, méthoxy, nitro, trifluorométhyle, trifluorométhoxy, acétamido, méthylsulfonyle et phényle;
X représente hydrogène ou chlore;
m et n étant tels que définis à la revendication 1,

leurs formes tautomères, et leurs sels d'addition avec des bases pharmaceutiquement acceptables.

5. Composés de formule 1 selon l'une quelconque des revendications 1, 3 et 4, **caractérisés en ce que** :

P représente $-(CO-NH)_m-SO_2-R$;
R représente phényle; phényle substitué par un ou plusieurs groupes Z identiques ou différents;

méthyle; cycloalkyle en $C_3$-$C_7$; ($C_3$-$C_7$)cycloalkyle-($C_1$-$C_4$)alkyle; styryle; thiényle; pyridyle, naphtyle; dibenzofuranyle; diphénylméthyle ou 2,2,2-trifluoroéthyle;
Z est choisi parmi fluoro, chloro, bromo, méthyle, méthoxy, nitro, trifluorométhyle, trifluorométhoxy, acétamido, méthylsulfonyle et phényle;
X représente hydrogène ou chlore;
m et n étant tels que définis,

leurs formes tautomères, et leurs sels d'addition avec des bases pharmaceutiquement acceptables.

**6.** Composés de formule 1 selon l'une quelconque des revendications 1, 3 et 4, choisis parmi :

N-[3,5-Diméthyl-4-[(nitrométhyl)sulfonyl]phényl]benzènesulfonamide;
3,4-Difluoro-N-[3,5-diméthyl-4[(nitrométhyl)sulfonyl]phényl]benzènesulfonamide;
3-Bromo-N-[3,5-diméthyl-4-[(nitrométhyl)sulfonyl]phényl]benzènesulfonamide;
N-[3,5-Diméthyl-4-[(nitrométhyl)sulfonyl]phényl]-2-(trifluorométhyl)benzènesulfonamide;
N-[3,5-Diméthyl-4-[(nitrométhyl)sulfonyl]phényl]-4-fluorobenzènesulfonamide;
N-[3,5-Diméthyl-4-[(nitrométhyl)sulfonyl]phényl]-3-fluorobenzènesuifonamide;
N-[3,5-Diméthyl-4-[(nitrométhyl)sulfonyl]phényl]phénylméthanesulfonamide;
2,3-Difluoro-N-[3,5-diméthyl-4-[(nitrométhyl)sulfonyl]phényl]benzènesulfonamide;
3,5-Difluoro-N-[3,5-diméthyl-4-[(nitrométhyl)sulfonyl]phényl]benzènesulfonamide; et
N-[3,5-Diméthyl-4-[(nitrométhyl)sulfonyl]phényl]-2-fluorobenzènesulfonamide.

**7.** Composés de formule (1) selon la revendication 1, **caractérisés en ce que** :

P représente :

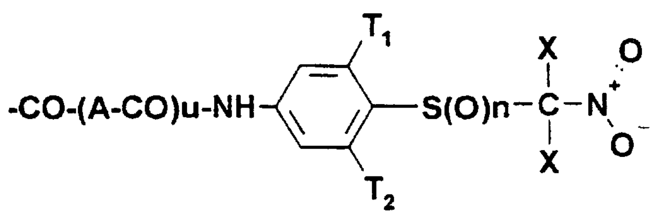

A représente une liaison ou alkylène en C1-C8;
u, n, X, $T_1$ et $T_2$ étant tels que définis à la revendication 1;

leurs formes tautomères et leurs sels d'addition avec des bases pharmaceutiquement acceptables.

**8.** Composés de formule 1 selon la revendication 1, **caractérisés en ce que** :

P représente :

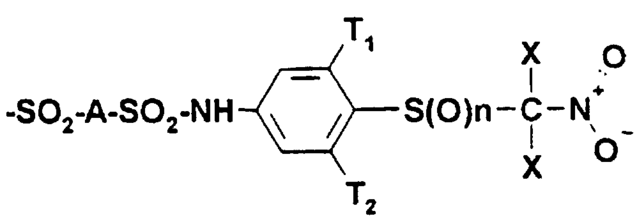

A représente le groupe

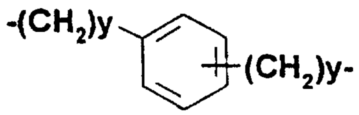

n, X, y, $T_1$ et $T_2$ étant tels que définis à la revendication 1,

leurs formes tautomères et leurs sels d'addition avec des bases pharmaceutiquement acceptables.

**9.** Composés de formule 1 selon la revendication 1, choisis parmi les composés:

N,N'-bis[3,5-diméthyl-4-[(nitrométhyl)suflonyl]phényl]-1,5-pentanediamide ;
N,N'-bis[3,5-diméthyl-4-[(nitrométhyl)sulfonyl]phényl]-1,8-octanediamide ;
N,N'-bis[4-[(nitrométhyl)sulfonyl]phényl]-1,5-pentanediamide ;
N,N'-bis[3,5-diméthyl-4-[(nitrométhyl)sulfonyl]phényl]-éthanediamide ;
N,N'-bis[3,5-diméthyl-4-[(nitrométhyl)sulfonyl]phényl]-urée ;
N,N'-bis[3,5-diméthyl-4-[(nitrométhyl)sulfonyl]phényl]-1,4-butanediamide ;
N,N'-bis[3,5-diméthyl-4-[(nitrométhyl)sulfonyl]phényl]-1,3-propanediamide ;
N,N'-bis[3,5-diméthyl-4-[(nitrométhyl)sulfonyl]phényl]-1,3-benzènedisulfonamide;
N,N'-bis[3,5-diméthyl-4-[(nitrométhyl)sulfonyl]phényl]-1,3-benzènediméthane sulfonamide.

**10.** Procédé de préparation des composés de formule 1 selon l'une quelconque des revendications 1 à 6, dans lesquels P représente $-(CO-NH)_mSO_2-R$, X est un atome d'hydrogène, m vaut zéro et n est égal à 2, **caractérisé en ce que** l'on traite le composé de formule 2

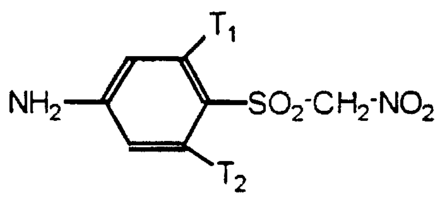

2

dans laquelle $T_1$ et $T_2$ sont tels que définis à la revendication 1, par un chlorure de sulfonyle de formule $RSO_2Cl$ où R est tel que défini à la revendication 1, en présence d'une base.

**11.** Procédé de préparation des composés de formule 1 selon l'une quelconque des revendications 1 à 5, dans lesquels P représente $-(CO-NH)_m-SO_2-R$, X représente un atome d'halogène, de préférence le chlore, m vaut zéro et n est égal à 2, **caractérisé en ce que** l'on traite un composé de formule 3 :

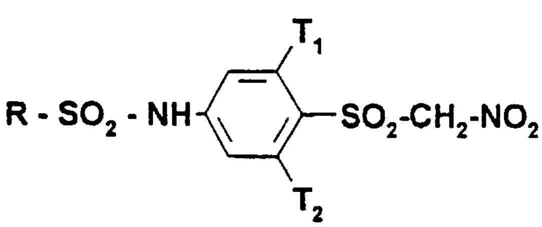

3

dans lequel R, $T_1$ et $T_2$ sont tels que définis à la revendication 1, par le N-halogénosuccinimide appropriée en présence d'un générateur de radicaux libres, par exemple le 2,2'-azobisisobutyronitrile.

**12.** Procédé de préparation des composés de formule 1 selon l'une quelconque des revendications 1 à 5, dans lesquels

P représente -$(CO\text{-}NH)_m$-$SO_2$-R, X est un atome d'hydrogène, m vaut 1 et n est égal à 2, **caractérisé en ce que** l'on traite le composé de formule 2

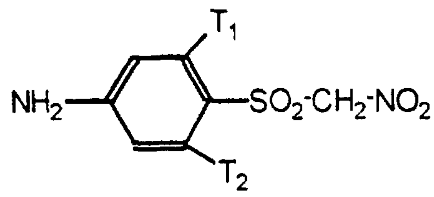

2

dans laquelle $T_1$ et $T_2$ sont tels que définis à la revendication 1, par un sulfonylisocyanate de formule $RSO_2NCO$, où R est tel que défini à la revendication 1.

**13.** Procédé de préparation des composés de formule 1 selon l'une quelconque des revendications 1 à 5, dans lesquels P représente -$(CO\text{-}NH)_m$-$SO_2$-R, X est un atome d'hydrogène et m et n sont égaux à zéro, **caractérisé en ce que** l'on traite le composé de formule 10

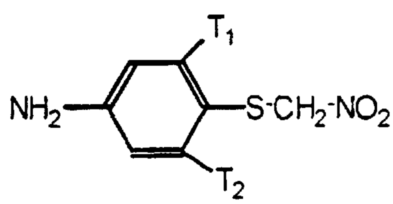

10

dans laquelle $T_1$ et $T_2$ sont tels que définis à la revendication 1, par un chlorure de sulfonyle de formule $RSO_2Cl$, où R est tel que défini à la revendication 1, en présence d'une base.

**14.** Procédé de préparation des composés de formule 1 selon l'une quelconque des revendications 1 à 5, dans lesquels P représente -$(CO\text{-}NH)_m$-$SO_2$-R, X est un atome d'hydrogène, m vaut 0 et n est égal à 1, **caractérisé en ce que** l'on traite un composé de formule 4.

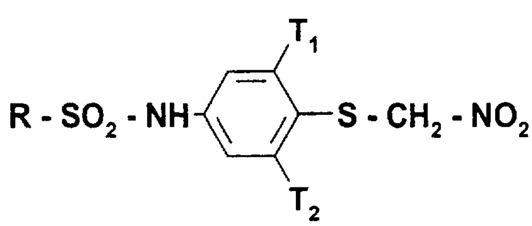

4

dans lequel R, $T_1$ et $T_2$ sont tels que définis à la revendication 1, par un agent oxydant tel que l'acide m-chlorobenzoïque.

**15.** Procédé pour la préparation de la 3,5-diméthyl-4-[(nitrométhyl)sulfonyl]aniline par hydrolyse basique du N-[3,5-diméthyl-4-[(nitrométhyl)thio]phényl]acétamide et oxidation avec $KMnO_4$ , **caractérisé en ce que** le N-[3,5-diméthyl-4-[(nitrométhyl)thio]phényl]acétamide est obtenu par réaction du nitrométhane sodé de formule $NaCH_2NO_2$ sur le thiocyanate de 4-acétamido-2,6-diméthylphényle.

**16.** Procédé de préparation des composés de formule (1) selon l'une quelconque des revendications 1, 7 et 9, dans lesquels P représente le radical (ii) :

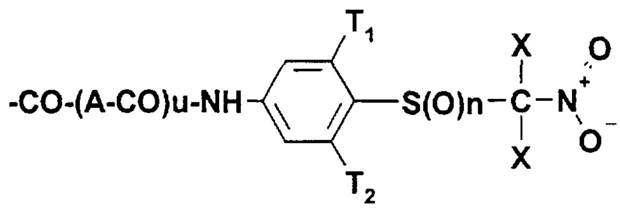

u est 1, X représente un atome d'hydrogène et n est égal à 2, **caractérisé en ce que** l'on traite un composé de formule 2

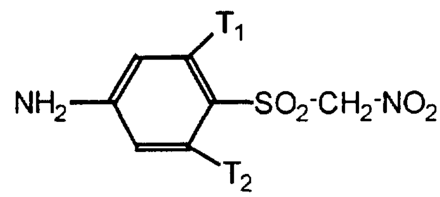

2

dans laquelle $T_1$ et $T_2$ sont tels que définis à la revendication 1, par un dichlorure de formule 5 :

**$Cl-(CO-A)_u-COCl$ 5**

dans laquelle A et u ont les mêmes significations qu'à la revendication 1, en présence d'une base, le rapport molaire du composé de formule 2 au composé de formule 5 étant au moins égal à 2.

**17.** Procédé de préparation des composés de formule (1) selon l'une quelconque des revendications 1, 8 et 9, dans lesquels P représente le radical (iii)

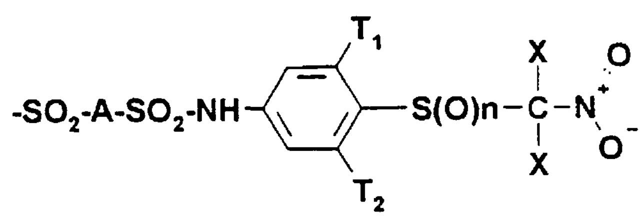

X représente un atome d'hydrogène et n est égal à 2, **caractérisé en ce que** l'on traite un composé de formule 2

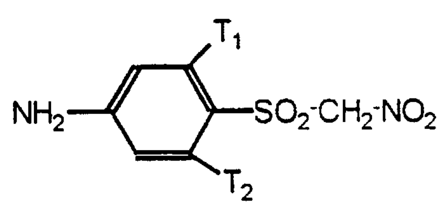

2

dans laquelle $T_1$ et $T_2$ sont tels que définis à la revendication 1, par un dichlorure de formule

$$Cl\text{-}SO_2\text{-}A\text{-}SO_2\text{-}Cl \qquad \underline{6}$$

en présence d'une base, le rapport molaire du composé de formule 2 au composé de formule 6 étant au moins égal à 2.

**18.** Procédé de préparation des composés de formule (1) selon l'une quelconque des revendications 1 et 7 dans lesquels P représente le radical (ii).

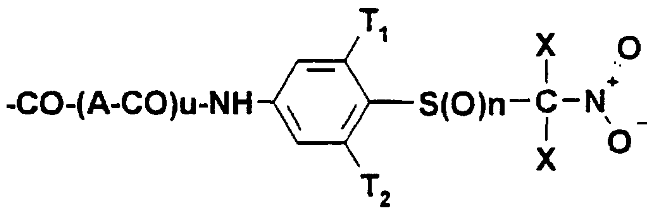

X représente un atome d'hydrogène, n est égal à 2 et u est égal à zéro, **caractérisé en ce que** l'on traite un composé de formule 2

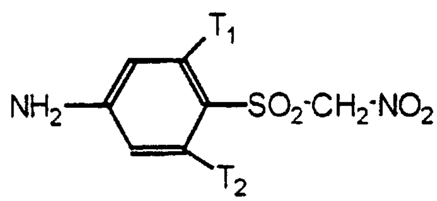

2

dans laquelle $T_1$ et $T_2$ sont tels que définis à la revendication 1, par le chloroformiate de trichlorométhyle en présence d'une base, le rapport molaire du composé de formule 2 au chloroformiate de trichlorométhyle étant au moins égal à 2.

**19.** Composition pharmaceutique comprenant, à titre de principe actif une quantité efficace d'au moins un composé selon l'une quelconque des revendications 1 à 9, en association avec un ou plusieurs véhicules pharmaceutiquement acceptables.

**20.** Composition pharmaceutique selon la revendication 18, **caractérisée en ce qu'**elle se présente sous forme de comprimés à libération immédiate, comprimés à libération contrôlée, gélules, solutés injectables, crèmes ou collyres.

**21.** Utilisation d'un composé selon l'une des revendications 1 à 9 pour la préparation d'un médicament destiné à inhiber l'aldose réductase.

**22.** Utilisation d'un composé selon l'une des revendications 1 à 9 pour la préparation d'un médicament destiné au traitement des complications diabétiques telles que cataracte, rétinopathies, neuropathies, néphropathies et maladies vasculaires.

### Patentansprüche

**1.** Verbindungen der Formel 1

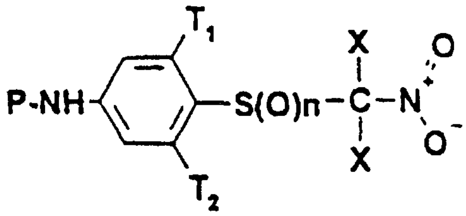

(1)

in der:

P für

den Rest (i): $-(CO-NH)_m-SO_2R$;
den Rest (ii):

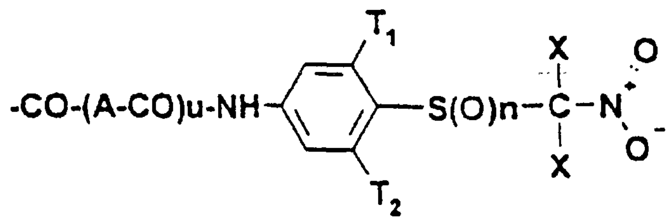

oder auch den Rest (iii):

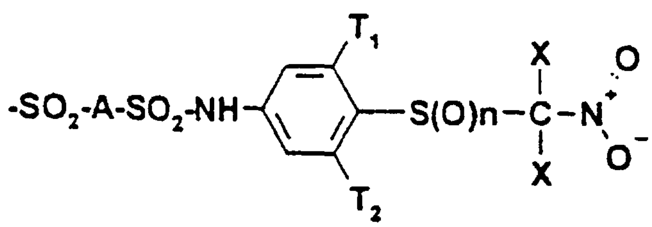

steht,

R für einen Rest steht, der ausgewählt ist aus Phenyl, Benzyl, Diphenylmethyl, Naphthyl, Cycloalkylalkyl mit einem $(C_1-C_4)$-Alkyl-Teil und einem $(C_3-C_7)$-Cycloalkyl-Teil und Styryl, wobei der Rest gegebenenfalls mit einer oder mehreren identischen oder verschiedenen Gruppen Z substituiert ist, oder auch
R für einen heterocyclischen aromatischen $(C_3-C_5)$-Rest steht, der 1 oder 2 Heteroatome umfasst, die ausgewählt sind aus O, S und N, wobei der Rest gegebenenfalls mit einer oder mehreren identischen oder verschiedenen Gruppen Z substituiert ist und gegebenenfalls mit 1 oder 2 Phenyl-Kernen kondensiert ist, welche gegebenenfalls mit einer oder mehreren identischen oder verschiedenen Gruppen Z substituiert sind, oder auch
R $(C_1-C_4)$-Alkyl, das gegebenenfalls mit einem oder mehreren identischen oder verschiedenen Halogenatomen substituiert ist, $(C_3-C_7)$-Cycloalkyl oder $(C_3-C_7)$-Cycloalkyl-$(C_1-C_4)$-alkyl darstellt;
Z ausgewählt ist aus einem Halogenatom, einer $(C_1-C_4)$-Alkylgruppe, einer $(C_1-C_4)$-Alkoxygruppe, Nitro, Cyano, Trifluormethyl, Trifluormethoxy, $(C_2-C_5)$-Alkanoylamino, $(C_1-C_4)$-Alkylsulfonyl, $(C_1-C_4)$-Alkylthio und Phenyl;
X ein Wasserstoff- oder Halogenatom darstellt;
m für 0 oder 1 steht;
n für 0, 1 oder 2 steht;

$T_1$ und $T_2$ unabhängig voneinander ein Wasserstoffatom oder eine ($C_1$-$C_4$)-Alkylgruppe darstellen;
u für 0 oder 1 steht;
A ($C_1$-$C_8$)-Alkylen oder die Gruppe

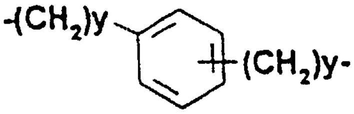

darstellt, wobei y eine ganze Zahl ausgewählt aus 0, 1, 2, 3 und 4 ist; mit der Voraussetzung, dass, wenn P für den Rest (ii) steht, A auch eine Bindung darstellen kann;

ihre tautomeren Formen und ihre Additionssalze mit pharmazeutisch annehmbaren Basen.

**2.** Verbindungen der Formel 1 nach Anspruch 1, **dadurch gekennzeichnet, dass**

P für -$(CO-NH)_m$-$SO_2$-R steht;
R einen Rest darstellt, der ausgewählt ist aus Phenyl, Diphenylmethyl, Naphthyl und Styryl, wobei der Rest gegebenenfalls mit einer oder mehreren identischen oder verschiedenen Gruppen Z substituiert ist, oder auch R einen heterocyclischen aromatischen ($C_3$-$C_5$)-Rest darstellt, der 1 oder 2 Heteroatome umfasst, die ausgewählt sind aus O, S und N, wobei der Rest gegebenenfalls mit einer oder mehreren identischen oder verschiedenen Gruppen Z substituiert ist und gegebenenfalls mit 1 oder 2 Phenyl-Kernen kondensiert ist, die gegebenenfalls mit einer oder mehreren identischen oder verschiedenen Gruppen Z substituiert sind; oder auch R für ($C_1$-$C_4$)-Alkyl, das gegebenenfalls mit einem oder mehreren identischen oder verschiedenen Halogenatomen substituiert ist, ($C_3$-$C_7$)-Cycloalkyl oder ($C_3$-$C_7$)-Cycloalkyl-($C_1$-$C_4$)-alkyl steht;
Z, X, m und n wie in Anspruch 1 definiert sind,

ihre tautomeren Formen und ihre Additionssalze mit pharmazeutisch annehmbaren Basen.

**3.** Verbindungen der Formel 1 nach Anspruch 1, **dadurch gekennzeichnet, dass**:

P für -$(CO-NH)_m$-$SO_2$-R steht;
R Phenyl; Phenyl, das mit einer oder mehreren identischen oder verschiedenen Gruppen Z substituiert ist; Benzyl; Benzyl, das mit einer oder mehreren identischen oder verschiedenen Gruppen Z substituiert ist; ($C_1$-$C_4$)-Alkyl, das gegebenenfalls mit einem oder mehreren identischen oder verschiedenen Halogenatomen substituiert ist; ($C_3$-$C_7$)-Cycloalkyl; ($C_3$-$C_7$)-Cycloalkyl-($C_1$-$C_4$)-alkyl; Styryl; Thienyl; Pyridyl; Naphthyl; Dibenzofuranyl; oder Diphenylmethyl darstellt;
Z ausgewählt ist aus einem Halogenatom, einer ($C_1$-$C_4$)-Alkylgruppe, einer ($C_1$-$C_4$)-Alkoxygruppe, Nitro, Trifluormethyl, Trifluormethoxy, ($C_2$-$C_5$)-Alkanoylamino, ($C_1$-$C_4$)-Alkylsulfonyl und Phenyl;
X, m und n wie in Anspruch 1 definiert sind;

ihre tautomeren Formen und ihre Additionssalze mit pharmazeutisch annehmbaren Basen.

**4.** Verbindungen der Formel 1 nach irgendeinem der Ansprüche 1 und 3, **dadurch gekennzeichnet, dass**:

P für -$(CO-NH)_m$-$SO_2$-R steht;
R Phenyl; Phenyl, das mit einer oder mehreren identischen oder verschiedenen Gruppen Z substituiert ist; Benzyl; Benzyl, das mit einer oder mehreren identischen oder verschiedenen Gruppen Z substituiert ist; Methyl; ($C_3$-$C_7$)-Cycloalkyl; ($C_3$-$C_7$)-Cycloalkyl-($C_1$-$C_4$)-alkyl; Styryl; Thienyl; Pyridyl; Naphthyl; Dibenzofuranyl; Diphenylmethyl oder 2,2,2-Trifluorethyl darstellt;
Z ausgewählt ist aus Fluor, Chlor, Brom, Methyl, Methoxy, Nitro, Trifluormethyl, Trifluormethoxy, Acetamido, Methylsulfonyl und Phenyl;
X für Wasserstoff oder Chlor steht;
m und n wie in Anspruch 1 definiert sind;

ihre tautomeren Formen und ihre Additionssalze mit pharmazeutisch annehmbaren Basen.

**5.** Verbindungen der Formel 1 nach irgendeinem der Ansprüche 1, 3 und 4, **dadurch gekennzeichnet, dass**:

P für $-(CO-NH)_m-SO_2-R$ steht;
R Phenyl; Phenyl, das mit einer oder mehreren identischen oder verschiedenen Gruppen Z substituiert ist; Methyl; $(C_3-C_7)$-Cycloalkyl; $(C_3-C_7)$-Cycloalkyl-$(C_1-C_4)$-alkyl; Styryl; Thienyl; Pyridyl; Naphthyl; Dibenzofuranyl; Diphenylmethyl oder 2,2,2-Trifluorethyl darstellt;
Z ausgewählt ist aus Fluor, Chlor, Brom, Methyl, Methoxy, Nitro, Trifluormethyl, Trifluormethoxy, Acetamido, Methylsulfonyl und Phenyl;
X für Wasserstoff oder Chlor steht;
m und n so wie definiert sind;

ihre tautomeren Formen und ihre Additionssalze mit pharmazeutisch annehmbaren Basen.

**6.** Verbindungen der Formel 1 nach irgendeinem der Ansprüche 1, 3 und 4, ausgewählt aus:

N-[3,5-Dimethyl-4-[(nitromethyl)sulfonyl]phenyl]benzolsulfonamid;
3,4-Difluor-N-[3,5-dimethyl-4-[(nitromethyl)sulfonyl]phenyl]benzolsulfonamid;
3-Brom-N-[3,5-dimethyl-4-[(nitromethyl)sulfonyl]phenyl]benzolsulfonamid;
N-[3,5-Dimethyl-4-[(nitromethyl)sulfonyl]phenyl]-2-(trifluormethyl)benzolsulfonamid;
N-[3,5-Dimethyl-4-[(nitromethyl)sulfonyl]phenyl]-4-fluorbenzolsulfonamid;
N-[3,5-Dimethyl-4-[(nitromethyl)sulfonyl]phenyl]-3-fluorbenzolsulfonamid;
N-[3,5-Dimethyl-4-[(nitromethyl)sulfonyl]phenyl]phenylmethansulfonamid;
2,3-Difluor-N-[3,5-dimethyl-4-[(nitromethyl)sulfonyl]phenyl]benzolsulfonamid;
3,5-Difluor-N-[3,5-dimethyl-4-[(nitromethyl)sulfonyl]phenyl]benzolsulfonamid; und
N-[3,5-Dimethyl-4-[(nitromethyl)sulfonyl]phenyl]-2-fluorbenzolsulfonamid.

**7.** Verbindungen der Formel 1 nach Anspruch 1, **dadurch gekennzeichnet, dass**:

P für:

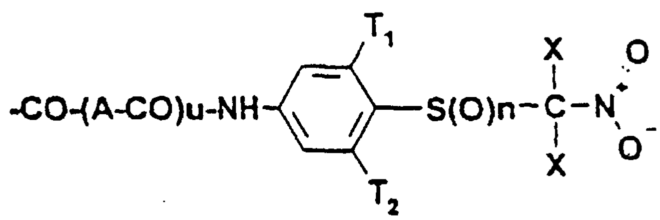

steht;
A eine Bindung oder $(C_1-C_8)$-Alkylen darstellt;
u, n, X, $T_1$ und $T_2$ wie in Anspruch 1 definiert sind;

ihre tautomeren Formen und ihre Additionssalze mit pharmazeutisch annehmbaren Basen.

**8.** Verbindungen der Formel 1 nach Anspruch 1, **dadurch gekennzeichnet, dass**:

P für

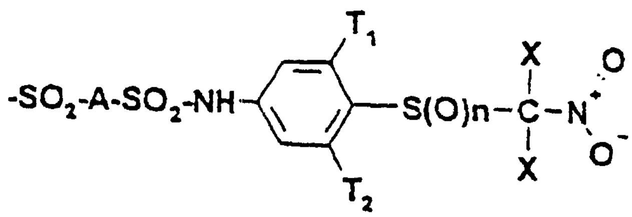

steht;
A die Gruppe

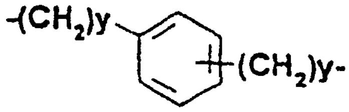

darstellt;
n, X, y, $T_1$ und $T_2$ wie in Anspruch 1 definiert sind,

ihre tautomeren Formen und ihre Additionssalze mit pharmazeutisch annehmbaren Basen.

**9.** Verbindungen der Formel 1 nach Anspruch 1, ausgewählt aus den Verbindungen:

N,N'-Bis[3,5-dimethyl-4-[(nitromethyl)sulfonyl]phenyl]-1,5-pentandiamid;
N,N'-Bis[3,5-dimethyl-4-[(nitromethyl)sulfonyl]phenyl]-1,8-octandiamid;
N,N'-Bis[4-[(nitromethyl)sulfonyl]phenyl]-1,5-pentandiamid;
N,N'-Bis[3,5-dimethyl-4-[(nitromethyl)sulfonyl]phenyl]ethandiamid;
N,N'-Bis[3,5-dimethyl-4-[(nitromethyl)sulfonyl]phenyl]harnstoff;
N,N'-Bis[3,5-dimethyl-4-[(nitromethyl)sulfonyl]phenyl]-1,4-butandiamid;
N,N'-Bis[3,5-dimethyl-4-[(nitromethyl)sulfonyl]phenyl]-1,3-propandiamid;
N,N'-Bis[3,5-dimethyl-4-[(nitromethyl)sulfonyl]phenyl]-1,3-benzoldisulfonamid;
N,N'-Bis[3,5-dimethyl-4-[(nitromethyl)sulfonyl]phenyl]-1,3-benzoldimethansulfonamid.

**10.** Verfahren zur Herstellung von Verbindungen der Formel 1 nach irgendeinem der Ansprüche 1 bis 6, in denen P für $-(CO-NH)_m-SO_2-R$ steht, X ein Wasserstoffatom ist, m für 0 steht und n gleich 2 ist, **dadurch gekennzeichnet, dass** man die Verbindung der Formel 2

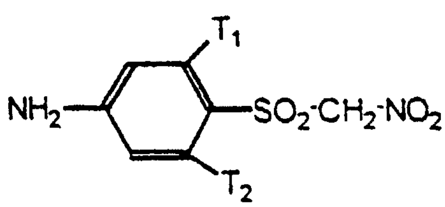

2

in der $T_1$ und $T_2$ wie in Anspruch 1 definiert sind, mit einem Sulfonylchlorid der Formel $RSO_2Cl$, worin R wie in Anspruch 1 definiert ist, in Anwesenheit einer Base behandelt.

**11.** Verfahren zur Herstellung von Verbindungen der Formel 1 nach irgendeinem der Ansprüche 1 bis 5, worin P für $-(CO-NH)_m-SO_2R$ steht, X ein Halogenatom, vorzugsweise Chlor, darstellt, m für 0 steht und n gleich 2 ist, **dadurch gekennzeichnet, dass** man eine Verbindung der Formel 3

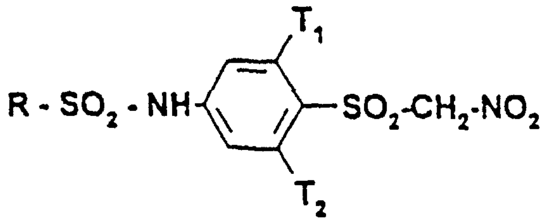

3

in der R, $T_1$ und $T_2$ wie in Anspruch 1 definiert sind, mit dem geeigneten N-Halogensuccinimid in Anwesenheit eines Erzeugers von freien Radikalen, beispielsweise 2,2'-Azobisisobutyronitril, behandelt.

**12.** Verfahren zur Herstellung von Verbindungen der Formel 1 nach irgendeinem der Ansprüche 1 bis 5, worin P für $-(CO-NH)_m-SO_2R$ steht, X ein Wasserstoffatom ist, m für 1 steht und n gleich 2 ist, **dadurch gekennzeichnet, dass** man die Verbindung der Formel 2

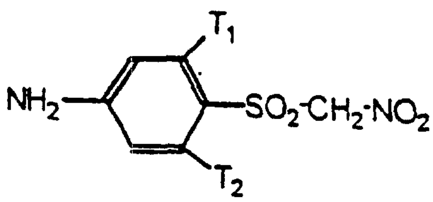

2

in der $T_1$ und $T_2$ wie in Anspruch 1 definiert sind, mit einem Sulfonylisocyanat der Formel $RSO_2NCO$, worin R wie in Anspruch 1 definiert ist, behandelt.

**13.** Verfahren zur Herstellung von Verbindungen der Formel 1 nach irgendeinem der Ansprüche 1 bis 5, worin P für $-(CO-NH)_m-SO_2R$ steht, X ein Wasserstoffatom ist und m und n gleich 0 sind, **dadurch gekennzeichnet, dass** man die Verbindung der Formel 10

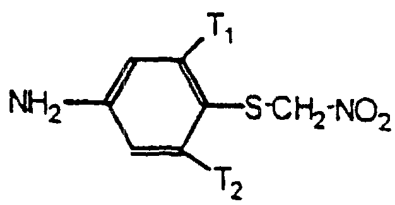

10

in der $T_1$ und $T_2$ wie in Anspruch 1 definiert sind, mit einem Sulfonylchlorid der Formel $RSO_2Cl$, worin R wie in Anspruch 1 definiert ist, in Anwesenheit einer Base behandelt.

**14.** Verfahren zur Herstellung von Verbindungen der Formel 1 nach irgendeinem der Ansprüche 1 bis 5, worin P für $-(CO-NH)_m-SO_2R$ steht, X ein Wasserstoffatom ist, m für 0 steht und n gleich 1 ist, **dadurch gekennzeichnet, dass** man eine Verbindung der Formel 4

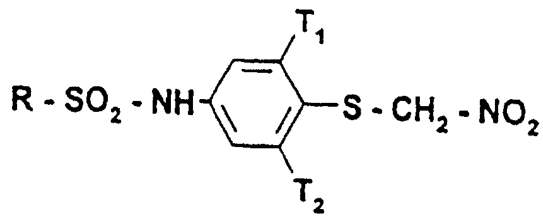

4

in der R, $T_1$ und $T_2$ wie in Anspruch 1 definiert sind, mit einem Oxidationsmittel, wie m-Chlorbenzoesäure, behandelt.

**15.** Verfahren zur Herstellung von 3,5-Dimethyl-4-[(nitromethyl)sulfonyl]anilin durch basische Hydrolyse von N-[3,5-Dimethyl-4-[(nitromethyl)thio]phenyl]acetamid und Oxidation mit $KMnO_4$, **dadurch gekennzeichnet, dass** N-[3,5-Dimethyl-4-[(nitromethyl)thio]phenyl]acetamid durch Umsetzung von Natriumnitromethan der Formel $NaCH_2NO_2$ mit dem Thiocyanat von 4-Acetamido-2,6-dimethylphenyl erhalten wird.

**16.** Verfahren zur Herstellung von Verbindungen der Formel 1 nach irgendeinem der Ansprüche 1, 7 und 9, worin P den Rest (ii):

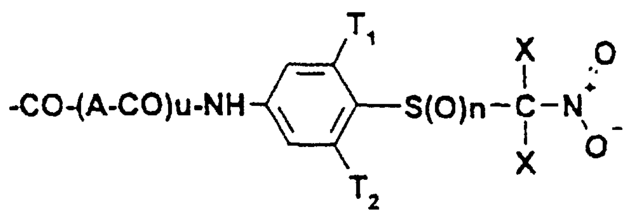

darstellt, u für 1 steht, X ein Wasserstoffatom darstellt und n gleich 2 ist, **dadurch gekennzeichnet, dass** man eine Verbindung der Formel 2

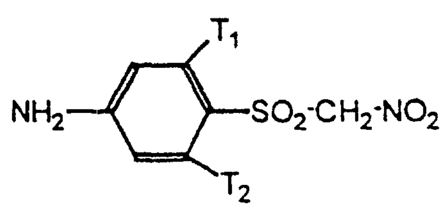

2

in der $T_1$ und $T_2$ wie in Anspruch 1 definiert sind, mit einem Dichlorid der Formel 5:

$$Cl\text{-}(CO\text{-}A)_uCOCl \qquad 5$$

in der A und u die gleichen Bedeutungen wie in Anspruch 1 aufweisen, in Anwesenheit einer Base behandelt, wobei das Molverhältnis der Verbindung der Formel 2 zur Verbindung der Formel 5 mindestens gleich 2 ist.

**17.** Verfahren zur Herstellung von Verbindungen der Formel 1 nach einem der Ansprüche 1, 8 und 9, worin P den Rest (iii)

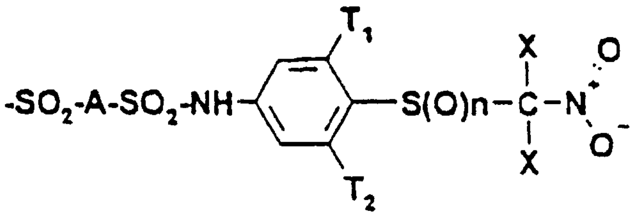

darstellt, X ein Wasserstoffatom darstellt und n gleich 2 ist, **dadurch gekennzeichnet, dass** man eine Verbindung der Formel 2

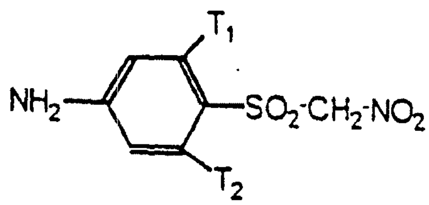

2

in der $T_1$ und $T_2$ wie in Anspruch 1 definiert sind, mit einem Dichlorid der Formel

$Cl-SO_2-A-SO_2-Cl$ 6

in Anwesenheit einer Base behandelt, wobei das Molverhältnis der Verbindung der Formel 2 zur Verbindung der Formel 6 mindestens gleich 2 ist.

**18.** Verfahren zur Herstellung von Verbindungen der Formel 1 nach irgendeinem der Ansprüche 1 und 7, worin P den Rest (ii)

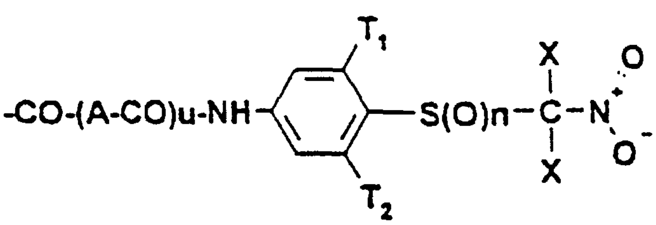

darstellt, X ein Wasserstoffatom darstellt, n gleich 2 ist und u gleich 0 ist, **dadurch gekennzeichnet, dass** man eine Verbindung der Formel 2

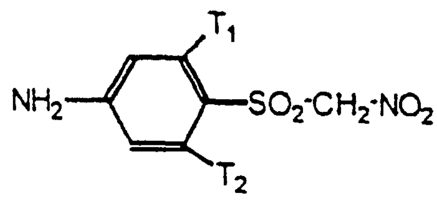

2

in der $T_1$ und $T_2$ wie in Anspruch 1 definiert sind, mit Trichlormethylchlorformiat in Anwesenheit einer Base behandelt, wobei das Molverhältnis der Verbindung der Formel 2 zum Trichlormethylchlorformiat mindestens gleich 2 ist.

**19.** Pharmazeutische Zusammensetzung, umfassend als Wirkstoff eine wirksame Menge mindestens einer Verbindung nach irgendeinem der Ansprüche 1 bis 9 in Verbindung mit einem oder mehreren pharmazeutisch annehmbaren Vehikeln.

**20.** Pharmazeutische Zusammensetzung nach Anspruch 19, **dadurch gekennzeichnet, dass** sie in Form von Tabletten mit sofortiger Freisetzung, Tabletten mit kontrollierter Freisetzung, Kapseln, injizierbaren Lösungen, Cremes oder Augentropfen bzw. Augensalben vorliegt.

**21.** Verwendung einer Verbindung nach einem der Ansprüche 1 bis 9 für die Herstellung eines Medikaments, das dazu bestimmt ist, die Aldosereduktase zu inhibieren.

**22.** Verwendung einer Verbindung nach einem der Ansprüche 1 bis 9 für die Herstellung eines Medikaments, das zur Behandlung von diabetischen Komplikationen, wie grauer Star, Retinopathien, Neuropathien, Nephropathien und vaskulären Erkrankungen, bestimmt ist.

## Claims

**1.** Compounds of the formula 1

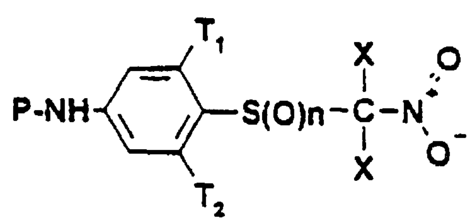

(1)

in which:

P represents

the radical (i): - $(CO\text{-}NH)_m\text{-}SO_2\text{-}R$;
the radical (ii):

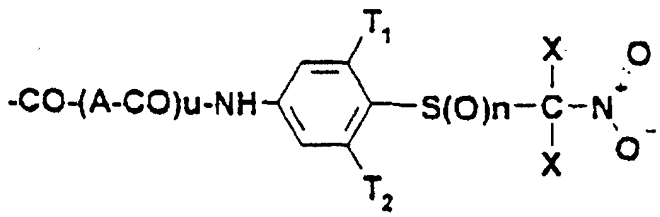

or the radical (iii):

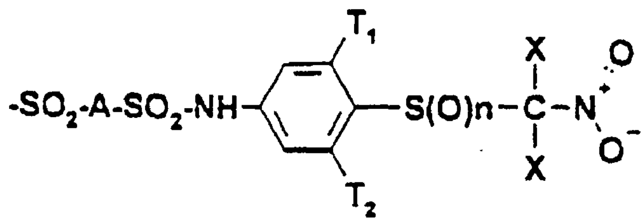

R represents a radical chosen from phenyl, benzyl, diphenylmethyl, naphthyl, cycloalkylalkyl in which the alkyl part is $C_1$-$C_4$ and the cycloalkyl part is $C_3$-$C_7$, and styryl, the said radical optionally being substituted with one or more groups Z which may be identical or different, or alternatively
R represents a $C_3$-$C_5$ aromatic heterocyclic radical comprising 1 or 2 hetero atoms chosen from O, S and N, the said radical optionally being substituted with one or more groups Z, which may be identical or different, and optionally being fused to 1 or 2 phenyl rings which are optionally substituted with one or more groups Z, which may be identical or different; or alternatively
R represents $C_1$-$C_4$ alkyl optionally substituted with one or more halogen atoms, which may be identical or different, $C_3$-$C_7$ cycloalkyl or cyclo($C_3$-$C_7$)alkyl($C_1$-$C_4$)alkyl;
Z is chosen from a halogen atom, a $C_1$-$C_4$ alkyl, $C_1$-$C_4$ alkoxy, nitro, cyano, trifluoromethyl, trifluoromethoxy, ($C_2$-$C_5$) alkylamino, ($C_1$-$C_4$) alkylsulphonyl, $C_1$-$C_4$)alkylthio and phenyl group;
X represents a hydrogen or halogen atom;
m is 0 or 1;
n is 0, 1 or 2;
$T_1$ and $T_2$ represent, independently of each other, a hydrogen atom or a $C_1$-$C_4$ alkyl group,
u is 0 or 1;
A represents $C_1$-$C_8$ alkylene or the group

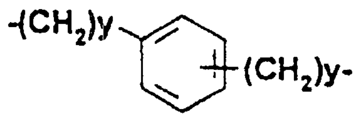

y being an integer chosen from 0, 1, 2, 3 and 4; it being understood that when P represents the radical (ii), A can also represent a bond;

the tautomeric forms thereof and the addition salts thereof with pharmaceutically acceptable bases.

2. Compounds of formula I according to Claim 1, **characterized in that**

P represents -$(CO\text{-}NH)_m$-$SO_2$-R
R represents a radical chosen from phenyl, diphenylmethyl, naphthyl and styryl, the said radical optionally being substituted with one or more groups Z, which may be identical or different, or alternatively
R represents a $C_3$-$C_5$ aromatic heterocyclic radical comprising 1 or 2 hetero atoms chosen from O, S and N, the said radical optionally being substituted with one or more groups Z, which may be identical or different, and optionally being fused to 1 or 2 phenyl rings which are optionally substituted with one or more groups Z,

which may be identical or different; or alternatively
R represents $C_1$-$C_4$ alkyl optionally substituted with one or more halogen atoms, which may be identical or different, $C_3$-$C_7$ cycloalkyl or cyclo ($C_3$-$C_7$)alkyl($C_1$-$C_4$)-alkyl;
Z, X, m and n being as defined in Claim 1,

the tautomeric forms thereof and the addition salts thereof with pharmaceutically acceptable bases.

**3.** Compounds of formula 1 according to Claim 1, **characterized in that**:

P represents $-(CO-NH)_m-SO_2-R$;
R represents phenyl; phenyl substituted with one or more groups Z, which may be identical or different; benzyl; benzyl substituted with one or more groups Z which may be identical or different; $C_1$-$C_4$ alkyl optionally substituted with one or more halogen atoms, which may be identical or different; $C_3$-$C_7$ cycloalkyl; cyclo($C_3$-$C_7$)alkyl($C_1$-$C_4$)alkyl; styryl; thienyl; pyridyl; naphthyl; dibenzofuryl; or diphenylmethyl;
Z is chosen from a halogen atom, a $C_1$-$C_4$ alkyl, $C_1$-$C_4$ alkoxy, nitro, trifluoromethyl, trifluoromethoxy, ($C_2$-$C_5$) alkylamino, ($C_1$-$C_4$)alkylsulphonyl and phenyl group;
X, m and n being as defined in Claim 1,

the tautomeric forms thereof and the addition salts thereof with pharmaceutically acceptable bases.

**4.** Compounds of formula 1 according to any one of Claims 1 to 3, **characterized in that** :

P represents $-(CO-NH)_m-SO_2-R$,
R represents phenyl; phenyl substituted with one or more groups Z, which may be identical or different; benzyl; benzyl substituted with one or more groups Z which may be identical or different; methyl; $C_3$-$C_7$ cycloalkyl; cyclo ($C_3$-$C_7$) alkyl ($C_1$-$C_4$)alkyl; styryl; thienyl; pyridyl; naphthyl; dibenzofuryl; diphenylmethyl or 2,2,2-trifluoroethyl;
Z is chosen from fluoro, chloro, bromo, methyl, methoxy, nitro, trifluoromethyl, trifluoromethoxy, acetamido, methylsulphonyl and phenyl;
X represents hydrogen or chlorine;
m and n being as defined in Claim 1,

the tautomeric forms thereof and the addition salts thereof with pharmaceutically acceptable bases.

**5.** Compounds of formula 1 according to any one of Claims 1, 3 and 4, **characterized in that**:

P represents$-(CO-NH)_m-SO_2-R$;
R represents phenyl; phenyl substituted with one or more groups Z, which may be identical or different; methyl; $C_3$-$C_7$ cycloalkyl; cyclo ($C_3$-$C_7$)alkyl($C_1$-$C_4$)alkyl; styryl; thienyl; pyridyl; naphthyl; dibenzofuryl; diphenylmethyl or 2,2,2-trifluoroethyl;
Z is chosen from fluoro, chloro, bromo, methyl, methoxy, nitro, trifluoromethyl, trifluoromethoxy, acetamido, methylsulphonyl and phenyl;
X represents hydrogen or chlorine;
m and n being as defined,

the tautomeric forms thereof and the addition salts thereof with pharmaceutically acceptable bases.

**6.** Compounds of formula 1 according to any one of Claims 1, 3 and 4, chosen from:

N-[3,5-dimethyl-4-[(nitromethyl)sulphonyl]phenyl]benzenesulphonamide;
3,4-difluoro-N-[3,5-dimethyl-4-[(nitromethyl)sulphonyl]phenyl]benzenesulphonamide;
3-bromo-N-[3,5-dimethyl-4-[(nitromethyl)sulphonyl]phenyl]benzenesulphonamide;
N-[3,5-dimethyl-4-[(nitromethyl)sulphonyl]phenyl]-2-(trifluoromethyl)benzenesulphonamide;
N-[3,5-dimethyl-4-[(nitromethyl)sulphonyl]phenyl]-4-fluorobenzenesulphonamide;
N-[3,5-dimethyl-4-[(nitromethyl)sulphonyl]phenyl]-3-fluorobenzenesulphonamide;
N-[3,5-dimethyl-4-[(nitromethyl)sulphonyl]phenyl]phenylmethanesulphonamide;
2,3-difluoro-N-[3,5-dimethyl-4-[(nitromethyl)sulphonyl]phenyl]benzenesulphonamide;
3,5-difluoro-N-[3,5-dimethyl-4-[(nitromethyl)sulphonyl]phenyl]benzenesulphonamide; and

N-[3,5-dimethyl-4-[(nitromethyl)sulphonyl]phenyl]-2-fluorobenzenesulphonamide.

**7.** Compounds of formula (1) according to Claim 1, **characterized in that**:

P represents

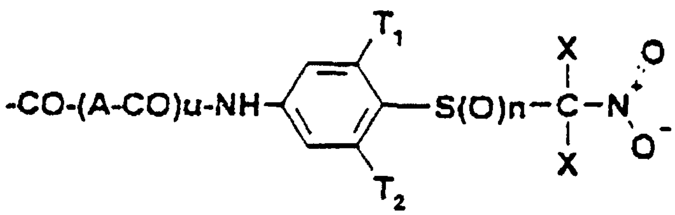

A represents a bond or C1-C8 alkylene;
u, n, X, $T_1$ and $T_2$ being as defined in Claim 1;

the tautomeric forms thereof and the addition salts thereof with pharmaceutically acceptable bases.

**8.** Compounds of formula 1 according to Claim 1, **characterized in that**:

P represents:

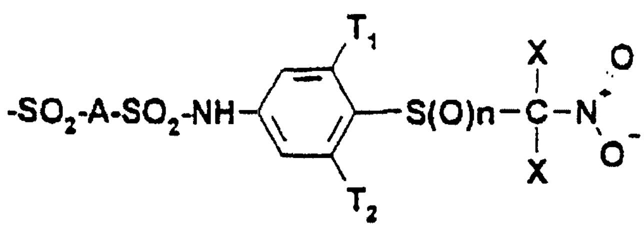

A represents the group

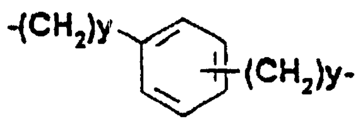

n, X, y, $T_1$ and $T_2$ being as defined in Claim 1,

the tautomeric forms thereof and the addition salts thereof with pharmaceutically acceptable bases.

**9.** Compounds of formula 1 according to Claim 1, chosen from the compounds:

N,N'-bis[3,5-dimethyl-4-[(nitromethyl)sulphonyl]phenyl]-1,5-pentanediamide;
N,N'-bis[3,5-dimethyl-4-[(nitromethyl)sulphonyl]phenyl]-1,8-octanediamide;
N,N'-bis[4-[(nitromethyl)sulphonyl]phenyl]-1,5-pentanediamide;
N,N'-bis[3,5-dimethyl-4-[(nitromethyl)sulphonyl]phenyl]-ethanediamide;
N,N'-bis[3,5-dimethyl-4-[(nitromethyl)sulphonyl]phenyl]-urea;
N,N'-bis[3,5-dimethyl-4-[(nitromethyl)sulphonyl]phenyl]-1,4-butanediamide;
N,N'-bis[3,5-dimethyl-4-[(nitromethyl)sulphonyl]phenyl]-1,3-propanediamide;
N,N'-bis[3,5-dimethyl-4-[(nitromethyl)sulphonyl]phenyl]-1,3-benzenedisulphonamide;
N,N'-bis[3,5-dimethyl-4-[(nitromethyl)sulphonyl]phenyl]-1,3-benzenedimethane sulphonamide.

**10.** Process for preparing the compounds of formula 1 according to any one of Claims 1 to 6, in which P represents $-(CO-NH)_m-SO_2-R$, X is a hydrogen atom, m is zero and n is equal to 2, **characterized in that** the compound of formula 2

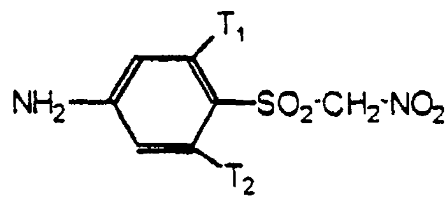

2

in which $T_1$ and $T_2$ are as defined in Claim 1, is treated with a sulphonyl chloride of formula $RSO_2Cl$ in which R is as defined in Claim 1, in the presence of a base.

**11.** Process for preparing the compounds of formula 1 according to any one of Claims 1 to 5, in which P represents $-(CO-NH)_m-SO_2-R$, X represents a halogen atom, preferably chlorine, m is zero and n is equal to 2, **characterized in that** a compound of formula 3

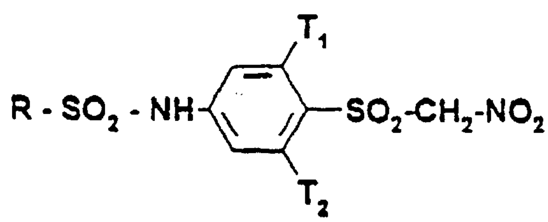

3

in which R, $T_1$ and $T_2$ are as defined in Claim 1, is treated with the appropriate N-halosuccinimide in the presence of a free-radical generator, for example 2,2'-azobisisobutyronitrile.

**12.** Process for preparing the compounds of formula 1 according to any one of Claims 1 to 5, in which P represents $-(CO-NH)_m-SO_2-R$, X is a hydrogen atom, m is 1 and n is equal to 2, **characterized in that** the compound of formula 2

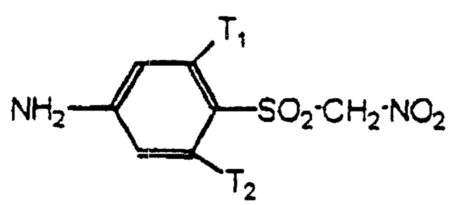

2

in which $T_1$ and $T_2$ are as defined in Claim 1, is treated with a sulphonyl isocyanate of formula $RSO_2NCO$, in which R is as defined in Claim 1.

**13.** Process for preparing the compounds of formula 1 according to any one of Claims 1 to 5, in which P represents $-(CO-NH)_m-SO_2-R$, X is a hydrogen atom and m and n are equal to zero, **characterized in that** the compound of formula 10

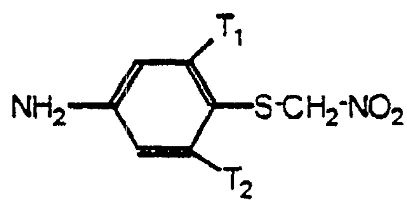

10

in which $T_1$ and $T_2$ are as defined in Claim 1, is treated with a sulphonyl chloride of formula $RSO_2Cl$, in which R is as defined in Claim 1, in the presence of a base.

**14.** Process for preparing the compounds of formula 1 according to any one of Claims 1 to 5, in which P represents $-(CO-NH)_m-SO_2-R$, X is a hydrogen atom, m is zero and n is equal to 1, **characterized in that** a compound of formula 4

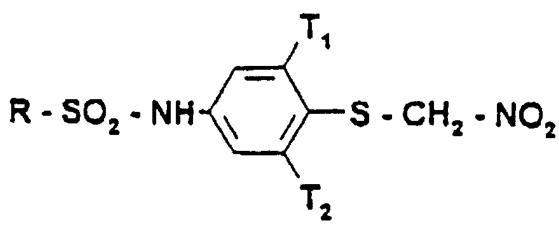

4

in which R, $T_1$ and $T_2$ are as defined in Claim 1, is treated with an oxidizing agent such as m-chlorobenzoic acid.

**15.** Process for preparing 3,5-dimethyl-4-[(nitromethyl)sulphonyl]aniline by basic hydrolysis of N-[3,5-dimethyl-4-[(nitromethyl)thio]phenyl]acetamide, and oxidation with $KMnO_4$ **characterized in that** the N-[3,5-dimethyl-4-[(nitromethyl)thio]phenyl]acetamide is obtained by reacting nitromethanesodium of formula $NaCH_2NO_2$ with 4-acetamido-2,6-dimethylphenyl thiocyanate.

**16.** Process for preparing the compounds of formula (1) according to any one of Claims 1, 7 and 9, in which P represents the radical (ii):

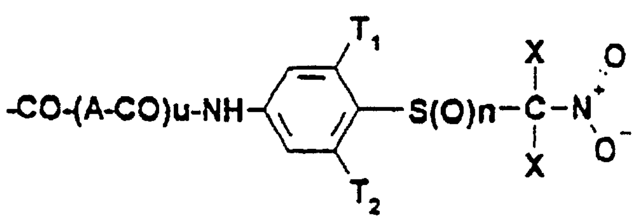

u is 1, X represents a hydrogen atom and n is equal to 2, **characterized in that** a compound of formula 2

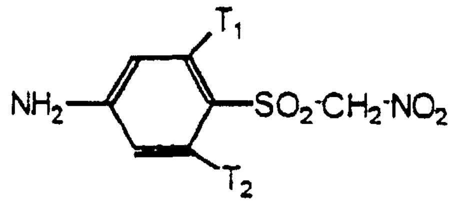

2

in which $T_1$ and $T_2$ are as defined in Claim 1, is treated with a dichloride of formula 5:

**$Cl-(CO-A)_u-COCl$ 5**

in which A and u have the same meanings as in Claim 1, in the presence of a base, the molar ratio of the compound of formula 2 to the compound of formula 5 being at least equal to 2.

**17.** Process for preparing the compounds of formula (1) according to any one of Claims 1, 8 and 9, in which P represents the radical (iii)

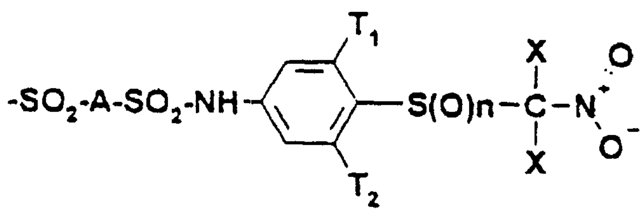

X represents a hydrogen atom and n is equal to 2, **characterized in that** a compound of formula 2

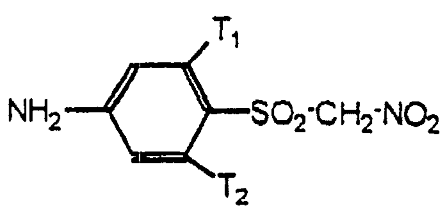

2

in which $T_1$ and $T_2$ are as defined in Claim 1, is treated with a dichloride of formula

**$Cl-SO_2-A-SO_2-Cl$ 6**

in the presence of a base, the molar ratio of the compound of formula 2 to the compound of formula 6 being at least equal to 2.

**18.** Process for preparing the compounds of formula (1) according to either of Claims 1 and 7, in which P represents the radical (ii)

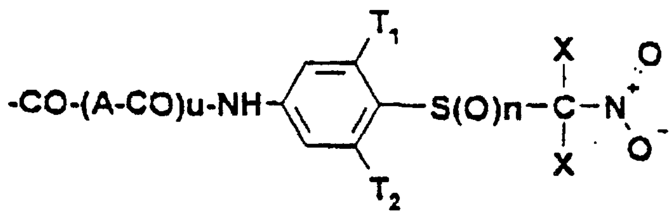

X represents a hydrogen atom, n is equal to 2 and u is equal to zero, **characterized in that** a compound of formula 2

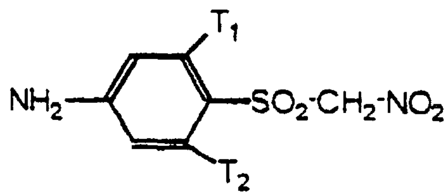

2

in which $T_1$ and $T_2$ are as defined in Claim 1, is treated with trichloromethyl chloroformate in the presence of a base, the molar ratio of the compound of formula 2 to the trichloromethyl chloroformate being at least equal to 2.

**19.** Pharmaceutical composition comprising, as active principle, an effective amount of at least one compound according to any one of Claims 1 to 9, in combination with one or more pharmaceutically acceptable vehicles.

**20.** Pharmaceutical composition according to Claim 18, **characterized in that** it is in the form of immediaterelease tablets, controlled-release tablets, gelatin capsules, injectable solutions, creams or eyedrops.

**21.** Use of a compound according to one of Claims 1 to 9 for the preparation of a medicinal product intended to inhibit aldose reductase.

**22.** Use of a compound according to one of Claims 1 to 9 for the preparation of a medicinal product intended for the treatment of diabetic complications such as cataracts, retinopathies, neuropathies, nephropathies and vascular diseases.